# EUROPEAN PATENT SPECIFICATION

(11) **EP 0 648 760 B1**
(45) Date of publication and mention of the grant of the patent: **27.01.1999**
(21) Application number: 94116173.9
(22) Date of filing: 13.10.1994
(51) Int. Cl.: C07D 401/10, C07D 417/10, C07D 403/12, C07D 401/04, C07D 413/10, C07D 253/07, C07D 253/06, C07D 253/075, A61K 31/53

(54) **Triazine derivative, production and use thereof**
Triazin-Derivate, Verfahren zu ihrer Herstellung und ihre Verwendung
Dérivés de la triazine, procédé pour leur préparation et leur utilisation

(30) Priority: 15.10.1993 JP 258654/93; 19.09.1994 JP 223761/94
(43) Date of publication of application: 19.04.1995
(73) Proprietor: TAKEDA CHEMICAL INDUSTRIES, LTD., Chuo-ku, Osaka 541 (JP)
(72) Inventor: Miki, Hideki, Toyone-gun, Osaka 563-01 (JP); Iwanaga, Koichi, Ikeda, Osaka 563 (JP); Matsuno, Toshimi, Yodogawa-ku, Osaka 532 (JP)
(74) Representative: Fürniss, Peter, Dr.

(56) References cited:
- EP-A- 0 383 285
- EP-A- 0 457 015
- EP-A- 0 476 439
- WO-A-86/00072
- DE-A- 2 532 363
- GB-A- 1 562 935
- US-A- 4 631 278
- J. MED. CHEM., vol.22, no.12, 1979 pages 1483 - 1487 M.W.MILLER ET AL 'Anticoccidial derivatives of 6-Azauracil'
- SYNTHETIC COMMUNICATIONS, vol.21, no.15, 1991 pages 1695 - 1703 J DANIEL ET AL 'Cyclisation of chlorosulfonyl isocyanate with phenylhydrazones'
- INDIAN JOURNAL OF HETEROCYCLIC CHEMISTRY, vol.3, October 1993 pages 121 - 126 R.M.ABDEL-RAHMAN ET AL 'Synthesis and biological activity of some new heterocyclic systems'
- TETRAHEDRON, vol.48, no.42, 1992 pages 9295 - 9304 A.ELGHANDOUR ET AL 'Studies with polyfunctionally substituted heteroaromatics'
- JUSTUS LIEBIG ANN. CHEM., vol.12, 1978 pages 2033 - 2043 H.GNICHTEL 'Isomere Heterocyclen durch Cyclisierung von syn- und anti- alpha Aminohydrazonen'
- JUSTUS LIEBIG ANN. CHEM., vol.713, 1968 pages 151 - 161 A.MUSTAFA ET AL 'Das Verhalten von Oxazolinonen-(5) und Thiazolinonen-(5) gegen N-Phenyl-hydroxylamin und Phenylhydrazin'

## Description

### Field of the Invention

The present invention relates to a novel triazine derivative or a salt thereof, and uses for them. More particularly, the invention relates to the novel triazine derivatives or a salt thereof, which is useful for controlling parasitic protozoa, particularly coccidia and the like, and an antiprotozoan composition comprising them.

### Background of the Invention

Parasitic protozoa are parasites on a broad range of animals inclusive of mammals, birds, fishes and insects. The parasitic protozoa establish themselves in the internal organs or the external organs such as the skin and eye of the host animal. As such, these parasites give the hosts serious lesion and often infect the producing farmers of domestic animals, poultry and fish, causing great economic damage. Coccidiosis, which is one of the diseases causing the most serious economic damage to breeding, is mainly caused by several kinds of protozoa of the genus Eimeria, such as E. tenella, E. necatrix, E. acervulira, E. maxima, E. brunetti and E. mivati.

For example, E. tenella parasitizes the intestinal inner walls, such as that of the caecum, and often inflicts fatal damage on the host. Thus, the E. tenella infection produces several manifestations such as extensive erosion, inflammation and hemorrhage of the intestinal paries due to the development of the protozoa, caecal blood retention, and, hence, anaemia, retardation of growth or death of the host. Endoparastic protozoa are usually transmitted orally and as to coccidiosis in particular, even intensive disinfection with potassium dichromate solution fails to kill the oocysts. Moreover, since their life cycle is as short as about 7 days, one engaged in large-scale animal farming has to face the outbreak and spread of disease without an effective countermeasure.

As far as fishes are concerned, ectoparasitic protozoa are serious problems of concern. Their parasitization damages the host's skin and gills, weakens the resistance of the host fish to infections and may occasionally be fatal. In large-scale fish farming, parasitic protozoa spread among the whole fish population on a farm and the resulting economic loss is too large to be overlooked.

A similar situation prevails for insects. Taking bees as an example, parasitic protozoan represented by Nosema apis are doing a great deal of harm to apiculture all over the world. Nosema apis destroys the internal organs to debilitate the host bee, and the host with accordingly decreased resistance tends to succumb to various other diseases.

Several drugs against parasitic protozoa have been proposed but most of them are limited in the indication and spectrum of activity and even protozoa with acquired resistance to certain drugs are already known. Furthermore, the weak activity of these drugs requires massive doses so that none are satisfactory enough from both economic and ecological points of view. Therefore, development of drugs which can be used broadly with sufficient effectiveness for control of such parasites in animals, poultry, fishes and insects are awaited in earnest.

As such drugs, 2-phenyl-6-azauracil derivatives were found to have an anticoccidial activity [J. Med. Chem. 22, 1483 (1979)] and a variety of 6-azauracil derivatives were synthesized and tested. However, these compounds were found to be teratogenic and, therefore, could not find application in the field. As compounds which overcame the problems related to the teratogenicity, 1,2,4-triazinediones are in use in some European countries, Australia and Hungary or South Africa as anticoccidial drugs. However, since these compounds remain in the body in long time their use is critically restricted and even banned in several countries including Japan.

In view of above state of the art, the present inventors have researched and found that a series of novel triazine derivatives have excellent activity against parasitic protozoa. Further intensive research led them to the discovery that this series of derivatives is suited for the control of a broad spectrum of parasitic protozoa encountered in rearing and raising animals such as mammals, birds, fish and insects under the usual husbandry and breeding conditions, are of low toxicity to animals, and exhibit remarkably high antiprotozoal activity even against strains resistant to the drugs heretofore available. This invention has been brought into being on the basis of the above findings.

### The summary of the Invention

The present invention is directed to:
(1) a triazine derivative of the formula 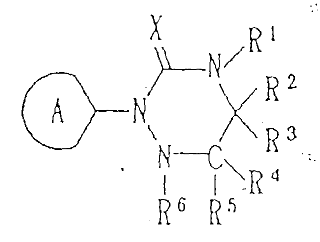
   wherein ring A is a phenyl or 5 or 6-membered unsaturated heterocyclic group which may be substituted with 1 to 3 substituents selected from the group consisting of (1) C₁₋₄ alkyl (2) C₂₋₄ alkenyl, (3) C₂₋₄ alkynyl, (4) C₃₋₆ cycloalkyl, (5) C₅₋₇ cycloalkenyl, (6) C₇₋₁₁ aralkyl, (7)phenyl, (8) C₁₋₆ alkoxy, (9) phenoxy, (10) C₁₋₆ alkanoyl, (11) benzoyl, (12) C₁₋₆ alkanoyloxy, (13) carboxyl, (14) C₂₋₇ alkoxycarbonyl, (15) carbamoyl, (16) N-mono-C₁₋₄ alkylcarbamoyl, (17) N-di-C₁₋₄ alkylcarbamoyl, (18) cycloaminocarbonyl, (19) halogen, (20) mono-, di- or tri-halo-C₁₋₄ alkyl, (21) oxo, (22) amidino, (23) imino, (24) amino which may be protected with a group selected from (i) formyl, (ii) C₁₋₆ alkylcarbonyl which may be substituted with halogen atoms, (iii) C₆₋₁₀ arylcarbonyl which may be substituted with 1 to 3 halogen atoms, C₁₋₆ alkylcarbonyl or nitro group, (iv) C₁₋₆ alkyloxycarbonyl which may be substituted with 1 to 3 halogen atoms; C₁₋₆ alkylcarbonyl or nitro group, (v) C₆₋₁₀ aryloxycarbonyl which may be substituted with 1 to 3 halogen atoms, C₁₋₆ alkylcarbonyl or nitro group, (vi) C₇₋₁₂ aralkyl-carbonyl which may be substituted with 1 to 3 halogen atoms, C₁₋₆ alkylcarbonyl or nitro group, (vii) trityl which may be substituted with 1 to 3 halogen atoms, C₁₋₆ alkylcarbonyl or nitro group and (viii) phthaloyl which may be substituted with 1 to 3 halogen atoms, C₁₋₈ alkylcarbonyl or nitro group, (25) mono-C₁₋₄ alkylamino, (26) di-C₁₋₄ alkylamino, (27) 3- to 6-membered cycloamino which may contain 1 to 8 hetero atoms selected from oxygen, sulfur, and nitrogen, (28) C₁₋₆ alkanamido, (29) benzamido, (30) carbamoylamino, (31) N-C₁₋₄ alkylcarbamoylamino, (32) N,N-di-C₁₋₄ alkylcarbamoylamino, (33) C₁₋₃ alkylenedioxy, (34) -B(OH)₂, (35) hydroxy, (36) epoxy, (37) nitro, (38) cyano, (39) mercapto, (40) sulfo, (41) sulfino, (42) phosphono, (43) dihydroxypolyol, (44) sulfamoyl, (45) mono-C₁₋₆ alkylsulfamoyl, (46) di-C₁₋₄ alkylsulfamoyl, (47) C₁₋₆ alkylthio, (48) phenylthio, (49) C₁₋₈ alkylsulfinyl, (50) phenylsulfinyl, (51) C₁₋₆ alkylsulfonyl, (52) phenylsulfonyl, and (53) 5- or 6-membered heterocyclic group containing 1 to 4 hetero atoms selected from oxygen, sulfur, and nitrogen which may bound through one or two atomic chain containing oxygen, sulfur nitrogen, and carbon, in which any group having a carbon chain of 2 or more carbon atoms or a cyclic group may be further substituted with one or two substituents selected from the group consisting of (a) halogen, (b) hydroxy, (c) oxo, (d) C₁₋₄ alkoxy, (e) di-C₁₋₄ alkylamino, (f) halo-C₁₋₄ alkyl, (g) C₁₋₄ acyl, (h) hydroxy-C₁₋₄ alkyl, (i) C₁₋₄ alkoxy-C₁₋₄ alkyl, (j) cyano, (k) thioxo, and (l) C₁₋₄ alkylthio, and in which when the substituent mentioned above exists on two ring-forming atoms adjacent to each other, they may bind together to form a ring;
   X is an oxygen or sulfur atom;
   R¹ and R⁶ are each a hydrogen atom, a C₁₋₇ acyl or a C₁₋₄ alkyl group;
   R² and R³ are each a hydrogen atom, a halogen atom, or a C₁₋₄ alkyl, or a phenyl group which may be bound through an oxygen or sulfur atom;
   R⁴ and R⁵ are each a hydrogen atom, a halogen atom, a phenyl group or C₁₋₄ alkyl group;
   R¹ and R², or R⁵ and R⁶ taken together may form a chemical bond; provided that where ring A is a phenyl group having at least a halogen atom in position 2 or 4 and X is an oxygen atom, R⁵ and R⁶ do not bind together to form a chemical bond; or salt thereof;
(2) an antiprotozoan composition comprising an effective amount of the triazine derivative or a salt thereof mentioned above (1) and a physiologically acceptable carrier, excipient or diluent; (3) a feed additive which comprises the triazine derivatives or a salt thereof as mentioned above, and (4)
a method of preparing the triazine derivatives mentioned above (1).

Referring to the above formula, the optionally substituted aromatic group, ring A, includes 5 to 6-membered homo- or hetero-aromatic groups which may have one to three substituents.

The carbocycle of said optionally substituted homoaromatic group may for example be benzene.

The heteroaromatic group includes 5- or 6-membered unsaturated heterocyclic groups containing 1 to 4 hetero-atoms selected from among oxygen, sulfur, nitrogen and the like in addition to at least one carbon atom, for example 5-membered heterocyclic groups containing 1 to 4 hetero-atoms selected from among oxygen, sulfur, nitrogen and the like in addition to at least one carbon atom, such as 2- or 3-thienyl, 2- or 3-furyl, 2- or 3-pyrrolyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-(1,2,3-thiadiazolyl), 1,2,5-thiadiazolyl, 1,2,3-triazolyl, 1,2,4-triazolyl, 1H- or 2H-tetrazolyl, etc., and 6-membered heterocyclic groups containing 1 to 4 hetero-atoms selected from among oxygen, sulfur, nitrogen and the like in addition to at least one carbon atom, such as 2-, 3- or 4-pyridyl, N-oxide-2-, 3- or 4-pyridyl, 2-, 4- or 5-pyrimidinyl, N-oxide-2-, 4- or 5-pyrimidinyl, oxoimidazinyl, dioxotriazinyl, pyranyl, thiopyranyl, 1,4-oxazinyl, 1,4-thiazinyl, 1,3-thiazinyl, triazinyl, oxotriazinyl, 3- or 4-pyridazinyl, pyrazinyl, N-oxide-3- or 4-pyridazinyl, etc. Among them, 6-membered heterocyclic groups having one hetero-atom as a ring member are preferred and nitrogen-containing heterocyclic groups are particularly desirable.

Such a homo- or hetero-aromatic group may be substituted, in 1 to 3 substitutable positions, by the substituent groups (1) to (53) mentioned above. Examples for
(1) C₁₋₄ alkyl are methyl, ethyl, propyl, isopropyl, butyl, isobutyl, sec-butyl, tert-butyl; for
(2) C₂₋₄ alkenyl are vinyl, 1-methylvinyl, 1-propenyl, allyl; for
(3) C₂₋₄ alkynyl are ethynyl, 1-propynyl, propargyl; for
(4) C₃₋₆ cycloalkyl are cyclopropyl, cyclobutyl, cyclopentyl, cyclohexyl; for
(5) C₅₋₇ cycloalkenyl are cyclopentenyl, cyclohexenyl; for
(6) C₇₋₁₁ aralkyl are benzyl, α-methylbenzyl, phenethyl; for
(7) C₁₋₆ alkoxy are methoxy, ethoxy, propoxy, isopropoxy, n-butoxy, iso-butoxy, sec-butoxy, tert-butoxy; for
(8) C₁₋₆ alkanoyl are formyl, acetyl, propionyl, n-butyryl, iso-butyryl; for
(9) C₁₋₆ alkanoyloxy are formyloxy, acetyloxy, propionyloxy, n-butyryloxy, iso-butyryloxy, etc. and benzoyloxy ; for
(10) C₂₋₇ alkoxycarbonyl are methoxycarbonyl, ethoxycarbonyl, n-propoxycarbonyl, iso-propoxycarbonyl, n-butoxycarbonyl, isobutoxycarbonyl, tert-butoxycarbonyl; for
(11) N-mono-C₁₋₄ alkylcarbamoyl are N-methylcarbamoyl, N-ethylcarbamoyl, N-propylcarbamoyl, N-isopropylcarbamoyl, N-butylcarbamoyl; for
(12) N-di-C₁₋₄ alkylcarbamoyl, are N,N-dimethylcarbamoyl, N,N-diethylcarbamoyl, N,N-dipropylcarbamoyl, N,N-dibutylcarbamoyl; for
(13) cycloaminocarbonyl, are 1-aziridinylcarbonyl, 1-azetidinylcarbonyl, 1-pyrrolidinylcarbonyl, 1-piperidinylcarbonyl, N-methylpiperazinylcarbonyl, morpholinocarbonyl; for
(14) halogens are F, Cl, Br, I ; for
(15) mono-, di- or tri-halo-C₁₋₄ alkyl are chloromethyl, dichloromethyl, trifluoromethyl, trifluoroethyl ; for
(16) mono-C₁₋₄ alkylamino are methylamino, ethylamino, propylamino, isopropylamino, butylamino ; for
(17) di-C₁₋₄ alkylamino are dimethylamino, diethylamino, dipropylamino, diisopropylamino, dibutylamino ; for
(18) 3- to 6-membered cycloamino which may contain 1 to 3 hetero-atoms selected from among oxygen, sulfur, nitrogen, etc. in addition to at least one carbon atom and one nitrogen atom, are aziridinyl, azetidinyl, pyrrolidinyl, pyrrolinyl, pyrrolyl, imidazolyl, pyrazolyl, imidazolidinyl, piperidino, morpholino, dihydropyridyl, pyridyl, N-methylpiperazinyl, N-ethylpiperazinyl; for
(19) C₁₋₆ alkanamido are formamido, acetamido, trifluoroacetamido, propionamido, butyrylamido, isobutyrylamido; for
(20) N-C₁₋₄ alkylcarbamoylamino, are N-methylcarbamoylamino, N-ethylcarbamoylamino, N-propylcarbamoylamino, N-isopropylcarbamoylamino, N-butylcarbamoylamino; for
(21) N-N-di-C₁₋₄ alkylcarbamoylamino, are N,N-dimethylcarbamoylamino, N,N-diethylcarbamoylamino, N,N-dipropylcarbamoylamino, N,N-dibutylcarbamoylamino ; for
(22) C₁₋₃ alkylenedioxy are methylenedioxy, ethylenedioxy for
(23) mono- C₁₋₆ monoalkylsulfamoyl are N-methylsulfamoyl, N-ethylsulfamoyl, N-propylsulfamoyl, N-isopropylsulfamoyl, N-butylsulfamoyl ; for
(24) di-C₁₋₄ alkylsulfamoyl are N,N-dimethylsulfamoyl, N,N-diethylsulfamoyl, N,N-dipropylsulfamoyl, N,N-dibutylsulfamoyl for
(25) C₁₋₆ alkylthio, are methylthio, ethylthio, propylthio, isopropylthio, n-butylthio, sec-butylthio, tert-butylthio ; for
(26) C₁₋₆ alkylsulfinyl are methylsulfinyl, ethylsulfinyl, propylsulfinyl, butylsulfinyl, for
(27) phenylsulfinyl
(28) C₁₋₆ alkylsulfonyl are methylsulfonyl, ethylsulfonyl, propylsulfonyl, butylsulfonyl; and for
(29) 5- or 6-membered heterocyclic groups containing 1 to 4 hetero-atoms selected from among oxygen, sulfur, nitrogen in addition to at least one carbon atom, which may be bound through a one or two atom chain containinq oxygen, sulfur, nitrogen, carbon are 2- or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, N-oxide-2-, 3- or 4-pyridyl, 2-, 4- or 5-pyrimidinyl, N-oxide-2-, 4- or 5-pyrimidinyl, oxoimidazinyl, dioxotriazinyl, pyranyl, thiopyranyl, 1,4-oxazinyl, 1,4-thiazinyl, 1,3-thiazinyl, triazinyl, oxotriazinyl, 3- or 4-pyridazinyl, pyrazinyl, N-oxide-3- or 4-pyridazinyl, and so on.

Of the above-mentioned groups (1) to (53) any group having a carbon chain of 2 or more C atoms or a cyclic group may be further substituted, in 1 or 2 substitutable positions, by
(a) halogens, e.g. Cl, F,
(b) hydroxy,
(c) oxo,
(d) C₁₋₄ alkoxy, e.g. methoxy, ethoxy,
(e) di-C₁₋₄ alkylamino, e.g. dimethylamino, diethylamino,
(f) halo-C₁₋₄ alkyl, e.g. chloromethyl, trifluoromethyl, trifluoroethyl,
(g) C₁₋₄ acyl, e.g. formyl, acetyl,
(h) hydroxy-C₁₋₄ alkyl, e.g. hydroxymethyl, 2-hydroxyethyl,
(i) C₁₋₄ alkoxy-C₁₋₄ alkyl, e.g. methoxymethyl, 2-ethoxyethyl,
(j) cyano
(k) thioxo and
(l) C₁₋₄ alkylthio, e.g. methylthio, ethylthio, propylthio, isopropylthio, n-butylthio, sec-butylthio, t-butylthio.

Further, when the substitution exists on two ring-forming atoms adjacent to each other, they may bind together to form a ring. The condensed ring thus formed is a 8 to 10 membered bicyclic ring which includes bicyclic aryl group such as 1- or 2-pentalenyl, 1- or 2- indenyl (1H- or 2H- indenyl) or 1- or 2- naphthylyl, and b-icyclic heterocyclic ring containing 1 to 4 hetero atom selected from among oxygen, sulfur, or nitrogen in addition to at least one carbon atom, such as indolyl, isoindolyl, benzofuryl, benzothiophenyl, benzothiazolyl, benzoxazolyl, tetrazolo[1,5-b]pyridazinyl, triazolo[4,5-b]pyridazinyl, benzimidazolyl, quinolyl, isoquinolyl, cinnolinyl, phthalazinyl, quinazolynyl, quinoxalinyl, indolidinyl, quinolidinyl, 1,8-naphthylidinyl, purinyl, putheridinyl, dibenzofuranyl, chromanyl, benzoxazinyl.

Among the above exemplified substituents, halogen atom, optionally substituted alkyl, especially C₁₋₄ alkyl group, haloalkyl group or optionally substituted aralkyl, especially phenyl C₁₋₄ alkyl group, a phenyl or heterocyclic group which may be bound through an atomic chain of 1 or 2 atoms such as phenoxy, phenylthio, benzoyl, benzoyloxy phenylsulfonyl, benzomide and heterocyclic group optionally bound through an atomic chain of 1 or 2 atoms are preferable.

In regard to substitution topology, the benzene ring, for instance, may preferably be substituted in position 3 and/or 5, more preferably be substituted in position 4 in addition to the substitution in 3 and/or 5 but these are not exclusive choices, of course.

X represents an oxygen or sulfur atom and is preferably an oxygen atom.

R¹ and R⁶ are a hydrogen atom, C₁₋₄ alkyl such as methyl, ethyl, propyl, isopropyl, butyl, isobutyl, s-butyl, t-butyl, or a C₁₋₇ acyl group, having a general formula of -COR^{a} wherein R^{a} is C₁₋₆ hydrocarbon group such as C₁₋₆ alkyl group (e.g. methyl, ethyl etc.), C₂₋₆ alkenyl group (e.g. vinyl), C₂₋₆ alkynyl group (e.g. ethynyl), C₃₋₆ cycloalkyl group (e.g. cyclohexyl), C₃₋₆ cycloalkenyl group (e.g. cyclohexenyl) or phenyl.

The group of R² and R³, includes a hydrogen atom, a halogen atom, or C₁₋₄ alkyl, e.g. methyl, ethyl, propyl, iso-propyl, etc., or phenyl, which may be bound through an oxygen or sulfur atom.

The halogen atom may for example be chlorine, bromine, fluorine or iodine.

The group of R⁴ and R⁵, includes a hydrogen atom, a halogen atom, C₁₋₄ alkyl, e.g. methyl, ethyl, propyl, iso-propyl, or phenyl.

The halogen may for example be chlorine, bromine, fluorine or iodine.

Preferred, among the above, are halogens and alkyl or phenyl group.

R¹ and R², or R⁵ and R⁶, may each bind together to form a chemical bond, that is to say a double bond between the carbon atom at 5-position and nitrogen atom at 4-position or between the carbon atom at 6-position and nitrogen atom at 1-position of the triazine ring. The double bond between 4- and 5-positions and that between 1- and 6-positions may exist concurrently but it is preferable that only one of them exists and is more preferable that a double bond be present between the 1- and 6-positions.

Where the triazine ring is tautomeric, the respective tautomers are also subsumed in the concept of triazine derivative of this invention.

Among the triazine derivatives of the present invention the compound of the following formula or salt thereof are preferable: 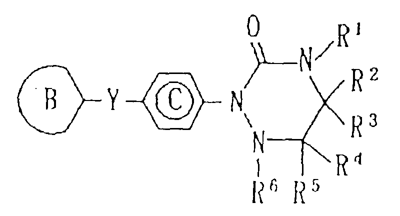 wherein R¹, R², R³, R⁴, R⁵ and R⁶ are the same meanings defined above,
ring B is an optionally substituted 5- or 6-membered cyclic group which may contain hetero atoms,
ring C is an optionally substituted phenylene group, Y is a chemical bond, -O-, -S(O)ₘ- or a C₁₋₄ alkylene group which may be substituted with 1 to 4 substituents selected from the group consisting of halogen, hydroxy, oxo cyano, C₁₋₄ alkoxy, mono- or di-C₁₋₄ alkylamino, halo- C₁₋₄ alkyl, C₁₋₄ acyl, hydroxy-C₁₋₄ alkyl, C₁₋₄ alkoxy-C₁₋₄ alkyl, C₁₋₄ alkoxy-carbonyl, thioxo and C₁₋₄ alkylthio and
m is 0, 1 or 2.

Optionally substituted 5- or 6-membered cyclic group represented by ring B includes carbon rings such as cycloalkyl, such as cyclopentyl or cyclohexyl, etc., cycloalkenyl, such as 1-, 2- or 3- cyclopentenyl, 1-, 2- or 3- cyclohexenyl, etc., phenyl or heteroaromatic groups containing 1 to 4 hetero-atoms selected from among oxygen, sulfur, nitrogen and the like in addition to at least one carbon atom, for example 2- or 3-thienyl, 2- or 3-furyl, 2- or 3-pyrrolyl, 2-, 3- or 4-pyridyl, 2-, 4- or 5-oxazolyl, 2-, 4- or 5-thiazolyl, 3-, 4- or 5-pyrazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl, 1H- or 2H-tetrazolyl, etc., N-oxide-2-, 3- or 4-pyridyl, 2-, 4- or 5-pyrimidinyl, N-oxide-2-, 4- or 5-pyrimidinyl, oxoimidazinyl, dioxotriazinyl, pyranyl, thiopyranyl, 1,4-oxazinyl, 1,4-thiazinyl, 1,3-thiazinyl, triazinyl, oxotriazinyl, 3- or 4-pyridazinyl, pyrazinyl, N-oxide-3- or 4-pyridazinyl,

Among them, 6-membered cyclic groups, especially phenyl is preferable and 6-membered nitrogen-containing heterocyclic groups are particularly desirable when ring B is a heterocyclic group.

Such a cyclic group may be substituted, in 1 to 3 substitutable positions, by the following substituent groups, among others:
(1) C₁₋₄ alkyl, e.g. methyl, ethyl, propyl, isopropyl, butyl, isobutyl, sec-butyl, tert-butyl,
(2) C₁₋₄ lower alkoxy, e.g. methoxy, ethoxy, propoxy, isopropoxy, butoxy, isobutosy, s-butoxy, t-butoxy,
(3) carboxy
(4) carbamoyl
(5) halogens, e.g. F, Cl, Br, I,
(6) mono-, di- or tri halo-C₁₋₄ alkyl e.g. chloromethyl, dichloromethyl, trifluoromethyl, trifluoroethyl,
(7) amino
(8) -B(OH)₂
(9) hydroxy
(10) nitro
(11) cyano
(12) mercapto
(13) sulfo
(14) sulfino
(15) phospho and
(16) C₁₋₄ acyl e.g. folmyl acetyl Halogen atom or alkyl or haloalkyl group is preferable.

When the substitutions exist on two ring-forming atoms adjacent to each other, they may bind together to form a ring which is condensed with the ring B.

The optionally substituted phenylene group represented by the ring C may be substituted by 1 to 4 preferably 1 to 2, substituents selected from those of the ring A. Among them, a halogen atom, alkyl, alkoxy or haloalkyl group is desirable.

The C₁₋₄ alkylene group of Y includes methylene, ethylene, propylene, trimethylene, or tetramethylene, This C₁₋₄ alkylene group may be substituted with 1 to 4 substituents selected from
(1) halogens, e.g. Cl, F,
(2) hydroxy,
(3) oxo,
(4) cyano,
(5) C₁₋₄ alkoxy, e.g. methoxy, ethoxy,
(6) mono- or di-C₁₋₄ alkylamino, e.g. methylamino, ethyklamino, propylamino, dimethylamino, diethylamino, dipropylamino,
(7) halo-C₁₋₄ alkyl, e.g. fluoromethyl, fluoethyl, chloromethyl, chloroethyl, bromomethyl, bromoethyl, trifluoromethyl, trifluoroethyl, chloropropyl,
(8) C₁₋₄ acyl, e.g. formyl, acetyl, propyonyl,
(9) hydroxy-C₁₋₄ alkyl, e.g. hydroxymethyl, hydroxyethyl, 2-hydroxyethyl, hydroxypropyl,
(10) C₁₋₄ alkoxy-C₁₋₄ alkyl, e.g. methoxymethyl, 2-ethoxyethyl
(11) C₁₋₄ alkoxy-carbonyl, e.g. methoxycarbonyl, ethoxycarbonyl, n-propoxycarbonyl, n-butoxycarbonyl, tert-butoxycarbonyl
(12) thioxo and
(13) C₁₋₄ alkylthio, e.g. methylthio, ethylthio, propylthio, isopropylthio, buthylthio, isobuthylthio, sec-buthylthio, tert-buthylthio etc. and so on.

Among them, a hydrocarbon group having 1 to 2 carbon atom in a straight chain which links the rings B and C such as optionally substituted methylene and ethylene, is preferable. Further, a C₁₋₃ alkylene, group which may optionally be substituted with 1 to 2 of the above mentioned substituents is most preferable.

For example, such a preferable embodiment includes methylene group optionally substituted with chloro, fluoro, methyl, monofluoromethyl, monochloromethyl, trifluoromethyl, hydroxy, carboxy, oxo (to a carbonyl group ), thioxo (to a thiocarbonyl group), methoxycarbonyl, ethoxycarbonyl, cyano or the like, ethylene group optionally substituted, at position 1 or 2 independently, with chloro, fluoro, methyl, monofluoromethyl, monochloromethyl, trifluoromethyl, hydroxy, carboxy, cyano or like, propylen group optionally substituted, at position 1, 2, or 3 independently, with chloro, fluoro, methyl, monofluoromethyl, monochloromethyl, trifluoromethyl, hydroxy carboxy, oxo (to form e.g. ethylidenecarbonyl, acetylethylene etc.), methoxy, ethoxy, methylthio, ethylthio, dimethylamino, diethylamino or the like.

In the optionally protected amino of A, the amino group may be protected with a group selected from
(1) formyl
(2) C₁₋₆ alkyl-carbonyl, e.g. acetyl, ethylcarbonyl, propylcarbonyl, butylcarbonyl which may substituted with halogen atoms, e.g. Cl, Br, F
(3) C₆₋₁₀ aryl-carbonyl, e.g. phenylcarbonyl which may substituted with 1 to 3 halogen atom, e.g. Cl, Br, F, C₁₋₆ alkylcarbonyl, e.g. methylcarbonyl, ethylcarbonyl, propylcarbonyl, butylcarbonyl or nitro group
(4) C₁₋₆ alkyloxycarbonyl, e.g. methoxycarbonyl, ethoxycarbonyl which may substituted with 1 to 3 halogen atom, e.g. Cl, Br, F , C₁₋₆ alkylcarbonyl, e.g. methylcarbonyl, ethylcarbonyl, propylcarbonyl, butylcarbonyl etc. or nitro group
(5) C₆₋₁₀ aryloxycarbony, e.g. phenoxycarbony which may substituted with 1 to 3 halogen atom, e.g. Cl, Br, F, C₁₋₆ alkylcarbonyl, e.g. methylcarbonyl, ethylcarbonyl, propylcarbonyl, butylcarbonyl or nitro group
(6) C₇₋₁₂ aralkyl-carbonyl, e.g. benzylcarbonyl, phenylethylcarbonyl which may substituted with 1 to 3 halogen atom, e.g. Cl, Br, F, C₁₋₆ alkylcarbonyl, e.g. methylcarbonyl, ethylcarbonyl, propylcarbonyl, butylcarbonyl or nitro group
(7) trityl which may substituted with 1 to 3 halogen atom, e.g. Cl, Br, F , C₁₋₆ alkylcarbonyl, e.g. methylcarbonyl, ethylcarbonyl, propylcarbonyl, butylcarbonyl or nitro group and
(8) phthaloyl which may substituted with 1 to 3 halogen atom, e.g. Cl, Br, F , C₁₋₆ alkylcarbonyl, e.g. methylcarbonyl, ethylcarbonyl, propylcarbonyl, butylcarbonyl or nitro group.

Among the divalent groups represented by Y, -O-, -S- or optionally substituted C₁₋₄ alkylene group is preferable.

In the above formula, prefered embodiments of R¹ and R⁶ are each hydrogen atom or an alkyl group.

Further, hydrogen, C₁₋₇ acyl group (e.g. acetyl, benzyl etc.) or an alkyl such as C₁₋₄ alkyl, is more preferred as R¹.

In the above formula, R² and R³ are preferably selected each from among hydrogen atom, halogen atom and an alkyl such as C₁₋₄ alkyl, C₁₋₄ alkoxy, C₁₋₄ alkylthio, phenyl, phenoxy, phenylthio, more preferably selected each from among hydrogen atom, halogen atom and an alkyl group. Further, at least either, especially both of R² and R³ may preferably be a hydrogen atom.

In the above formula, R⁴ and R⁵ are preferably selected each from among, hydrogen atom, halogen atom and a C₁₋₄ alkyl group and a phenyl group; more preferably selected from hydrogen or a C₁₋₄ alkyl group. Further, at least either, especially both, of R⁴ and R⁵ may preferably be a hydrogen atom.

R⁶ are preferred to be hydrogen, a C₁₋₄ alkyl group such as C₁₋₄ alkyl, or C₁₋₄ acyl.

The compounds in which R¹ and R² and/or R⁵ and R⁶ bind together to form a chemical bond are also desired.

The salt of a triazine derivative according to this invention is preferably a salt physiologically acceptable for animals and as such includes salts with alkali metals such as sodium, potassium, etc., salts with alkaline earth metals such as calcium etc., salts with inorganic acids such as hydrochloric acid, nitric acid, sulfuric acid, phosphoric acid, etc., and salts with organic acids such as acetic acid, succinic acid and so on.

The triazine derivative of this invention can be produced by, inter alia, the following reaction routes. 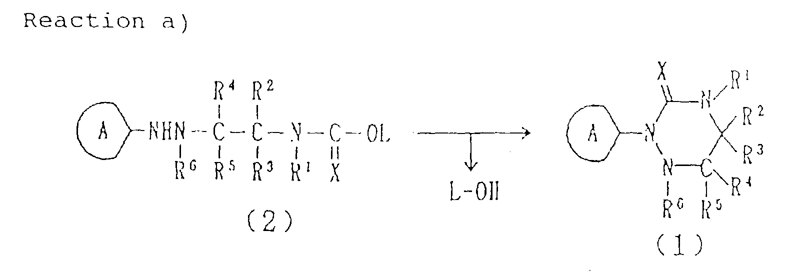 wherein ring A, X, R¹, R², R³, R⁴, R⁵ and R⁶ have the meanings defined hereinbefore; L represents hydrogen or an alkyl or aryl group.

The above reaction a) is directed to cyclization of hydrazine derivative (2) to a compound of general formula (1).

This reaction is generally conducted in an inert solvent or in the absence of a solvent, optionally in the presence of a Lewis acid or a Lewis base. The reaction temperature is generally about 60 to about 200°C and preferably about 100 to about 160°C. For this reaction, virtually any inert organic solvent can be employed. Thus, it may can be any of the reaction solvents which are generally used in organic chemistry, for example, benzene, ligroin, benzine, toluene, xylene, methylene chloride, ethylene chloride, chloroform, carbon tetrachloride, dichloroethane, chlorobenzene, o-dichlorobenzene, ethers (e.g. dibutyl ether, glycol dimethyl ether, diglycol dimethyl ether, tetrahydrofuran, dioxane, etc.), ketones (e.g. methyl ethyl ketone, methyl isopropyl ketone, methyl isobutyl ketone, etc.), esters (e.g. ethyl acetate etc.), nitriles (e.g. acetonitrile, propionitrile, etc.), amides (e.g. dimethylformamide, dimethylacetamide, hexamethylphosphorotriamide, etc.), N-methylpyrrolidone, dimethylsulfoxide, tetramethylenesulfone, mercaptoacetic acid, pyridine, and so on. This reaction may be carried out while the byproduct such as alcohol or water is removed. 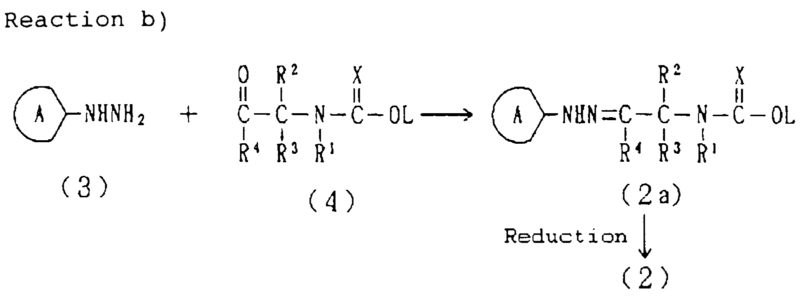 wherein ring A, R¹ - R⁴, L and X have the meanings defined hereinbefore.

The above reaction b) comprises reacting hydrazine (3) with a carbomic acid derivative (4) to give a hydrozone derivative (2a) followed by reduction to provide a hydrazine derivative (2).

The compound (2a) obtained by reacting compound (3) with compound (4) in an inert organic solvent is then reduced in the conventional manner. The inert organic solvent that can be used includes, among others, hydrocarbons (e.g. nonane, decane, dodecane, xylene, toluene, benzene, etc.), halogenated hydrocarbons (e.g. chloroform, dichloromethane, carbone tetrachloride, chlorobenzene, dichloroethane, etc.), alcohols (e.g. diethylene glycol etc.), ethers (e.g. diethylene glycol monobutyl ether, diethylene glycol dibutyl ether, etc.), dioxane, tetrahydrofuran, dimethylformamide, sulfoxides and sulfones such as dimethyl sulfoxide, tetramethylenesulfone and so on. This reaction can also be conducted in the presence of a Lewis acid or a dehydrating agent (e.g. dicyclohexylcarbodiimide, carbonyldiimidazole, etc.).

This reaction can be carried out generally at a temperature within the range of about -10°C to about 150°C. In particular, the temperature range of about 10 to about 20°C is preferred when a dehydrating agent is used and the range of about 60 to about 110°C is preferred in other instances.

Reduction of compound (2a) can be achieved by treating (2a) in the presence of about 1 to about 10 equivalents of a catalyst (palladium, NaBH₄, LiAlH₄, etc.) in alcohol or water at about 25 to about 60°C for about 0.5 to about 10 hours.

The compound (4) can be synthesized by the method of Tamejiro Hiyama et al. [Bull. Chem, Soc. Japan., 45, 1863-1866 (1972)]. 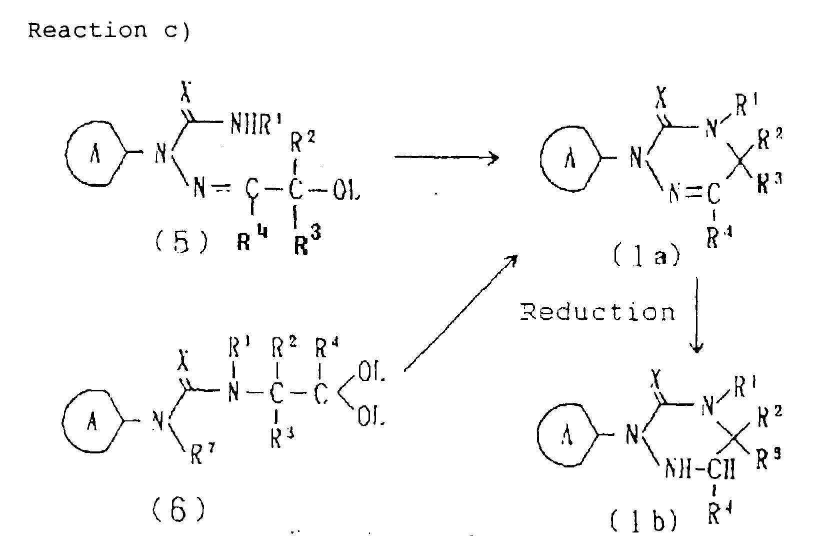 wherein ring A, R¹, R², R³, R⁴, L and X have the same meanings defined hereinbefore; R⁷ represents an optionally protected amino.

The above reaction c) is directed to cyclization of semicarbazone derivative (5) to synthesize a compound of the present invention. The cyclization reaction is carried out in an inert solvent or in the absence of a solvent, optionally in the presence of a Lewis acid or a Lewis base. The reaction is generally conducted at a temperature within the range from about 0°C to about 200°C. In particular, the reaction temperature of about 5 to about 30°C is preferred when a hydroxyl-activating agent (e.g. trifluoroacetic anhydride, acetic anhydride, phosphorus oxychloride, etc.) is employed. Where no hydroxyl-activating agent is employed, the reaction is carried out at about 100 to about 200°C, preferably at about 140 to about 180°C. As the reaction solvent, virtually any inert organic solvent can be employed. Thus, such solvent includes aliphatic and aromatic hydrocarbons (e.g. benzene, ligroin, benzine, toluene, xylene, etc.), halogenated hydrocarbone (e.g. methylene chloride, ethylene chloride, chloroform, carbon tetrachloride, dichloroethane, chlorobenzene, o-dichlorobenzene, etc.), ethers (e.g. dibutyl ether, glycol dimethyl ether, diglycol dimethyl ether, tetrahydrofuran, dioxane, etc.), ketones (e.g. methyl ethyl ketone, methyl isopropyl ketone, methyl isobutyl ketone, etc.), esters (e.g. ethyl acetate etc.), nitriles ( e.g. acetonitrile, propionitrile, etc.), amides (e.g. dimethylformamide, dimethylacetamide, hexamethylphosphoric triamide, etc.), N-methyl-pyrrolidone, dimethyl sulfoxide, tetramethylenesulfone, mercaptoacetic acid, pyridine, etc. To remove the residual hydroxyl-activating agent after the reaction, an organic base such as pyridine, triethylamine, dimethylpyridine, etc. or an inorganic base such as potassium hydroxide, sodium hydroxide, etc. can be employed.

This reaction may be conducted in the presence of a dehydrating agent, such as dicyclohexylcarbodiimide, carbonyldiimidazole and so on.

The tetrahydrotriazine compound (1a) thus obtained can be reduced in the manner described for reaction b) to give the hexahydrotriazine compound (1b).

Furthermore, when the 3,3-disubstituted semi-carbazide derivative (6) is used as an intermediate, it can be heat-cyclized in the same manner as the compound (5) to synthesize the tetrahydrotriazine compound (1a). The cyclization reaction is carried out generally at a temperature of about 60 to about 160°C, preferably about 80 to about 120°C. This reaction can also be conducted with the aid of a catalyst, that is to say in the presence of a Lewis acid or the like (e.g. trifluoroborane etherate, methanesulfonic acid, sulfuric acid, hydrochloric acid, phosphoric acid, polyphosphoric acid, etc.). In this reaction, the protection group for the optionally protected amino represented by R⁷ is any group generally used in organic chemistry [c.f. Shinjikken Kagaku koza Vol. 14, p.2555 Edit. Nihon seikagakkai].

The compound (6), wherein R⁷ is amino, can be obtained by a process which comprises dissolving an N-alkyl-N-(2,2-dialkoxyethyl)-N'-phenylurea in an aprotic solvent (e.g. dimethylformamide, dimethyl sulfoxide, N-methylpyrrolidone, etc.), adding a finely divided (ca. 50 - 100 µm) powder of potassium hydroxide or sodium hydroxide, then adding an aminating agent (e.g. hydroxyamino-O-sulfonic acid, 3-chloro-2-cyanopherethoxy amine, etc.) in a few portions under vigorous stirring at about 0 to about 10°C, further stirring the reaction mixture at about 25 to about 30°C for another 2 hours, pouring it in iced water, neutralizing the mixture with diluted hydrochloric acid, and extracting it with chloroform.

The N-alkyl-N-(2,2-dialkoxyethyl)-N'-phenylurea can be synthesized by the conventional reaction between phenyl isocyanate and N-2,2-dialkoxyethylamine.

The compound (6) in which R⁷ is a protected amino can be obtained by following reaction (c). 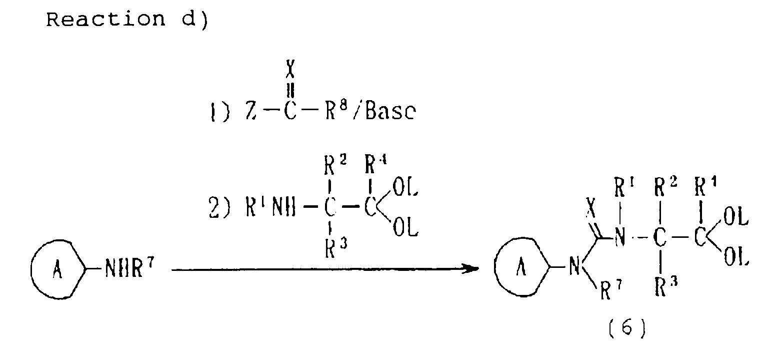 wherein A, R¹, R², R³, R⁴, R⁷, L and X have the same meanings defined hereinbefore; R⁸ is halogen atom or an optionally substituted C₁₋₄ alkoxy (e.g. halo C₁₋₄ alkyl etc.) or phenoxy and Z is halogen atom.

The above reaction (d) is directed to an amination followed by an acylation of a hydrazine derivative in the presence of base to provide compound (6). The reaction is generally conducted at a temperature with in the range of about -5 to about 40°C, preferably about 5 to about 10°C. The base used therein includes an organic base such as pyridine, triethylamine, DBU, collidine, 1,1,3,3-tetramethylguanizine and so on.

As the reaction solvent, virtually any inert organic solvent can be employed. Thus, such solvent includes aliphatic and aromatic hydrocarbons (e.g. benzene, ligroin, benzine, toluene, xylene, etc.), halogenated hydrocarbone (e.g. methylene chloride, ethylene chloride, chloroform, carbon tetrachloride, dichloroethane, chlorobenzene, o-dichlorobenzene, etc.), ethers (e.g. dibutyl ether, glycol dimethyl ether, diglycol dimethyl ether, tetrahydrofuran, dioxane, etc.), ketones (e.g. methyl ethyl ketone, methyl isopropyl ketone, methyl isobutyl ketone, etc.), esters (e.g. ethyl acetate etc.), nitriles ( e.g. acetonitrile, propionitrile, etc.), amides (e.g. dimethylformamide, dimethylacetamide, hexamethylphosphoric triamide, etc.), N-methyl-pyrrolidone, dimethyl sulfoxide, tetramethylenesulfone, mercaptoacetic acid, pyridine, etc.

Of the compounds of this invention, species in which position-5 represents =S can be obtained by a process which comprises heating a 1,2,4-triazine-1,3-dione compound (which can be synthesized in accordance with the manner reported by Max W. Miller et al., J. Med. Chem., 22, 1483, 1979) with Lawesson's reagent or phosphorus pentasulfide in a solvent such as aliphatic or aromatic hydrocarbons which may optionally be substituted (e.g. benzene, ligroin, benzine, toluene, xylene, methylene chloride, ethylene chloride, chloroform, carbon tetrachloride, dichloroethane, chlorobenzene, o-dichlorobenzene, etc.), ethers (e.g. dibutyl ether, glycol dimethyl ether, diglycol dimethyl ether, tetrahydrofuran, dioxane, etc.), and ketones (e.g. methyl ethyl ketone etc.). The thus-synthesized 5-thion (=S) compound can be reduced with the aid of Raney nickel to give the 5-methylene compound.

The compound (1) and physiologically acceptable salt of the present invention are suitable for the control of parasitic protozoa encountered in the husbandry and/or production of animals such as mammals, birds, fish or insects and show activity against individual or all stages of growth of such pathogenic parasitic protozoa. Furthermore, these compounds show sufficiently effective activity within the usual range of doses against the protozoa which are resistant to the known drugs. As a result, morbidity and the mortality of host animals are decreased and, hence, the efficiency of animal production and reproduction (e.g. the efficiency of production of meat, milk, furs, hides and skins, eggs, honey, etc. as well as the efficiency of breeding) are increased. Moreover, use of the compound of the present invention enables economical raising of various animals with good efficiency by preventing protozoal infection such as coccidial infection.

The protozoan diseases that can be controlled by the compound of this invention are of a broad range. Thus, the parasitic protozoa that can be controlled include, among others, protozoa of the Apicomplexa, particularly of the family Eimeriidae, such as the genus Eimeria, e.g. E. acervulina, E. adenoides, E. alabamensis, E. arloinqi, E. auburnensis, E. bovis, E. brunetti, E. canis, E. contorta, E. ellipsoidalis, E. falciformis, E. gallopavonis, E. hagani, E. intestinalis, E. magna, E. maxima, E. meleagridis, E. meleagrimitis, E. mitis, E. mivati, E. necatrix, E. ninakohlyakimovae. E. ovis, E. parva, E. pavonis, E. perforans, E. piriformis, E. praecox, E. stiedai, E. suis, E. tenella, E. truncata, E. zuernii, etc., the genus Isospora such as I. belli, I. canis, I. felis, I. rivolta, I. suis, etc., Toxoplasma gondii, and the genus Cryptosporidium, particularly Cryptosporidium s.p., the family Sarcocystidae, e.g. S. bovicanis, S. bovihominis, S. ovicanis, S. ovifelis, S. suihominis, etc., the genus Leucocytozoon, e.g. L. simondi , L. caulleryi, etc., the family Plasmodiidae, e.g. P. berghei, P. falciparum, P. malariae, P. ovale, P. vivax, etc., protozoa of the subclass Piroplasmea, more specifically of the genus Babesia, e.g. B. argentina, B. bovis, B. canis, etc., the genus Theileria, e.g. T. parva etc., Adeleina, Hepatozoon canis and so on.

Furthermore, protozoa taxonomically belonging to Myxospora or Microspora, protozoa of the genus Glugea and those of the genus Nosema may also be mentioned.

The compound (1) and its physiologically acceptable salt can be used both prophylactically and therapeutically in protozoan infections in mammalian animals (e.g. cattle, horse, swine, sheep, goat, camel, buffalo, donkey, rabbit, deer, reindeer, mink, chinchilla, raccoon, mouse, rat, guinea pig, golden hamster, dog, cat, etc.), birds (e.g. chicken, quail, goose, turkey, duck, wild duck, dove, pigeon, etc.), fresh-water and sea-water fishes (e.g. carp, eel, trout, smelt fish, salmon, ruffer, sole, flatfish, seabream, sea bass, catfish, etc.) and even in insects such as honey bees.

The compound (1) and its physiologically acceptable salt can be safely administered to any of the above-mentioned animals, either as they are or in various dosage forms according to the route of administration which may be oral or parenteral. The dosage forms mentioned above can be manufactured by the per se known methods (e.g. Japanese patent application unexamined publication No. H5-1047 which corresponds to EP-A-476439, Japanese patent application unexamined publication No. H5-117250 which corresponds to EP-A-457015, Japanese patent application unexamined publication No. H2-240003 which corresponds to EP-A-383285, Japanese patent application unexamined publication No. S62-61972 which corresponds to EP-A-215354, etc.).

The compound of the general formula (1) or physiologically acceptable salt thereof in the present invention can be used for preparing a prophylactic and therapeutic agent for protozoal disease by mixing them with a pharmaceutically acceptable additive(s) such as a diluent and an excipient, if necessary, to formulate the antiprotozoal composition according to a known pharmaceutical method, and then incorporated in feed or drinking water for administration.

The antiprotozoal agent of the present invention is prepared, for example, by diluting a compound of the general formula (1) or its physiologically acceptable salt, independently or in a mixed state, with a solid or liquid carrier, by undiluting them, or by stabilizing them by coating and the like to formulate powders, dusts, granules, tablets, solutions, emulsions, pastes, capsules, premixed, injections and the like. The antiprotozoal agent of the present invention is also prepared by dispersing directly the compound of the general formula (1) or its physiologically acceptable salt in feed, drink and the like, or by incorporating therein after dispersed in a carrier. The carrier may be any one, as long as it is physiologically harmless per se. The carriers which function as feed or a component of feed are preferable. The solid carriers include, for example, lactose, sucrose, starch, wheat meal, corn meal, wheat bran, soybean cake, extracted rice bran, rape seed cake, soybean crude meal, cellulose yeast, fish meal, peanut meal, shell powder and calcium carbonate. Examples of the liquid carriers include water, physiological saline and physiologically acceptable organic solvents. In addition, other suitable adjuvants such as emulsifiers, dispersants, suspension aids, wetting agents, thickening agents, gel forming agents and solubilizers may be added in suitable amounts. There may be further incorporated preservatives, fungicides, colorants, aromatics, antibacterial agents, antibiotics, enzyme preparations, lactobacillus preparations, antifebriles, analgesics, antiphlogistics and so on, and other agents for protozoal disease may also be compounded in combination as long as they are different from the compound of the present invention in mechanism of action. Furthermore, various vitamins, minerals and amino acids may be incorporated.

The antiprotozoal agents of the present invention are administered to animals such as mammals, birds, fish or insects, for the purpose of prophylaxis or/and treating protozoal disease. Since in the live-stock industry, domestic animals are usually bred or farmed in groups, it is also included in the scope of this invention of course to administer the antiprotozoal agents of the present invention to infected individuals isolated from the group or to the whole of the group through feed, drinking water and the like, when it has been confirmed that some animals in the group are attacked with protozoal disease.

The antiprotozoan composition of this invention may contain one or more species of the compound or salt of this invention. Furthermore, the composition may contain other drugs for improving the general condition of animals or drugs for prophylaxis or therapy of the indicated disease. It can be used in combination with such drugs unless adverse interactions or dilution of efficacy is foreseen.

The antiprotozoal composition of this invention should contain compound (I) or a physiologically acceptable salt thereof in a concentration of about 0.01 ppm to about 1%, preferably about 0.1 ppm to about 0.1%. In the case of a preparation for extemporaneous dilution, it is prepared so as to contain the active drug in a concentration of about 0.01 to about 90% or, preferably, about 0.1 to about 30%.

Generally, the antiprotozoal composition of this invention can be administered in a daily dose of about 0.01 to about 50 mg/kg body weight, preferably about 0.1 to about 5 mg/kg body weight, as compound (I) or a salt. By way of illustration, the antiprotozoan composition of this invention can be admixed into the animal ration or diet at the level of, as compound (I) or a salt thereof, about 0.01 to about 100 ppm, preferably about 0.1 to about 50 ppm. The resulting ration can be used for both therapeutic and prophylactic purposes. Such a ration can be generally prepared by manufacturing a concentrate or premix containing about 0.5 to about 30 weight %, preferably about 1 to about 20 weight %, of compound (I) or salt with a feed excipient and blending it with a regular feed. The excipient mentioned just above may for example be a corn meal or corn-soya meal containing a small quantity of some dust-preventive edible oil such as corn oil or soybean oil or a mineral salt. The resulting premix is evenly admixed into a regular animal diet for administration.

For the treatment or prevention of coccidiosis in poultry, particularly in chickens, quails, ducks, wild ducks, geese, and turkeys, generally about 0.01 to about 100 ppm, preferably about 0.1 to about 50 ppm, of compound (I) or salt is administered as previously mixed with suitable edible materials such as nutrient feeds. The drug can be added to drinking water for ingestion.

For the treatment of animals, e.g. for the therapy of coccidiosis or toxoplasmosis, compound (I) or a physiologically acceptable salt thereof is administered in a daily dose of about 0.5 to about 100 mg/kg body weight. Depending on the body weight of animals, therapeutic regimen, species or breeds of animals, individual responses to the antiprotozoan drug, dosage form or formulation, timing and intervals of administration, etc., it may at times be necessary to depart from the above-mentioned dosage range. Thus, a reduced dose may prove effective in some cases, while an increased dose may be necessary in other cases. For massive administration, the daily dosage may be advantageously administered in divided doses.

The treatment of fish is carried out by the oral route, such as through feed, or by the short-time "drug bath" method which comprises transferring the fish from the farming-pond to a tank filled with drug solution (drug bath) and keeping them therein for a predetermined time (ranging from a few minutes to several hours).

However, a temporary or permanent treatment of the whole habitat (for example a pool, aquarium, tank or pond) can also be instituted.

In such cases, compound (I) or a physiologically acceptable salt is applied in a form suited to the particular situation. The concentration of the antiprotozoal agent of this invention may range from about 1 ppm to about 10 weight per volume %.

For the "drug bath" treatment or the omnibus habitat treatment (pool treatment) of fish, it is preferable to employ a solution of the antiprotozoan drug of this invention in a polar solvent or solvent mixture which can be diluted or suspended with water.

To prepare such a solution, compound (1) or a physiologically acceptable salt is dissolved or suspended in a polar water-soluble vehicle. It is preferable that after addition of the compound (1) or/and physiologically acceptable salt the vehicle shows a pH range of 7 to 10, especially about 8 to 10.

Since administration of the compound of this invention controls parasitic protozoan to thereby decrease the incidence of the associated diseases and death and improve the growth retardation and deteliorated general condition, the invention is useful for preventing the decrease of yields in the production of meat, milk, furs, eggs, honey and so on. Moreover, the invention also contributes remarkably to a safe husbandry of ornamental animals and pets.

The following examples, test examples and formulation examples are intended to describe this invention in further detail and should by no means be construed as defining the scope of the invention. The chemical structure of the compound obtained in the following Examples are shown in the Table 3.

### Test Example 1 Effect on Biological Test (1)

The anticoccidal effect of the compound of this invention was evaluated in chickens. Using 9-day-old male white Leghorn chicks in groups of 3, the animals in all groups except the non-infected, untreated control group were orally inoculated with 5x10⁴ sporulated oocysts/bird of the laboratory standard strain of Eimeria tenella. As the drug, a dried, crushed batch of the compound of this invention was admixed with 31.3 ppm of the standard basal ration (SDL No. 1; Nippon Haigo Shiryo Co., Ltd.) and the chicks were allowed free access to the resulting diet for 9 consecutive days beginning 24 hours before the infection until day 8 after the infection. During the feeding period, the body weight gain of each chick was determined. Furthermore, the number of bloody droppings was counted and the count of excreted oocysts was taken to evaluate the anticoccidial effect of the drug. The results are shown in Table 1. In the table, Compound No. corresponds to the Compound No. in Table 3.

**Table 1**

| Compound No. | Relative body weight gain (%)¹⁾ | Number of bloody droppings²⁾ | OPG³⁾ (log) |
|---|---|---|---|
| Non-infected/treatment group | 100 | 0 | ND⁴⁾ |
| Infected/untreated control group | 40.2 | 3.0 | 5.2 |
| 1 | 100 | 0 | ND |
| 2 | 102.9 | 1.25 | ND |
| 3 | 96.1 | 0 | ND |
| 4 | 91 | 0 | ND |
| 5 | 100 | 0 | ND |
| 6 | 100 | 0 | ND |
| | | | |
| 22 | 111.9 | 0 | ND |
| 38 | 100 | 0 | ND |
| 39 | 100 | 0 | ND |
| 40 | 100 | 0 | ND |
| 43 | 100 | 0 | ND |
| 44 | 100 | 0 | ND |
| 45 | 104.6 | 0 | ND |

| | | | |
|---|---|---|---|
| 1) Relative body weight gain = $\frac{\text{Average body weight gain in treatment group}}{\text{Average body weight gain in non-infected control group}}$ x 100 | | | |
| 2) Number of bloody droppings: The number of blood droppings per bird as detected on the paper set under the floor-net on the peak day of excretion from the intestine of the chick. | | | |
| 3) OPG: The number of oocysts excreted in 1 gram of feces (on day 7 after infection) | | | |
| 4) ND: Not detected. | | | |

It is apparent from the data in Table 1 that compared with the infected group, the groups treated with the compound of this invention invariably showed a relative body weight gain, indicating that the compound of this invention has excellent anticoccidial activity.

### Test Example 2 Effect on the Biological Test (2)

The anticoccidal effect of the compound of the invention was evaluated following the method in the Test Example 1 by administration of the standard ration containing 4 ppm of the compound. The results are shown in Table 2.

**Table 2**

| Compound No. | Relative body weight gain (%) | Number of bloody droppings) | OPG (log) |
|---|---|---|---|
| Non-infected/treatment group | 100 | 0 | ND |
| Infected/untreated control group | 40.2 | 3.0 | 5.2 |
| 53 | 92.3 | 0 | ND |
| | | | |
| 54 | 90.7 | 0 | ND |
| 55 | 92.2 | 0 | ND |
| 56 | 95.7 | 0 | ND |
| 57 | 95.8 | 0 | ND |
| 58 | 95.7 | 0 | ND |
| 59 | 92.2 | 0 | ND |
| 60 | 90.3 | 0 | ND |
| 61 | 90.3 | 0.7 | ND |
| 62 | 94.5 | 0 | ND |
| 65 | 90.0 | 0 | ND |
| 66 | 98.4 | 0 | ND |
| 67 | 92.6 | 0 | ND |
| 68 | 97.0 | 0 | ND |
| 69 | 94.1 | 0.2 | ND |

### Reference Example 1

### 3,5-dichloro-4-(4'-chloro-1-methoxycarbonyl)benzyl nitrobenzene

In 100 ml acetonitrile was dissolved 4.00 g p-chlorophenyl acetate, 4.54 g 3,4,5-trichloronitrobenzen and 2.60 g 1,1,3,3-tetramethyl guanidine followed by refluxtion for 8 hours and concentrated to dryness. The residue was dissolved with 100 ml toluene, washed with 100 ml iced water and 100 ml cold water, dried over MgSO₄ and concentrated. After the concentrate was added ethanol, 6.82 g of the title compound was filtlated as crystals. m.p. 92-93°C

### Reference Example 2

### 3,5-dichloro-4-(4'-chloro-methoxycarbonyl)benzylaniline

In 50 ml of ethanol was dissolved 6.00 g 3,5-dichloro-4-(4'-chloro-methoxycarbonyl)benzylnitrobenzen prepared according to Reference Example 1 followed by addition of 5-fold molar SnCl₂ and refluxed for 2 hours after the reaction solution was concentrated, powdered in 1 l iced water and extracted with 100 ml of ethyl acetate, after the solution was made to be alkaline by adding 10% NaOH. The extract was washed with water, dried over MgSO₄ and recrystalized with ethanol to provide 5.12 g of the title compound. m.p. 151-152°C

### Reference Example 3

### 3,5-dichloro-4-(4'-chloro-1-methoxycarbonyl)benzylphenylhydrazine

In the mixture of 100 ml of acetic acid and 30 ml of hydrochloride was dissolved 5.00 g of 3,5-dichloro-4-(4'-chloro-1-methoxycarbonyl)benzylaniline prepared according to Reference Example 2 followed by addition of 10 ml of 2-fold molar sodium nitrate solution dropwise with stirring at 5 - 10°C.

After completion of the addition, the solution was reacted at 10 - 20°C for 2 hours and further reacted at 10-20°C for 2 hours after addition of 5-fold molar SnCl₂ dissolved in 50 ml hydrochloride dropwise with stirring at 5 - 10°C. The crystals thus obtained was suspended in iced water and extracted with ethyl acetate. The extract was dried over MgSO₄, concentrated and recrystalized to give 4.20 g of the title compound. m.p. 113-114°C

### Reference Example 4

### 4-(4'-chloro-1-methoxycarbonyl)benzyl-3-trifluoromethyl aniline

Starting with 4-(4'-chloro-1-methoxycarbonyl)benzyl-3-trifluoromethylnitrobenzene, the title compound was synthesized in otherwise a similar manner as Reference Example 2. m.p. 103-104°C

### Reference Example 5

### 4-(4'-chloro-1-methoxycarbonyl)benzyl-3-trifluoromethylphenyl hydrazine

Starting with 4-(4'-chloro-1-methoxycarbonyl)benzyl-3-trifluoromethylaniline prepared according to Reference Example 4, in otherwise a similar manner as Reference Example 3. m.p. 95-97°C

### Example 1

### 2-(3,5-Dichlorophenyl)-5-methoxy-2,3-dihydro-1,2,4-triazin-3-one (Compound No. 16)

In THF was dissolved 2.77 g of 2-(3,5-dichlorophenyl)-5-chloro-2,3-dihydro-1,2,4-triazin-3-one followed by addition of sodium methoxide in an equimolar amount to the starting compound, and the mixture was stirred at room temperature for 1 hour. After completion of this reaction, the reaction mixture was concentrated under reduced pressure and ice-water was added to the residue. The resulting crystals were collected by filtration, dried and dissolved in 100 ml of chloroform. The chloroform solution was dried over anhydrous magnesium sulfate, concentrated and purified by column chromatography (Merck Silica Gel 60; dichloromethane-methanol = 10:1) to provide 0.4 g of white crystals (m.p. 164°C).

| Elemental analysis for C₁₀H₇Cl₂N₃O₂ | | | |
|---|---|---|---|
| | C | H | N |
| Calcd. | 44.14; | 2.59; | 15.44 |
| Found | 43.92; | 2.61; | 15.31 |

- NMR [CDCl₃] δ:: 4.09 (s, 3H), 7.25-7.48 (m, lH), 7.51-7.75 (m, 2H)

### Example 2

### 2-[3,5-Dichloro-4-(4'-chloro-1-cyanobenzyl)phenyl]hexahydro-1,2,4-triazin-3-one (Compound No. 38)

To 50 ml of dichloromethane was added 2.07 g of 2-[3,5-dichloro-4-(4'-chloro-1-cyanobenzyl)phenyl]-1-(2-hydroxyethyl)semicarbazide as well as 2-fold molar of pyridine. The solution was cooled to 0 to 5°C and equimolar of trifluoroacetic anhydride was added dropwise with constant stirring. After completion of the dropwise addition, the reaction was further carried out under the same conditions for one hour. The dichloromethane was then removed by concentration and 50 ml of 1,4-dioxane was added to the concentrate. The mixture was refluxed for 4 hours, after which the solvent was distilled off and the residue was dissolved in chloroform. The chloroform solution was washed with iced water, dried over anhydrous magnesium sulfate and concentrated. This residue was further purified by column chromatography (Merck Silica Gel 60; chloroform) to provide 0.1 g of the title compound as a white substance melting at 138 - 139°C (dec.).

| Elemental analysis for C₁₇H₁₃Cl₃N₄O | | | |
|---|---|---|---|
| | C | H | N |
| Calcd. | 51.60; | 3.31; | 14.16 |
| Found | 51.53; | 3.36; | 14.14 |

- NMR [CDCl₃] δ:: 3.00-3.64 (m, 4H), 4.19 (t, J=6Hz, 1H), 5.69 (br, 1H), 6.09 (s, 1H), 7.29 (s, 4H), 7.92 (s, 2H)

### Example 3

### 2-[3,5-Dichloro-4-(4'-chloro-1-cyanobenzyl)phenyl]-2,3,4,5-tetrahydro-1,2,4-triazin-3-one (Compound No. 41)

Starting with 2.00 g of 2-[3,5-dichloro-4-(4'-chloro-1-cyanobenzyl)phenyl]-1-(2-hydroxymethylmethylidene semicarbazone, 0.38 g of the white title compound was synthesized in otherwise the same manner as Example 2. m.p. 166-167°C

| Elemental analysis for C₁₇H₁₁Cl₃N₄O | | | |
|---|---|---|---|
| | C | H | N |
| Calcd. | 51.86; | 2.82; | 14.23 |
| Found | 51.60; | 2.87; | 13.93 |

- NMR [CDCl₃] δ:: 4.00-4.20 (m, 2H), 6.14 (s, 1H), 6.45 (br, lH), 7.15 (br, lH), 7.30 (s, 4H), 7.72 (s, 2H)

### Example 4

### 2-[3,5-Dichloro-4-(4-chloro-1'-cyanobenzyl)phenyl]-4-methylhexahydro-1,2,4-triazin-3-one (Compound No. 40)

In 50 ml of dimethylformamide was dissolved 1.18 g of l-methyl-l-(2,2-diethoxyethyl)-2-[3,5-dichloro-4-(4'-chloro-1-cyanobenzyl)phenyl]semicarbazide and the reaction was carried out at 140 - 145°C with stirring for 2 hours. The reaction mixture was then poured in 300 ml of iced water and extracted with 200 ml of chloroform. The extract was dried over anhydrous magnesium sulfate, concentrated to dryness. The residue was reducted with LiAlH₄ in THF and purified by column chromatography (Merck Silica Gel 60; chloroform) to provide 0.1 g of the title compound. m.p. 137 - 138°C.

| Elemental analysis for C₁₈H₁₅Cl₃N₄O | | | |
|---|---|---|---|
| | C | H | N |
| Calcd. | 52.77; | 3.69; | 13.68 |
| Found | 52.74; | 3.62; | 13.68 |

- NMR [CDCl₃] δ:: 3.00 (s, 3H), 3.20-3.60 (m, 4H), 4.25 (br, lH), 6.08 (s, 1H), 7.28 (s, 4H), 7.89 (s, 2H)

### Example 5 (Reference Example)

### 2-(3,5-Dichlorophenyl)-2,3,4,5-tetrahydro-1,2,4-triazin-3-one-5-thione (Compound No. 7)

To 100 ml of toluene was added 2.58 g of the starting compound 3,5-dione as well as 1/2 equivalent of Lawesson's reagent and the mixture was refluxed for 5 hours. The reaction mixture was then concentrated and purified by column chromatography (Merck Silica Gel 60; chloroform) to provide 0.9 g of light-yellow crystals, m.p. 200 - 201°C.

| Elemental analysis for C₉H₅Cl₂N₃OS | | | |
|---|---|---|---|
| | C | H | N |
| Calcd. | 39.43; | 1.84; | 15.33 |
| Found | 39.45; | 1.97; | 15.05 |

- NMR [CDCl₃] δ:: 7.67 (s, 3H), 7.84 (s, 1H), 13.80 (br, 1H)

### Example 6

### 2-(3,5-Dichlorophenyl)-2,3,4,5-tetrahydro-1,2,4-triazin-3-one (Compound No. 1)

In 50 ml of 70% ethanol was dissolved 2.74 g of Compound No. 7, synthesized in Example 5, followed by addition of 10 equivalents of activated Raney nickel. The mixture was stirred at room temperature overnight, after which the insoluble matter was filtered off. The filtrate was concentrated under reduced pressure and the residue was dissolved in 100 ml of ethyl acetate. This solution was dehydrated over anhydrous magnesium sulfate, concentrated and the resulting red-brown oil was purified by column chromatography (Merck Silica Gel 60; chloroform) to provide 1.0 g of the title compound as a pale yellow substance. m.p. 179 - 181°C

| Elemental analysis for C₉H₇Cl₂N₃O | | | |
|---|---|---|---|
| | C | H | N |
| Calcd. | 44.29; | 2.89; | 17.22 |
| Found | 44.59; | 2.98; | 17.41 |

- NMR [CDCl₃] δ:: 4.05-4.20 (m, 2H), 6.30-6.60 (br, 1H), 7.00-7.30 (m, 2H), 7.52 (d, J=2Hz, 2H)

### Example 7

### 2-(3,5-Dichlorophenyl)-5-phenyl-2,3-dihydro-1,2,4-triazin-3-one (Compound No. 27)

In 50 ml of dioxane was dissolved 2.20 g of 2-(3,5-dichlorophenyl)semicarbazide followed by addition of 1.52 g of phenylglyoxal monohydrate, and the mixture was refluxed for 5 hours. The reaction mixture was then concentrated and the residue was recrystallized from acetonitrile to provide 1.34 g of the title compound. m.p. 160 - 161°C.

| Elemental analysis for C₁₅H₉Cl₂N₃O | | | |
|---|---|---|---|
| | C | H | N |
| Calcd. | 56.63; | 2.85; | 13.21 |
| Found | 56.52; | 2.78; | 13.34 |

- NMR [CDCl₃] δ:: 7.38 (t, J=2Hz, 1H), 7.45-7.70 (m, 3H), 7.75 (d, J=2Hz, 2H), 8.05-8.30 (m, 2H), 8.48 (s, 1H)

### Example 8

Starting with 2-(3,5-dichlorophenyl)-1-(2-hydroxy-2-methylpropylidene)semicarbazone, Compound 2 was synthesized in otherwise a similar manner as Example 2. m.p. 120-122°C

### Example 9

Starting with 2-(3,5-dichlorophenyl)-1-(2-hydroxy-2-methylthioethylidene)semicarbazone, Compound 3 was synthesized in otherwise a similar manner as Example 2. m.p. 115-116°C

### Example 10

Starting with 2-(3,5-dichlorophenyl)-1-(2,2-dimethylthio-2-hydroxyethyl)semicarbazide, Compound 4 was synthesized in otherwise a similar manner as Example 2. m.p. 151-152°C

### Example 11

Starting with Compound 1, Compound 5 was synthesized in otherwise a similar manner as Example 4. m.p. 148-149°C

### Example 12

Starting with 2-(3,5-dichlorophenyl)-6-methyl-2,3,4,5-tetrahydro-1,2,4-triazin-3-one-5-thione, Compound 6 was synthesized in otherwise a similar manner as Example 6. m.p. 164-165°C

### Example 13 (Reference example)

Starting with 2-(3,5-dichlorophenyl)-4-methylhexahydro-1,2,4-triazin-3,5-dione, Compound 8 was synthesized in otherwise a similar manner as Example 5. m.p. 149-150°C

### Example 14 (Reference Example)

Starting with 2-(3,5-dichlorophenyl)-hexahydro-1,2,4-triazin-3,5-dione, Compound 9 was synthesized in otherwise a similar manner as Example 5. m.p. 221-223°C (dec)

### Example 15

Starting with 2-(3,5-dichlorophenyl)-1-(2-hydroxy-2-phenylethyl)semicarbazide, Compound 10 was synthesized in otherwise a similar manner as Example 2. m.p. 164-165°C

### Example 16

Starting with 2-(3,5-dichlorophenyl)-1-(2-hydroxy-2-phenylethylidene)semicarbazone, Compound 11 was synthesized in otherwise a similar manner as Example 2. m.p. 139-140°C

### Example 17

Starting with Compound 8, Compound 12 was synthesized in otherwise a similar manner as Example 6. m.p. 210-211°C

### Example 18

Starting with 2-(3,5-dichlorophenyl)-6-methylhexahydro-1,2,4-triazin-3-one-5-thione, Compound 13 is synthesized in otherwise a similar manner as Example 6.

### Example 19

Using methyl mercaptan in place of sodium methoxide, Compound 14 was synthesized in otherwise a similar manner as Example 1. m.p. 190-191°C

### Example 20 (Reference Example)

Using benzyl mercaptan in place of sodium methoxide, Compound 15 was synthesized in otherwise a similar manner as Example 1. m.p. 152-153°C

### Example 21

### 5-chloro-2-(3,5-dichlorophenyl)-2,3-dihydro-1,2,4-triazine-3-one (compound No. 17)

In 30 ml of dichloromethane suspended with 1.00 g of 2-(3,5-dichlorophenyl)-2,3,4,5-tetrahydro-1,2,4-triazine-3,5-dione followed by addition of 2-fold morlar each of carbone tetrachloride and triphenylphosphine and refluxed for 12 hours. After the completion of the reaction, the resulted solution were purified by colomun chlomatography (Merck Silica Gel 60 dichloromethane-carbontetrachloride=2:1). m.p. 148-149°C
- NMR [CDCl₃] δ:: 7.42(t,J=2Hz,1H), 7.66(d,J=2Hz,2H), 7.91(s,1H)

### Example 22

Using potassium fluoride in place of sodium methoxide, Compound 18 was synthesized in otherwise a similar manner as Example 1. m.p. 93-95°C

### Example 23 (Reference Example)

Using p-chlorothiophenol in place of sodium methoxide, Compound 19 was synthesized in otherwise a similar manner as Example 1. m.p. 176-178°C

### Example 24

Using t-butyl mercaptan in place of sodium methoxide, Compound 20 was synthesized in otherwise a similar manner as Example 1. m.p. 97-99°C

### Example 25

Using potassium t-butoxide in place of sodium methoxide, Compound 21 was synthesized in otherwise a similar manner as Example 1. m.p.91-92°C

### Example 26

Using phenol in place of sodium methoxide, Compound 22 was synthesized in otherwise a similar manner as Example 1. m.p. 126-127°C

### Example 27 (Reference Example)

Using cyclopropylmethanol in place of sodium methoxide, Compound 23 was synthesized in otherwise a similar manner as Example 1. m.p. 68-69°C

### Example 28 (Reference Example)

Using 2-fluoroethanol in place of sodium methoxide, Compound 24 was synthesized in otherwise a similar manner as Example 1. m.p. 110-111°C

### Example 29 (Reference Example)

Using 2,2,2-trifluoroethanol in place of sodium methoxide, Compound 25 was synthesized in otherwise a similar manner as Example 1. m.p. 80-81°C

### Example 30 (Reference Example)

Using 1,3-dimercaptopropane in place of sodium methoxide, Compound 26 was synthesized in otherwise a similar manner as Example 1. m.p. 195-196°C

### Example 31

Starting with 2-(3,5-dichlorophenyl)-5-chloro-2,3,4,5-tetrahydro-1,2,4-triazin-3-one and using methyl mercaptan in place of sodium methoxide, Compound 3 was synthesized in otherwise a similar manner as Example 1. m.p. 115-116°C.

### Example 32

Using 2-(3,5-dichlorophenyl)-1-benzoylmethylidenesemicarbazone, Compound 27 was synthesized in otherwise a similar manner as Example 2. m.p. 160-161°C

### Example 33

Using 2-(3,5-dichlorophenyl)-1-benzoyl-1-phenylmethylidenesemicarbazone, Compound 28 was synthesized in otherwise a similar manner as Example 2. m.p. 158-159°C

### Example 34

Starting with 2-(3,5-dichlorophenyl)-5-chloro-1,2,3,6-tetrahydro-1,2,4-triazin-3-one and using methyl mercaptan in place of sodium methoxide, Compound 29 was synthesized in otherwise a similar manner as Example 1. m.p. 133-134°C.

### Example 35 (Reference Example)

Using 2-phenyl-2,3,4,5-tetrahydro-1,2,4-triazin-3,5-dione, Compound 30 was synthesized in otherwise a similar manner as Example 5. m.p. 183-184°C

### Example 36

Starting with 2-phenyl-5-chloro-2,3-dihydro-1,2,4-triazin-3-one and using methyl mercaptan in place of sodium methoxide, Compound 31 was synthesized in otherwise a similar manner as Example 1. m.p. 115-116°C

### Example 37

Starting with 2-phenyl-5-chloro-2,3-dihydro-1,2,4-triazin-3-one, Compound 32 was synthesized in otherwise a similar manner as Example 1. m.p. 102-103°C

### Example 38 (Reference Example)

Using 2-(4-chlorophenyl)-2,3,4,5-tetrahydro-1,2,4-triazin-3,5-dione, Compound 33 was synthesized in otherwise a similar manner as Example 5. m.p. 198-199°C

### Example 39 (Reference example)

Starting with 2-(4-chlorophenyl)-5-chloro-2,3-dihydro-1,2,4-triazin-3-one and using methyl mercaptan in place of sodium methoxide, Compound 34 was synthesized in otherwise a similar manner as Example 1. m.p. 168-169°C

### Example 40 (Reference esample)

Using 2-(4-chlorophenyl)-5-chloro-2,3-dihydro-1,2,4-triazin-3-one, Compound 35 was synthesized in otherwise a similar manner as Example 1. m.p. 151-152°C

### Example 41 (Reference example)

Using 2-[3,5-dichloro-4-(4'-chloro-1-cyanobenzyl)phenyl]-2,3,4,5-tetrahydro-1,2,4-triazin-3,5-dione, Compound 36 was synthesized in otherwise a similar manner as Example 5. m.p. 249-250°C

### Example 42 (Reference Example)

Using 2-[3,5-dichloro-4-(4'-chloro-1-cyanobenzyl)-phenyl]hexahydro-1,2,4-triazin-3,5-dione, Compound 37 was synthesized in otherwise a similar manner as Example 5. m.p. 215-216°C (dec)

### Example 43

Using 2-[3,5-dichloro-4-(4'-chloro-1-cyanobenzyl)-phenyl]-4-methyl-2,3,4,5-tetrahydro-1,2,4-triazin-3-one-5-thione, Compound 40 was synthesized in otherwise a similar manner as Example 6. m.p. 137-138°C

### Example 44

Using 2-[3,5-dichloro-4-(4'-chloro-1-cyanobenzyl)-phenyl]-5-chloro-2,3-dihydro-1,2,4-triazin-3-one, Compound 42 was synthesized in otherwise a similar manner as Example 1. m.p. 193-194°C

### Example 45

Compound 36 was dissolved in THF and reacted with equimolar of methyl iodide to provide Compound 43. m.p. 204-205°C

### Example 46

Compound 43 was dissolved in dichloromethane and reacted with chlorine gas to provide Compound 44. m.p. 184-185°C

### Example 47

### 2-(3,5-dichlorophenyl)-2,3,4,5-tetrahydro-1,2,4-triazin-3-one (compound No. 1)

In 20 ml acetonitrile was dissolved 1.77 g of 3,5-dichlorophenylhydrazine followed by addition of 1.10 g benzaldehyde dropwise with constant stirring at 10 to 20°C and obtained 2.70 g hydrazone. In 30 ml of acetonitrile was dissolved 1.33 g of the hydrazone followed by the addition of 3-fold mole pyridine, and 0.6-fold mole trichlolomethylchloroformate was added dropwise to the mixture with constant stirring at 0 to 10°C. After completion of the dropwise addition, the mixture was stirred at 20 to 25°C for 1 hour.

In 30 ml acetonitrile was dissolved 0.60 g of aminoacetoaldehyde dimethyl acetate followed by the addition of the above prepared reaction solution with constant stirring at 5 to 10°C. After completion of the dropwise addition, the reaction solution was stirred at 20 to 25°C for 3 hours. After concentrated the thus obtained reaction solution, iced water was added and extracted with 50 ml of dichloromethane. After dried over anhydrous magnesium sulfate, the extract was concentrated and purified by column chromatography (Merck Silica Gel 60; chloroform) to provide 1.54 g of 1-benzilidene-2-(3,5-dichlorphenyl)-4-(2,2-dimethoxyethyl)semicarbazone as white crystals (m.p. 119-120°C).

In 20 ml acetonitrile was dissolved 1.00 g of the thus obtained semicarbazone, followed by heat-reaction at 50 to 60°C with 0.1 ml conc. hydrochloride for 20 minutes. After completed the reaction, the resulted crystal was collected by filtration and washed with water. Thus obtained crystal was recrystallized from ethyl acetate to provide 0.72 g the title compound.
m.p. 179-181°C
N

### Example 48

### 2-[3,5-dichloro-4-(4-chlorophenylthio)phenyl]-6-methyl-2,3,4,5-tetrahydro-1,2,4-triazin-3-one (compound No. 45)

After refluxed 1.60 g of 3,5-dichloro-4-(4-chlorophenylthio)phenylhydrazone and 0.9 g of N-acetonylphenylcarbamate in 30 ml toluene, 0.8 g of 1,8-diazabicyclo[5,4,0]-7-undensen was added therein and refluxed 2 more hours. After completed the reaction, the solution was concentrated, added ice water and extracted from 50 ml of dichloromethane. After dried over with anhydrous magnesium sulfate, the extract was concentrated to dryness and purified by column chromatography (Merck Silica Gel 60; chloroform) to provide 0.34 g of the title compound as a white substance. m.p. 258-259°C
- NMR (d₆-DMSO)δ:: 2.04(s,3H), 3.98(br-d,J=2Hz,2H), 7.22(g,J=8Hz,4H), 7.80(br,1H), 7.88(s,2H)

### Example 49

### 2-[3,5-dichloro-4-(4-chlorobenzoyl)phenyl]-6-methyl-2,3,4,5-tetrahydro-1,2,4-triazin-3-one (compound No. 46)

Starting with 3,5-dichloro-4-(4-chlorobenzoyl)phenylhydrazine, the title compound was synthesized in otherwise a similar manner as Example 48. m.p. 257-259°C

### Example 50

### 4-acetyl-2-[3,5-dichloro-4-(4-chlorobenzyl)phenyl]-6-methyl-2,3,4,5-tetrahydro-1,2,4-triazin-3-one (compound No. 47)

The title compound was obtained by acetylating the compound 46 prepared according to Example 49 with acetic acid anhydride in toluene. m.p. 101-102°C

### Example 51

### 2-(3,5-dichlorophenyl)-1-methyl-hexahydro-1,2,4-triazin-3-one(conpound No. 49)

Chrystals obtained by reacting 1-aminoacethyl-1-methyl-2-(3,5-dichrolophenyl) hydrazine with phenyl chloroformate under the existance of a base was dissolved in THF followed by reduction with NaBH₄ to provide the title compound m.p. 150-151°C.

### Example 52

### 6-chloro-2-(3,5-dichlorophenyl)-2,3,4,5-tetrahydro-1,2,4-triazin-3-thione (compound No. 49)

In 30 ml dichloromethane 1.33 g of 1-(3,5-dichlorophenyl)-2-benzyldenhydrazone and 5-fold molar pyridine were dissolved followed by addition of 2-fold molar thiophosgen dropwise with constant stirring at 5 to 10°C. After completed the dropwise addition, the resulting solution was stirred at 20 to 25 for 1 hour and added the equimolar ethylglcinate hydrochloride salt with stirring at 5 to 10°C. After two hour reaction, the reaction solution was washed with water and concentrated to dryness. The residue was dissolved in 30 ml of acetonitrile and reacted with 0.1 ml conc. hydrochloride at 50 to 60°C for 20 minutes to provide 2-(3,5-dichlorophenyl)-hexahydro-1,2,4-triazin-6-one-3-thion. The resulted compound was chlorized by known manner to provided the title compound. m.p. 207-208°C

### Example 53

### 2-[3,5-dichlorophenyl)-1,5-dimethylhexahydro-1,2,4-triazin-3-one (compound No. 50)

Reacting 1-(2-aminopropyl)-1-methyl-2-(3,5-dichlorophenyl)hydrazine with phenyl chloroformate under presence of base to provide the title compound. m.p. 129-130°C

### Example 54

### 2-(3,5-dichlorophenyl)-1-methyl-hexahydro-1,2,4-triazin-3-thione (compound No. 51)

Starting with 1-(2-aminoethyl)-2-(3,5-dichlorophenyl)methylhydrazide and thiophosgene, the title compound was synthesized in otherwise a similar manner as Example 52. m.p. 240-241°C

### Example 55

### 2-[3-chloro-4-(4'-chloro-1-cyanobenzyl)-5-methylphenyl]-2,3,4,5-tetrahydro-1,2,4-triazin-3-one (compound No. 52)

Starting with 3-chloro-4-(4'-chloro-1-cyanobenzyl)-5-methylphenylhydrazine, the title compound was synthesized in otherwise a similar manner as Example 47. m.p. 192-193°C

### Example 56

### 2-[3-Chloro-4-(4'-chloro-1-cyanobenzyl)-5-methylphenyl]-hexahydro-1,2,4-triazin-3-one (compound No. 53)

Reducing the compound 52 prepared according to Example 55 with LiAlH₄ in THF to provide the title compound. m.p. 201-202°C

### Example 57 (Reference Example)

### 2-[3-chloro-4-(4'-chloro-1-cyanobenzyl)-5-methylphenyl]-2,3,4,5-tetrahydro-1,2,4-triazin-3-one-5-thione (compound No. 54)

Starting with 2-[3-chloro-4-(4'-chloro-1-cyanobenzyl)-5-methylphenyl] -2,3,4,5-tetrahydro-1,2,4-triazine-3,5-dione, the title compound was synthesized in otherwise a similar manner as Example 5. m.p. 234-236°C
- NMR (d₆-DMSO)δ:: 2.41(s,3H), 6.36(s,lH), 7.17-7.70(m,6H), 7.87(s,lH), 13.86(br,lH)

### Example 58 (Reference Example)

### 2-[3-chloro-4-(4'-chloro-1-cyanobenzyl)-5-methylphenyl]hexahydro-1,2,4-triazine-3-one-5-thione (compound No. 55)

Reducing the compound 54 prepared according to Example 57 with LiAlH₄ in THF to provide the title compound. m.p. 217-218°C

### Example 59

### 2-[3,5-dichloro-4-(4'-chloro-1-benzoyl)phenyl]-2,3,4,5-tetrahydro-1,2,4-triazin-3-one (compound No. 56)

Starting with 3,5-dichloro-4-(4'-chlorobenzoyl)phenylhydrazine, the title compound was synthesized in otherwise a similar manner as Example 47.
m.p. 212-213°C

### Example 60

### 2-[3,5-dichloro-4-(4'-chloro-1-hydroxybenzyl)phenyl]-2,3,4,5-tetrahydro-1,2,4-triazin-3-one (compound No. 57)

Reducing the compound 56 prepared according to Example 59 with LiAlH₄ in THF to provide the title compound. m.p. 115-116°C

### Example 61

### 2-[3-chloro-4-(4-chlorobenzoyl)phenyl]-2,3,4,5-tetrahydro-1,2,4-triazin-3-one (compound No. 58)

Starting with 3-chloro-4-(4-chlorobenzoyl)phenylhydrazine, the title compound was synthesized in otherwise a similar manner as Example 47. m.p. 158-159°C

### Example 62

### 2-[3-chloro-4-(4'-chloro-l-hydroxybenzyl)phenyl]-2,3,4,5-tetrahydro-1,2,4-triazin-3-one (compound No. 59)

The compound 58 prepared according to Example 61 was reduced with LiAlH₄ in THF to provide the title compound. m.p. 135-136°C

### Example 63

### 2-[3,5-dichloro-4-(4-chlorophenylthio)phenyl]-2,3,4,5-tetrahydro-1,2,4-triazin-3-one (compound No. 60)

Starting with 3,5-dichloro-4-(4-chlorophenylthio)phenylhydrazine, the title compound was synthesized in otherwise a similar manner as Example 47. m.p. 224-225°C

### Example 64

### 2-[4-(4-chlorobenzyl)-3-chlorophenyl]-2,3,4,5-tetrahydro-1,2,4-triazin-3-one (compound No. 61)

Starting with 3-chloro-4-(4-chlorobenzyl)phenylhydrazine, the title compound was synthesized in otherwise a similar manner as Example 47. m.p. 196-197°C

### Example 65

### 2-[3-chloro-4-(2-chloropyridin-5-yl-cyanomethyl)-5-methylphenyl]-2,3,4,5-tetrahydro-1,2,4-triazin-3-one (compound No. 62)

Starting with 3-chloro-4-(2-chloropyridin-5-yl-cyanomethyl)-5-methylphenylhydrazine, the title compound was synthesized in otherwise a similar manner as Example 47. m.p. 211-212°C

### Example 66

### 2-[3-chloro-4-(2-chlorothiazol-5-yl-cyanomethyl)-5-methylphenyl]-2,3,4,5-tetrahydro-1,2,4-triazin-3-one (compound No. 63)

Starting with 3-chloro-4-(2-chlorothiazol-5-yl-cyanomethyl)-5-methylphenylhydrazine, the title compound was synthesized in otherwise a similar manner as Example 47. m.p. 238-239°C

### Example 67

### 2-[3-chloro-4-(1-methylimidazol-2-yl-thio)-5-methylphenyl]-2,3,4,5-tetrahydro-1,2,4-triazin-3-one (compound No. 64)

Starting with 3-chloro-4-(l-methylimidazol-2-yl-thio)-5-methylphenylhydrazine, the title compound was synthesized in otherwise a similar manner as Example 47. m.p. 173-174°C

### Example 68

### 2-[3,5-dichloro-4-(4'-chloro-1-fuluorobenzyl)phenyl]-2,3,4,5-tetrahydro-1,2,4-triazin-3-one (compound No. 65)

In 20 ml dichloromethane was suspended 0.38 g of the compound 57 followed by addition of 0.16 g of diethylaminosulfurtrifuluoride (DAST) dissolved in 5 ml dropwise with constant stirring at about -50°C. After reacted for 30 minutes under same condition, the reaction solution was further reacted at 20-25°C for 1 hour and concentrated. The residue was purified by column chromatography (Merck Silica Gel 60; chloroform) to provide 0.22 g of the title compound. m.p. 182-183°C
- NMR (CDCl₃)δ:: 4.09(t,J=2Hz,2H), 6.65(br,1H), 7.12(t,J=2H₂,1H), 7.22(d,J=46Hz,1H), 7.30(s,4H), 7.67(d,J=2Hz,2H)

### Example 69

### 2-[3-chloro-4-(4-chloro-l-fuluorobenzyl)pheny]-2,3,4,5-tetrahydro-1,2,4-triazin-3-one (compound No. 66)

Starting with 2-[3-chloro-4-(4'-chloro-1-hydroxybenzyl)phenyl-2,3,4,5-tetrahydro-1,2,4-triazin-3-one, the title compound was synthesized in otherwise a similar manner as Example 68. m.p. 124-125°C

### Example 70

### 2-[3,5-dichloro-4-(4'-chlorobenzyl)phenyl]-2,3,4,5-tetrahydro-1,2,4-triazin-3-one (compound No. 67)

Starting with 3,5-dichloro-4-(4-chlorobenzyl)phenylhydrazine the title compound was synthesized in otherwise a similar manner as Example 47. m.p. 196-197°C

### Example 71

### 2-[3,5-dichloro-4-(4-chlorophenylthio)phenyl]hexahydro-1,2,4-triazin-3-one (compound No. 68)

The compound 60 was reduced with LiAlH₄ in THF to provide the title compound. m.p. 242-243°C

### Example 72

### 2-[4-(4-chlorobenzyl)-3-chlorophenyl]hexahydro-1,2,4-triazin-3-one (compound No. 69)

The compound 61 was reduced with LiAlH₄ in THF to provide the title compound. m.p. 157-158°C

### Example 73

### 2-(2-chloro-4-trifluoromethylpyridin-6-yl)-2,3,4,5-tetrahydro-1,2,4-triazin-3-one (compound No. 70)

Starting with 2-chloro-4-trifluoromethylpyridin-6-yl-hydrazine, the title compound was synthesized in otherwise a similar manner as Example 47. m.p. 197-198°C

### Example 74

### 2-(2-chloro-4-trifluoromethyl-pyridin-6-yl)hexahydro-1,2,4-triazin-3-one (compound No. 71)

The compound 70 prepared according to Example 73 was reduced with LiAlH₄ in THF to provide the title compound. m.p. 191-192°C

### Example 75

### 2-[3,5-dichloro-4-[2-(4-chlorophenyl)-1-cyanoethyl]phenyl]-2,3,4,5-tetrahydro-1,2,4-triazin-3-one (compound No. 72)

Starting with 3,5-dichloro-4-[2-(4-chlorophenyl)-1-cyanoethyl]phenylhydrazine, the title compound was synthesized in otherwise a similar manner as Example 47. m.p. 213-213°C

### Example 76

### 2-[3,5-dichloro-4-(4'-chloro-1-methoxycarbonylbenzyl)phenyl]-2,3,4,5-tetrahydro-1,2,4-triazin-3-one (compound No. 73)

Starting with 3,5-dichloro-4-(4'-chloro-1-methoxycarbonylbenzyl)phenylhydrazine, the title compound was synthesized in otherwise a similar manner as Example 47. m.p. 206-207

### Example 77

### 2-[3,5-dichloro-4-(4'-chloro-1-hydroxymethylbenzyl)phenyl]hexahydro-1,2,4-triazin-3-one (compound No. 88)

The compound 73 prepared according to Example 76 was reduced with LiAlH₄ in THF to provide the title compound. m.p. 108-109°C
- NMR (CDCl₃) δ;: 3.00-3.60(br,4H), 4.00-4.70(br-m,4H), 5.12(t,lH), 5.88(br-s,lH), 7.19(s,4H), 7.73(s,2H)

### Example 78

### 2-[3-trifluoromethyl-4-(4'-chloro-1-methoxycarbonylbenzyl)phenyl]-2,3,4,5-tetrahydro-1,2,4-triazin-3-one (compound No. 74)

Starting with 3-trifluoromethyl-4-(4'-chloro-1-methoxycarbonylbenzyl)phenylhydrazine, the title compound was synthesized in otherwise a similar manner as Example 47. m.p. 118-119°C

### Example 79

### 2-[3-chloro-4-(4-chlorobenzyl)-5-methylphenyl]-2,3,4,5-tetrahydro-1,2,4-triazin-3-one (compound No. 89)

Starting with 3-chloro-4-(4-chlorobenzyl)-5-methylphenylhydrazine, the title compound was synthesized in otherwise a similar manner as Example 47.
m.p. 209-210°C

### Example 80

### 2-[3-chloro-4-(4-chlorobenzyl)-5-methylphenyl] hexahydro-1,2,4-triazin-3-one (compound No. 90)

The compound 89 prepared according to Example 79 was reduced with LiAlH₄ in THF to provide the title compound. m.p. 197-198°C

### Example 81

### 2-[3-chloro-4-(4'-chloro-1-fluorobenzyl)-5-methylphenyl)hexahydro-1,2,4-triazin-3-one (compound No. 91)

Starting with 3-chloro-4-(4'-chloro-1-fluorobenzyl)-5-methylphenylhydrazine, the title compound was synthesized in otherwise a similar manner as Example 47. m.p. 160-161°C (dec)

### Example 82

### 2-[3-chloro-4-(4'-chlorophenoxy)-5-methylphenyl]hexahydro-1,2,4-triazin-3-one (compound No. 92)

Starting with 3-chloro-4-(4'-chlorophenoxy)-5-methylphenylhydrazine, the title compound was synthesized in otherwise a similar manner as Example 47 and reduced with LiAlH₄ in THF. m.p. 188-189°C

### Example 83 (Reference Example)

### 2-[3-chloro-4-(4'-chlorobenzylthio)-5-methylphenyl]hexahydro-1,2,4-triazin-3-one (compound No. 93)

Starting with 3-chloro-4-(4'-chlorobenzylthio)-5-methylphenylhydrazine, the title compound was synthesized in otherwise a similar manner as Example 82. m.p. 165-167°C

### Example 84 (Reference Example)

### 2-{3-chloro-4-(2-(4'-chlorophenyl)-2-cyanovinylen]-5-methylphenyl}-2,3,4,5-tetrahydro-1,2,4-triazin-3-one (compound No. 94)

Starting with 3-chloro-4-[2-(4'-chlorophenyl)-2-cyanovinylene]-5-methylphenylhydrazine, the title compound was synthesized in otherwise a similar manner as Example 47. m.p. 250-251°C

### Example 85

### 2-[3-trifluoromethyl-4-(4'-chloro-1-hydroxymethylbenzyl)phenyl]-2,3,4,5-tetrahydro-1,2,4-triazin-3-one (compound No. 95)

The compound 74 prepared in Example 78 was reduced with LiAlH₄ in THF to provide the title compound.
m.p. 76-77°C
Further reduction of the title compound in THF with LiAlH₄, and obtained 2-[3-trifluoromethyl-4-(4'-chloro-1-hydroxymethylbenzyl)phenyl]-hexahydro-1,2,4-triazin-3-one (compound No. 77).
m.p. 87-88°C

### Example 86

### 2-[3,5-dichloro-4-(4'-chloro-1-cyanobenzyl)phenyl]-6-methyl-2,3,4,5-tetrahydro-1,2,4-triazin-3-one (compound No. 39)

Starting with 3,5-dichloro-4-(4'-chloro-1-cyanobenzyl)phenylhydrazine is disolved into toluene and heated with acetonylamine for two hours. After adding equimolar of phenyl chloroformate, the result solution was heated for farther two hours. After completion of the reaction, the reaction solution is cooled and filtrated to provide the title compound as crystal.

### Example 87

### 2-[3-trifluoromethyl-4-(4'-chloro-1-fluoromethylbenzyl)phenyl]-2,3,4,5-tetrahydro-1,2,4-triazin-3-one (compound No. 75)

Starting with compound 77, the title compound was synthesized in otherwise a similar manner as Example 68. m.p. 163-164°C

### Example 88

### 2-[3,5-dichloro-4-(4'-chloro-1-methylthiomethylbenzyl)phenyl]-2,3,4,5-tetrahydro-1,2,4-triazin-3-one (compound No. 96)

In methanol, 2-[3,5-dichloro-4-(4'-chloro-1-chloromethylbenzyl)phenyl]-2,3,4,5-tetrahydro-1,2,4-triazin-3-one is reacted with sodium thiomethoxide to provide the title compound.

### Example 89

### 2-[3,5-dichloro-4-(4'-chloro-1-dimethylaminomethylbenzyl)phenyl]-2,3,4,5-tetrahydro-1,2,4-triazin-3-one (compound No. 97)

In dimethylformamide, 2-[3,5-dichloro-4-(4'-chloro-1-chloromethylbenzyl)phenyl]-2,3,4,5-tetrahydro-1,2,4-triazin-3-one is reacted with dimethylamine solution.

### Example 90

### 2-[3,5-dichloro-4-(4'-chloro-1-trifluoromethylbenzyl)phenyl]-2,3,4,5-tetrahydro-1,2,4-triazin-3-one (compound No. 98)

Starting with 3,5-dichloro-4-(4'-chloro-1-trifluoromethylbenzyl)phenylhydrazine, the title compound is synthesized in otherwise a similar manner as Example 47.

### Example 91

### 2-[3,5-dichloro-4-(4'-chloro-1-hydroxymethylbenzyl)phenyl]-2,3,4,5-tetrahydro-1,2,4-triazin-3-one (compound 99)

Starting with 3,5-dichloro-4-(4'-chloro-1-hydroxymethylbenzyl)phenylhydrazine, the title compound is synthesized in otherwise a similar manner as Example 47.

### Example 92

### 2-[3,5-dichloro-4-(4'-chloro-1-fluoromethylbenzyl)phenyl]-2,3,4,5-tetrahydro-1,2,4-triazin-3-one (compound No. 100)

Starting with 2-[3,5-dichloro-4-(4'-chloro-1-hydroxymethylbenzyl)phenyl]-2,3,4,5-tetrahydro-1,2,4-triazine-3-one, the title compound is synthesized in otherwise a similar manner as Example 68.

### Example 93

### 2-[3,5-dichloro-4-(4'-chloro-1-chloromethylbenzyl)phenyl]-2,3,4,5-tetrahydro-1,2,4-triazin-3-one (compound No. 101)

In toluene, 2-[3,5-dichloro-4-(4'-chloro-1-hydroxymethylbenzyl)phenyl]-2,3,4,5-tetrahydro-1,2,4-triazin-3-one is heated with thionylchloride and purified to give the title compound by a column chromatography.

### Example 94

### 2-[3,5-dichloro-4-(4'-chloro-1-methylbenzyl)phenyl]-2,3,4,5-tetrahydro-1,2,4-triazin-3-one (compound No. 102)

2-[3,5-dichloro-4-(4'-chloro-1-chloromethylbenzyl)phenyl]-2,3,4,5-tetrahydro-1,2,4-triazin-3-one is dissolved in acetic acid and refluxed with 5-fold molar zinc powder for 3 hours. After concentrated, iced water is added to the results and extracted with ethyl acetate. The extract is dried by MgSO₄ and concentrated and purified by a column chromatography to provide the title compound.

### Example 95

### 2-[3,5-dichloro-4-(4'-chloro-1-methoxymethylbenzyl)phenyl]-2,3,4,5-tetrahydro-1,2,4-triazin-3-one (compound No. 103)

In methanol, 2-[3,5-dichloro-4-(4'-chloro-1-chloromethylbenzyl)phenyl]-2,3,4,5-tetrahydro-1,2,4-triazin-3-one is reacted with sodium methoxide to provide the title compound.

### Example 96

### 2-[3-trifluoromethyl-4-(4'-chloro-1-methylbenzyl)phenyl]-2,3,4,5-tetrahydro-1,2,4-triazin-3-one (compound No. 104)

In acetic acid is dissolved 2-[3-trifluoromethyl-4-(4'-chloro-1-methylbenzyl)phenyl]-2,3,4,5-tetrahydro-1,2,4-triazin-3-one followed by addition of 5-fold molar Zinc powder and refluxed for 3 hours with heated. After adding ice water, the reaction solution is extracted with ethyl acetate. The extract is dried with MgSO₄, concentrated and subjected to purification by column chromatography to provide the title compound.

### Example 97 (Reference Example)

### 2-{4-[2-(4-chlorophenyl)-1-cyanovinyl]-3,5-dichlorophenyl}-4,5-dihydro-1,2,4-triazin-3(2H)-one (compound No. 87)

Starting with 4-[2-(4-chlorophenyl)-1-cyanovinyl]-3,5-dichlorophenylhydrazine, the title compound was synthesized in otherwise a similar manner as Example 47. m.p. 83-85°C
- H-NMR (CDCl₃) δ;: 4.12-4.17(2H,J=2.1Hz,t), 5.83-5.86(1H,J=2.5Hz,d), 7.07-7.89(8H,m)

### Example 98

### 2-[3,5-dichloro-4-(5-trifluoromethyl-1,2,4-oxadiazol-3-yl-methyl)phenyl]-4,5-dihydro-1,2,4-triazin-3(2H)-one (compound No. 86)

Staring with 3,5-dichloro-4-(5-trifluoromethyl-1,2,4-oxadiazol-3-yl-methyl)phenylhydrazine, the title compound was synthesized in otherwise a similar manner as Example 47. m.p 164-165°C

### Example 99

### 2-[4-(4-chloro-α-chloromethylbenzyl)-3-trifluoromethylphenyl]-4,5-dihydro-1,2,4-triazin-3(2H)-one (compound No.76)

Staring with Compound No.95, the title compound was synthesized in otherwise a similar manner as Example 68. m.p. 157-158°C

### Example 100

### 2-[3-chloro-4-(4-chloro-α-fluorobenzyl)-5-methylpheyl]-4.5-dihydro-1,2,4-triazin-3(2H)-one (compound No.78)

Staring with Compound No.79, the title compound was synthesized in otherwise a similar manner as Example 68. m.p. 141-142°C (dec.)

### Example 101

### 2-[3-chloro-4-(4-chloro-α-hydroxybenzyl)-5-methylpheyl]-1,4,5,6-tetrahydro-1,2,4-triazin-3(2H)-one (compound No.80)

Staring with Compound No.81, Compound No.79 was synthesized in otherwise a similar manner as Example 60, followed by additon of excess sodium borate to synthsize compound No. 80. m.p. 146-147°C

### Example 102

### 2-[3-chloro-4-(4-chlorobenzoyl)-5-methylpheyl]-4.5-dihydro-1,2,4-triazin-3(2H)-one (compound No.81)

Starting with 3-chloro-4-(4-chlorobenzoyl)-5-methylpheylhydrazine, the title compound was synthesized in otherwise a similar manner as Example 47. m.p. 171-172°C

### Example 103 (Reference Example)

### 2-[3-chloro-4-(4-chlorobenzoyl)-5-methylpheyl]-1,2,4-triazin-3(2H)-one-5(4H)-thione (compound No.82)

Starting with .2-[3-chloro-4-(4-chlorobenzoyl)-5-methylpheyl]-1,2,4-triazin-3,5(2H,4H)-dione, the title compound was synthesized in otherwise a similar manner as Example 5. m.p. 104-106°C
- H'-NMR[d6-DMSO] δ;: 2.16(3H, s), 7.60 - 7.89 (7H, m), 13.89(1H, br-s).
Example 104

### 2-[(3-chloro-5-methyl-4-phenyl)phenyl]-4,5-dihydro-1,2,4-triazin-3(2H)-one (compound No. 83)

Starting with (3-chloro-5-methyl-4-phenyl)phenylhydrazine, the title compound was synthesized in otherwise a similar manner as Example 47. m.p. 175-176°C

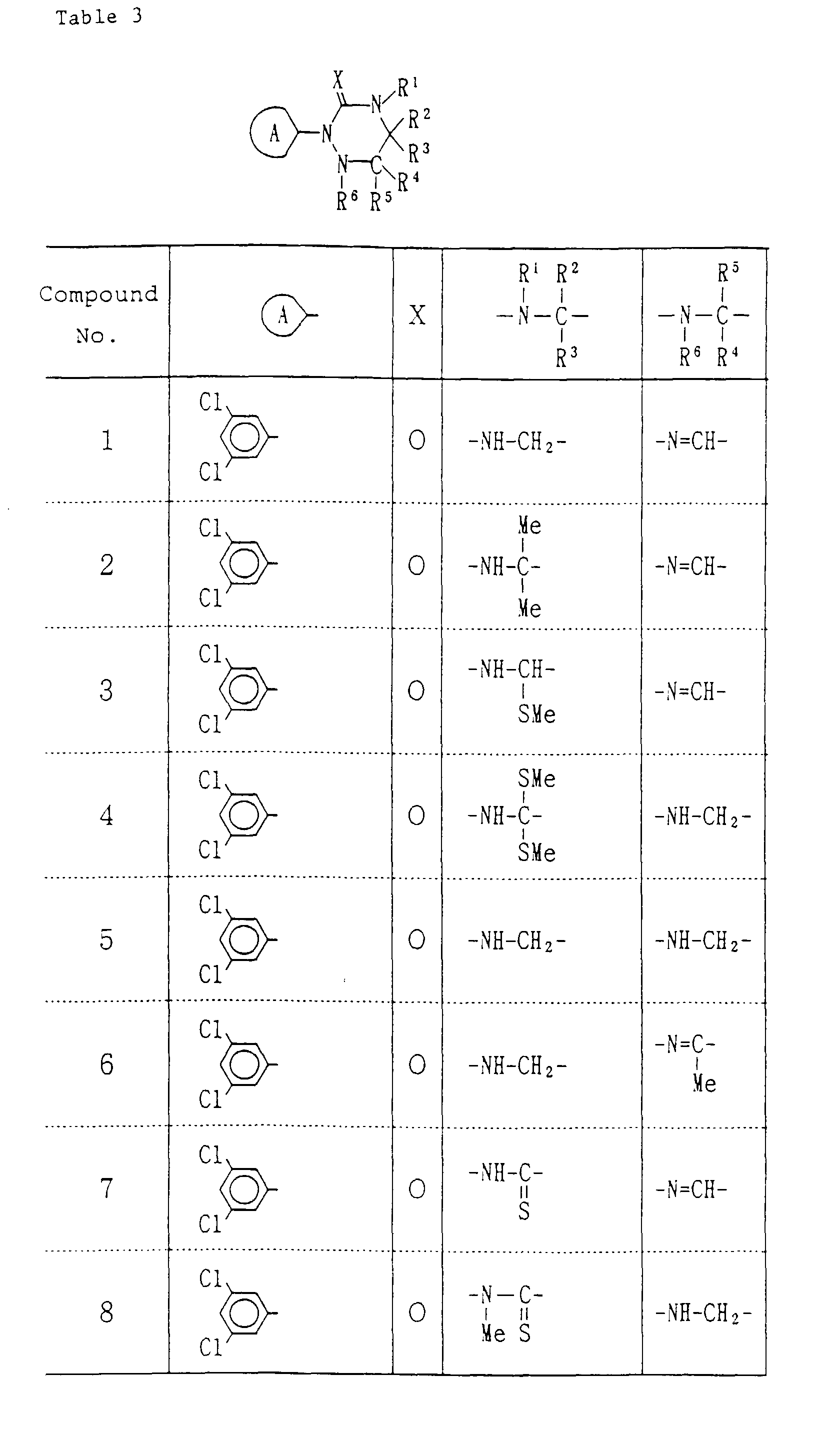

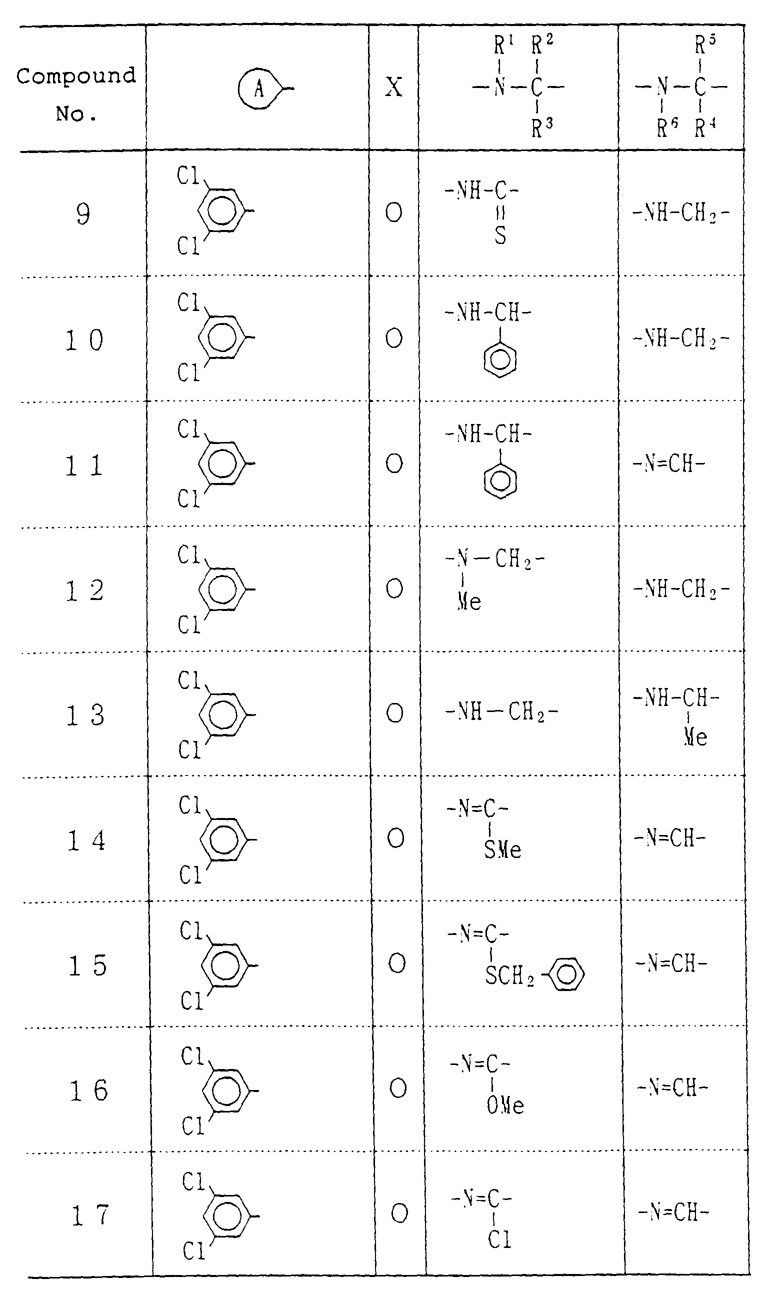

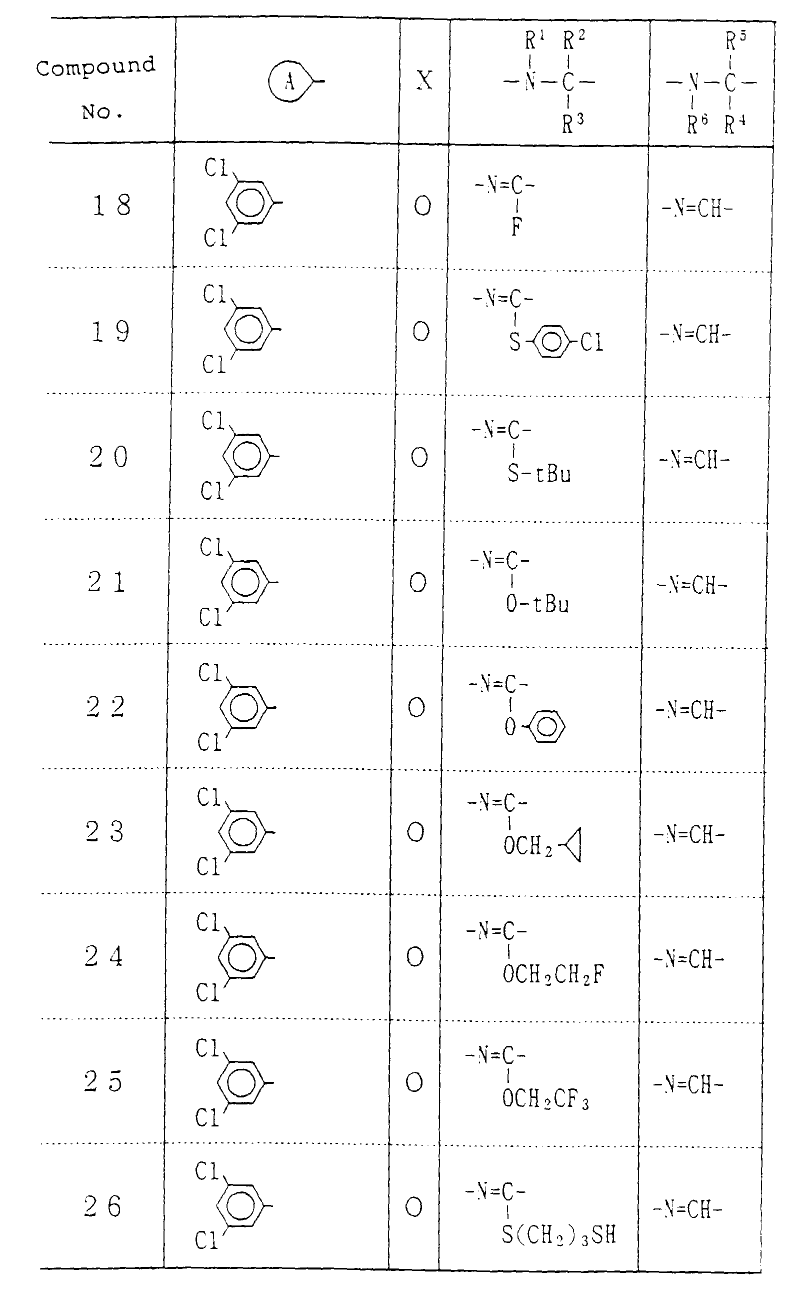

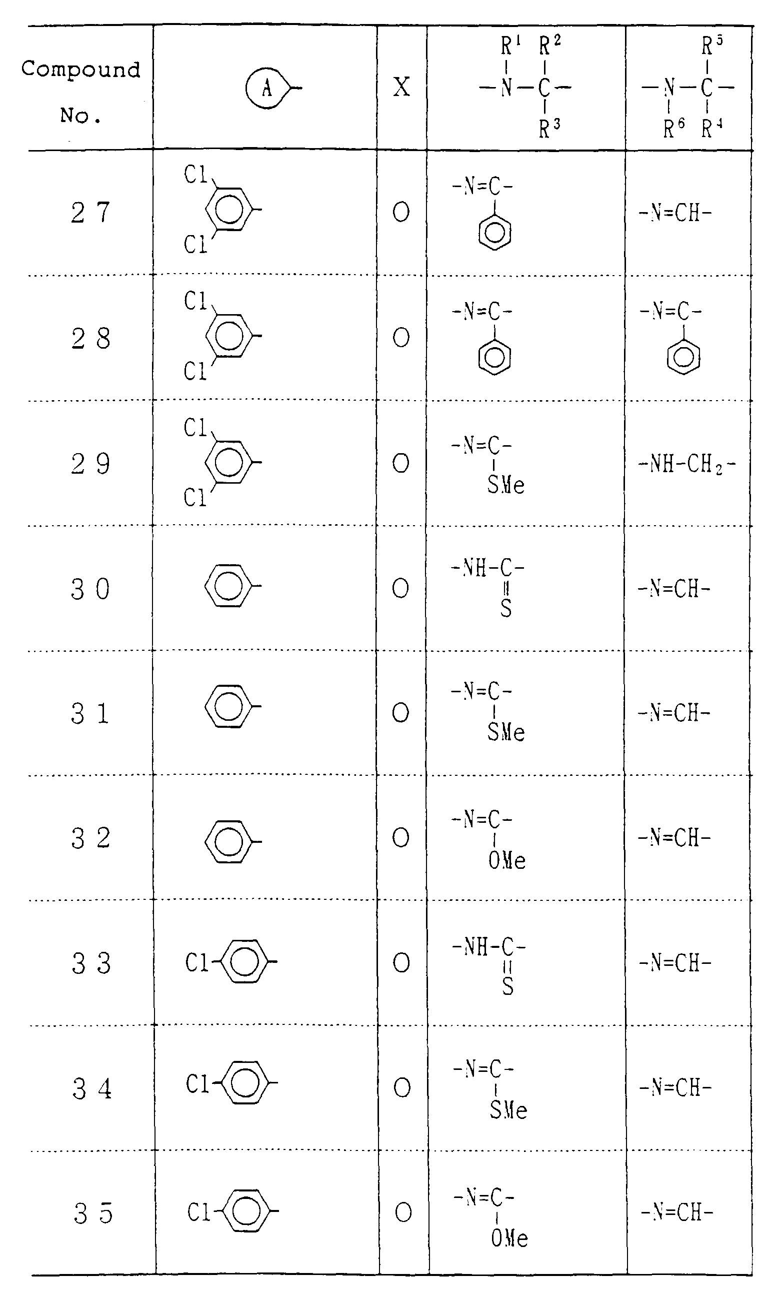

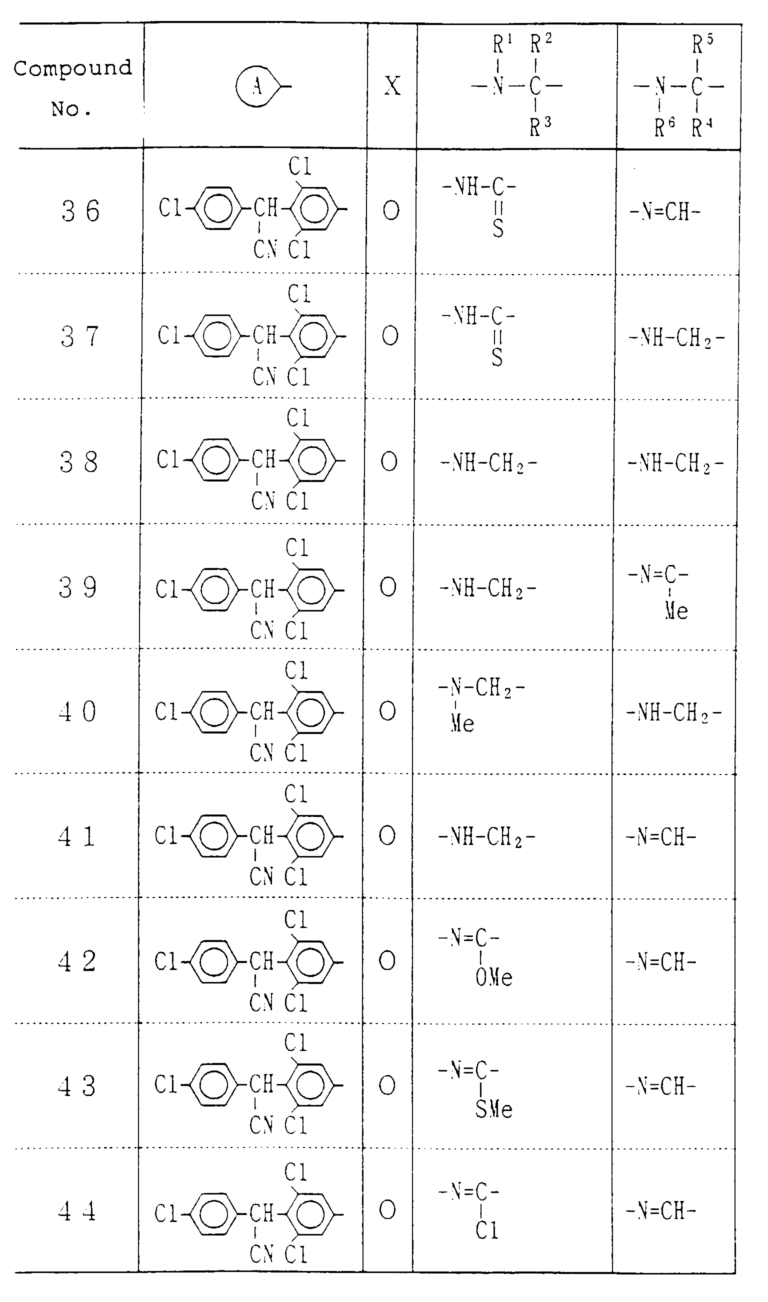

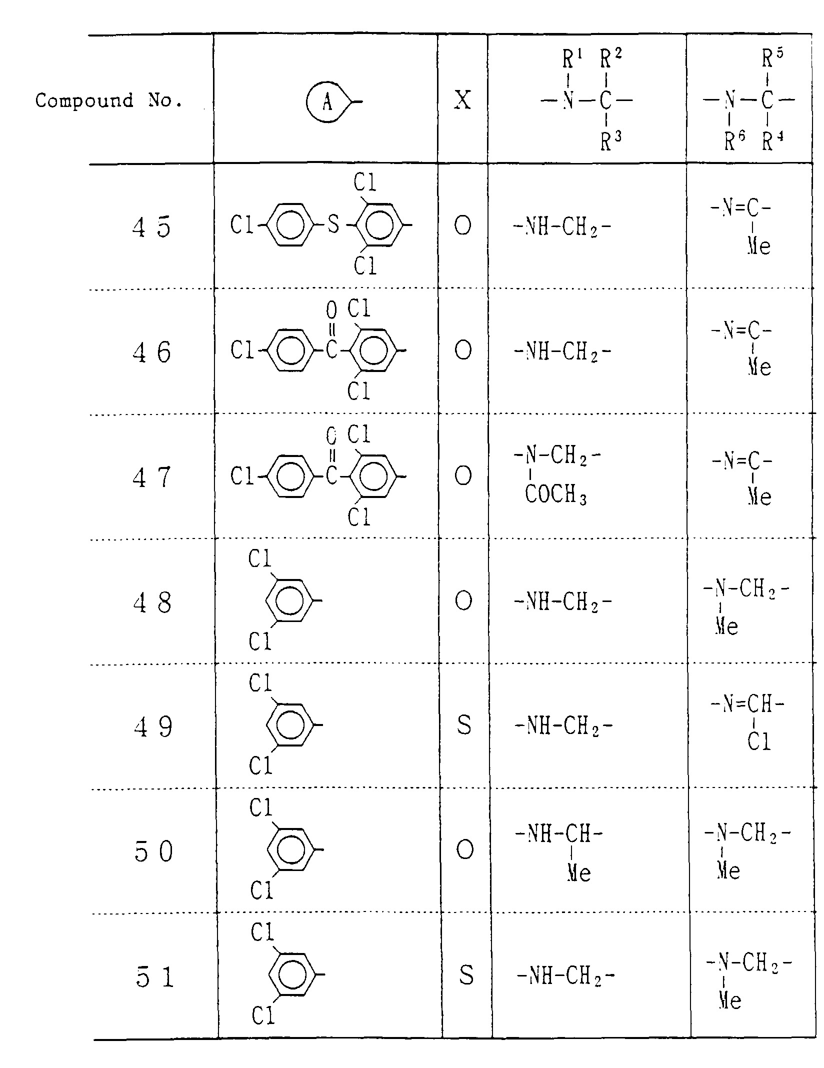

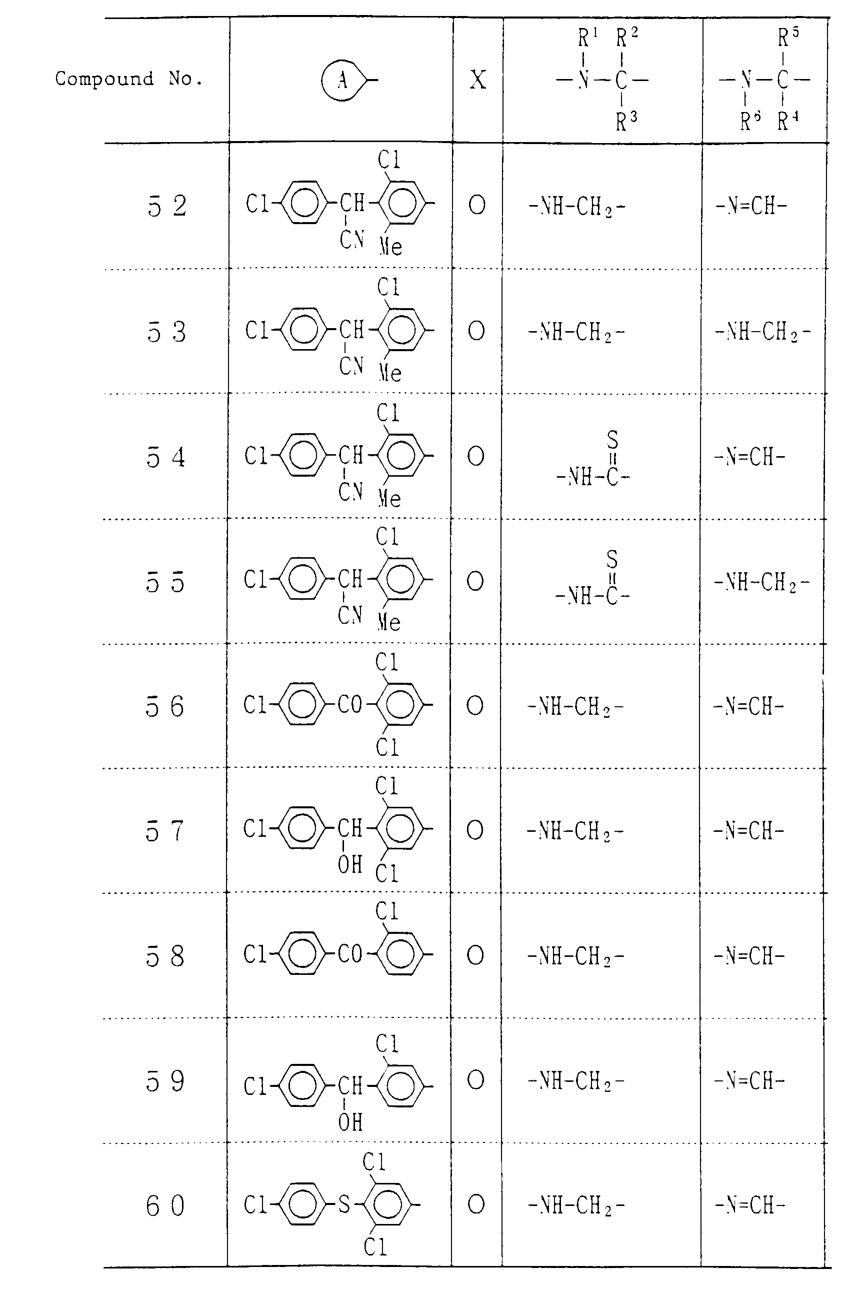

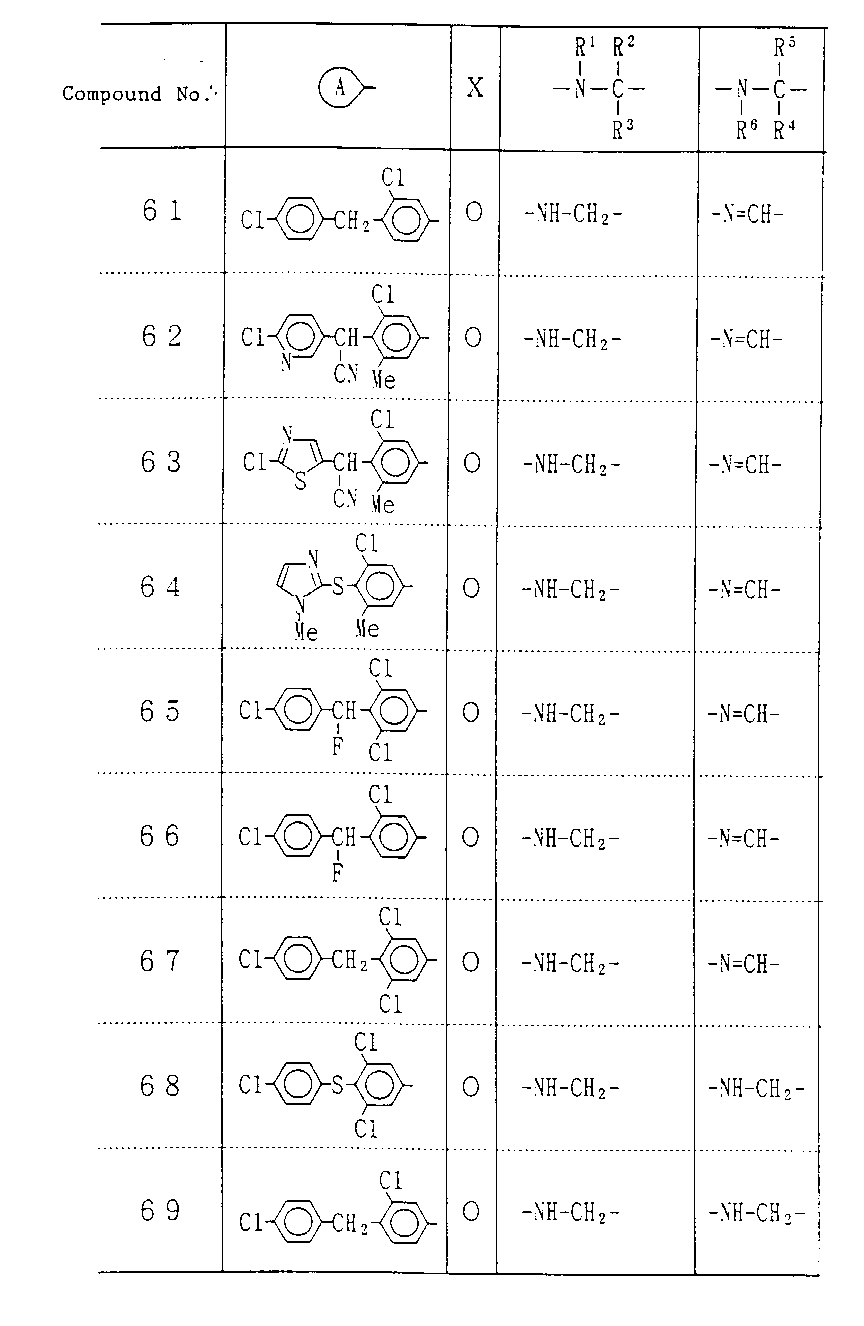

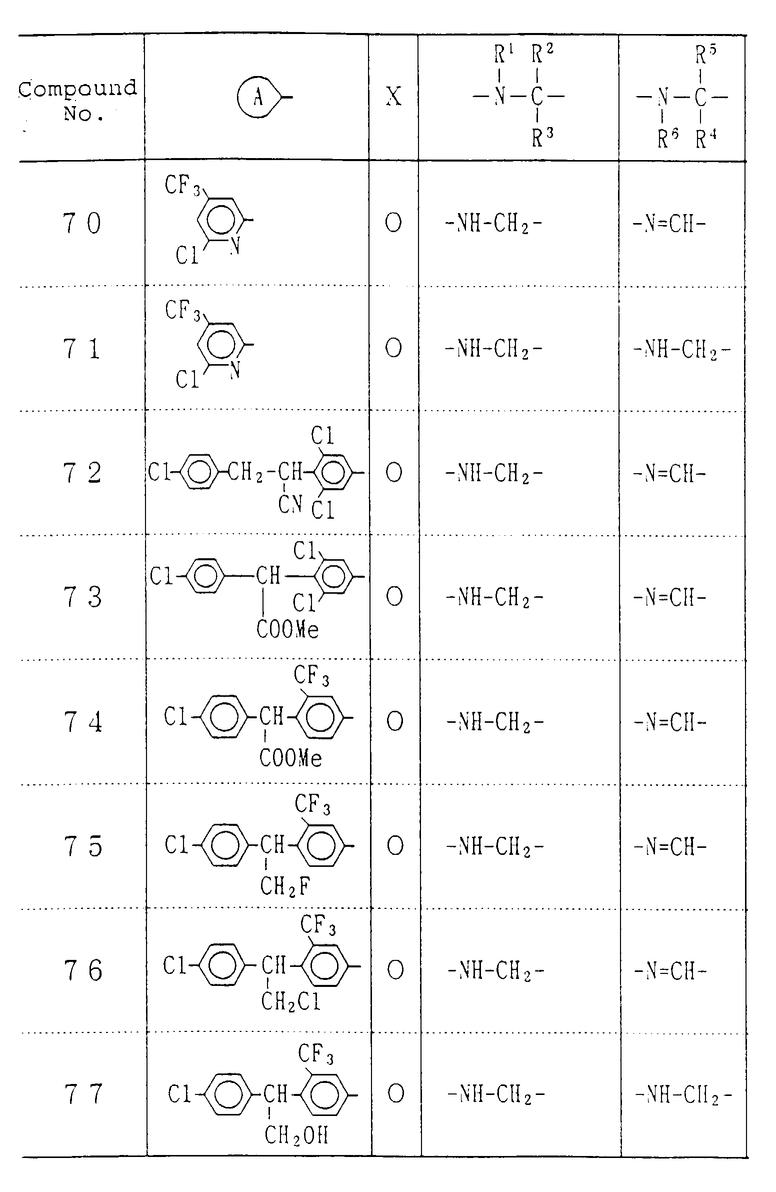

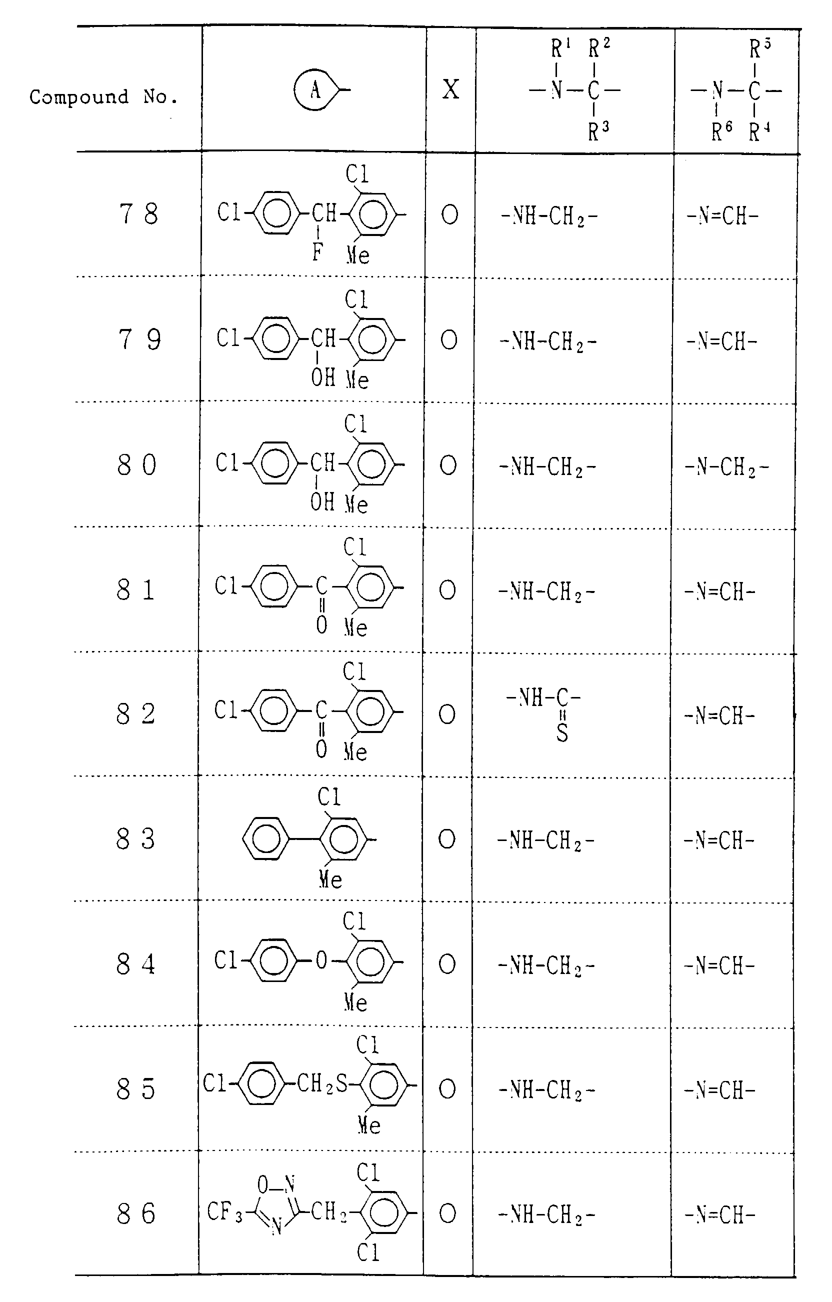

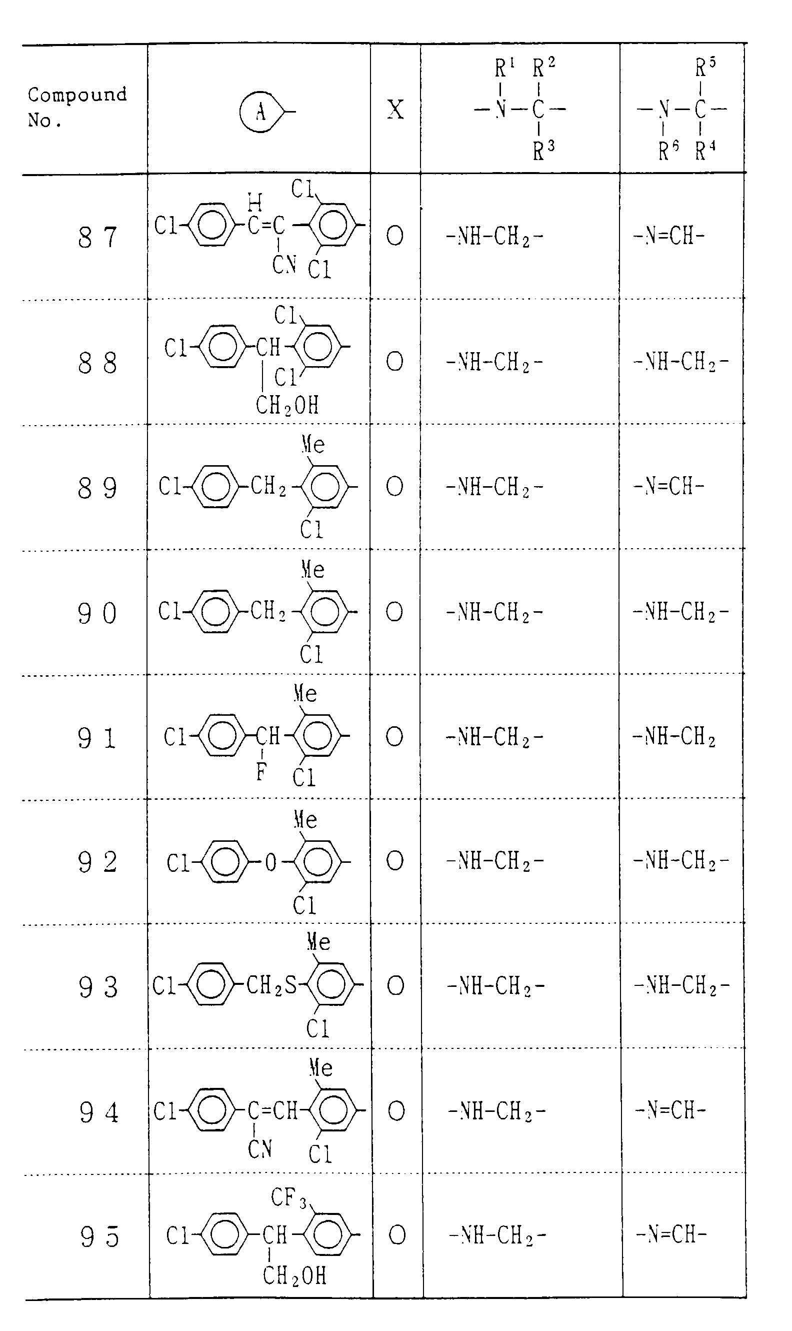

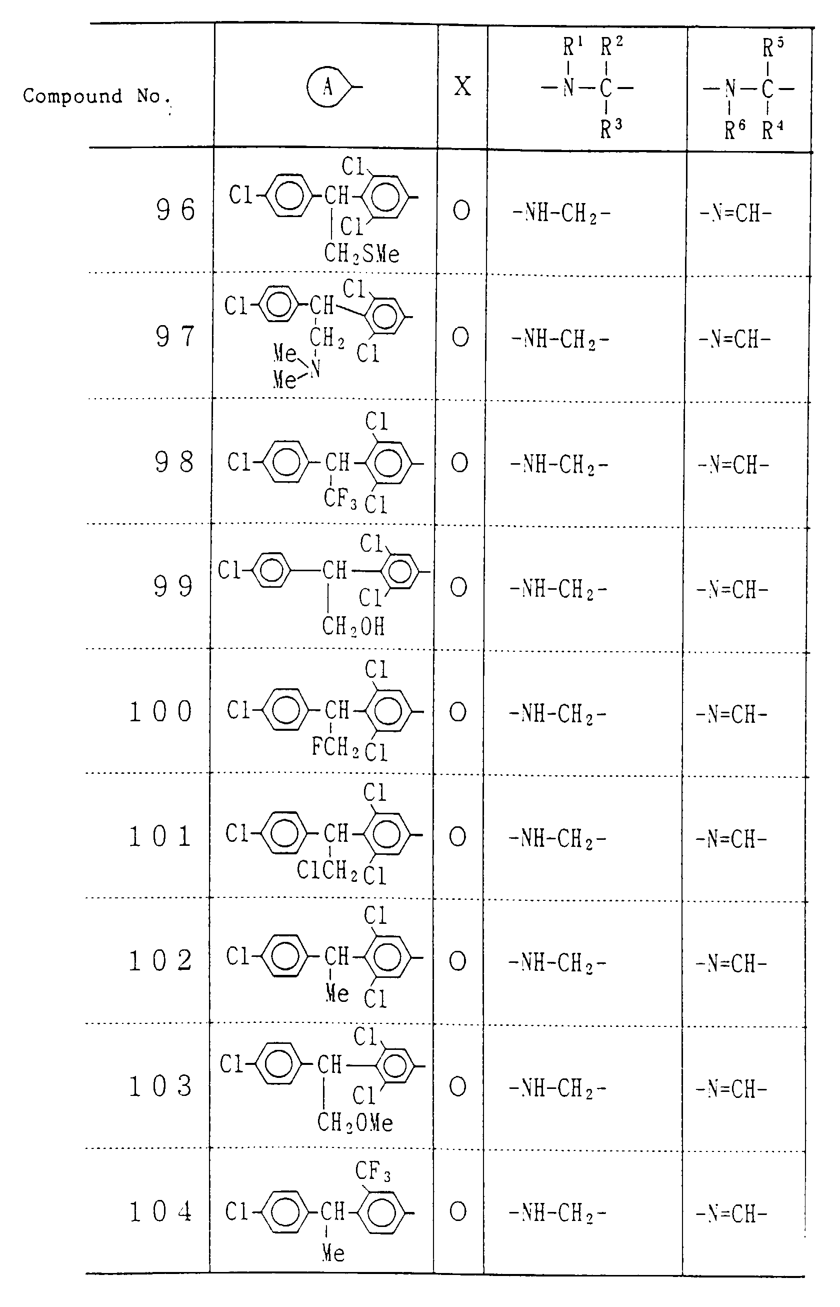

### Formulation Example 1

2-(3,5-Dichlorophenyl)-5-methoxy-2,3-dihydro-1,2,4-triazin-3-one (Compound No. 18), 25 g, was pulverized to pass a 355 µm sieve thoroughly and evenly blended with 975 g of defatted rice bran (1:1).

### Formulation Example 1

2-(3,5-Dichlorophenyl)-2,3,4,5-tetrahydro-1,2,4-triazin-3-one (Compound No. 1), 5.0 g, was dissolved in 10 cc of methanol, followed by addition of 100 g of soybean. After stirring, the mixture was dried in vacuo at 50°C for 10 hours. This mixture was crushed to pass a 500 µm sieve thoroughly and blended uniformly with 895 g of soybean meal to provide a composition.

## Claims

1. A compound of the formula: 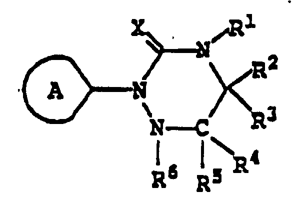
wherein ring A is a phenyl or 5- or 6-membered unsaturated heterocyclic group which may be substituted with 1 to 3 substituents selected from the group consisting of (1) C₁₋₄ alkyl, (2) C₂₋₄ alkenyl, (3) C₂₋₄ alkynyl, (4) C₃₋₆ cycloalkyl, (5) C₅₋₇ cycloalkenyl, (6) C₇₋₁₁ aralkyl, (7) phenyl, (8) C₁₋₆ alkoxy, (9) phenoxy, (10) C₁₋₆ alkanoyl, (11) benzoyl, (12) C₁₋₆ alkanoyloxy, (13) carboxyl, (14) C₂₋₇ alkoxycarbonyl, (15) carbamoyl, (16) N-mono-C₁₋₄ alkylcarbamoyl, (17) N-di-C₁₋₄ alkylcarbamoyl, (18) cycloaminocarbonyl, (19) halogen, (20) mono-, di- or tri-halo-C₁₋₄ alkyl, (21) oxo, (22) amidino, (23) imino, (24) amino which may be protected with a group selected from (i) formyl, (ii) C₁₋₆ alkyl-carbonyl which may be substituted with halogen atoms, (iii) C₆₋₁₀ arylcarbonyl which may be substituted with 1 to 3 halogen atoms, C₁₋₆ alkylcarbonyl or nitro group, (iv) C₁₋₆ alkyloxycarbonyl which may be substituted With 1 to 3 halogen atoms, C₁₋₆ alkylcarbonyl or nitro group, (v) C₆₋₁₀ aryloxycarbonyl which may be substituted with 1 to 3 halogen atoms, C₁₋₆ alkylcarbonyl or nitro group, (vi) C₇₋₁₂ aralkyl-carbonyl which may be substituted with 1 to 3 halogen atoms, C₁₋₆ alkylcarbonyl or nitro group, (vii) trityl which may be substituted with 1 to 3 halogen atoms, C₁₋₆ alkylcarbonyl or nitro group and (viii) phthaloyl which may be substituted with 1 to 3 halogen atoms, C₁₋₆ alkylcarbonyl or nitro group, (25) mono-C₁₋₄ alkylamino, (26) di-C₁₋₄ alkylamino, (27) 3- to 6-membered cycloamino which may contain 1 to 3 hereto atoms selected from oxygen, sulfur, and nitrogen, (28) C₁₋₆ alkanamido, (29) benzamido, (30) carbamoylamino, (31) N-C₁₋₄ alkylcarbamoylamino, (32) N,N-di-C₁₋₄ alkylcarbamoylamino, (33) C₁₋₈ alkylenedioxy, (34) -B(OH)₂, (35) hydroxy, (36) epoxy, (37) nitro, (38) cyano, (39) mercapto, (40) sulfo, (41) sulfino, (42) phosphono, (43) dihydroxypolyol, (44) sulfamoyl, (45) mono-C₁₋₆ alkylsulfamoyl, (46) di-C₁₋₄ alkylsulfamoyl, (47) C₁₋₆ alkylthio, (48) phenylthio, (49) C₁₋₆ alkylsulfinyl, (50) phenylsulfinyl, (51) C₁₋₆ alkylsulfonyl, (52) phenylsulfonyl, and (53) 5- or 6-membered heterocyclic group containing 1 to 4 hetero atoms selected from oxygen, sulfur, and nitrogen which may bound through one or two atomic chain containing oxygen, sulfur nitrogen, and carbon, in which any group having a carbon chain of 2 or more carbon atoms or a cyclic group may be further substituted with one or two substituents selected from the group consisting of (a) halogen, (b) hydroxy, (c) oxo, (d) C₁₋₄ alkoxy, (e) di-C₁₋₄ alkylamino, (f) halo-C₁₋₄ alkyl, (g) C₁₋₄ acyl, (h) hydroxy-C₁₋₄ alkyl, (i) C₁₋₄ alkoxy-C₁₋₄ alkyl, (j) cyano, (k) thioxo, and (l) C₁₋₄ alkylthio, and in which when the substituent mentioned above exists on two ring-forming atoms adjacent to each other, they may bind together to form a ring;
X is oxygen or sulfur;
R¹ and R⁶ are each a hydrogen atom, a C₁₋₇ acyl or a C₁₋₄ alkyl group;
R² and R³ are each a hydrogen atom, a halogen atom, or a C₁₋₄ alkyl or a phenyl group which may be bound through an oxygen or sulfur atom,
R⁴ and R⁵ are each a hyarogen atom, a halogen atom, a phenyl group or C₁₋₄ alkyl group;
R¹ and R² or R⁵ and R⁶ may together form a chemical bond, provided that where ring A is a phenyl group having at least a halogen atom in position 2 or 4 and X is an oxygen atom, then R⁵ and R⁶ do not bind together to form a chemical bond; or a salt thereof.

2. A compound or a salt thereof as claimed in claim 1, wherein ring A is a phenyl group which may be substituted with 1 to 3 substituents selected from the group consisting of halogen, C₁₋₄ alkyl group, halo-C₁₋₄ alkyl group, phenyl-C₁₋₄ alkyl group, and phenyl or 5- or 6-membered heterocyclic group containing 1 to 4 hetero atoms selected from oxygen, sulfur and nitrogen, which may be bound through an atomic chain of 1 or 2 atoms containing oxygen, sulfur, nitrogen and carbon.

3. A compound or a salt thereof as claimed in claim 1, wherein said heterocyclic group is a monocyclic heterocyclic group which may be substituted with 1 to 3 substituents selected from the group consisting of (1) C₁₋₄ alkyl, (2) C₂₋₄ alkenyl, (3) C₂₋₄ alkynyl, (4) C₃₋₆ cycloalkyl, (5) C₅₋₇ cycloalkenyl, (6) C₇₋₁₁ aralkyl, (7) phenyl, (8) C₁₋₆ alkoxy, (9) phenoxy, (10) C₁₋₆ alkanoyl, (11) benzoyl, (12) C₁₋₆ alkanoyloxy, (13) carboxyl, (14) C₂₋₇ alkoxycarbonyl, (15) carbamoyl, (16) N-mono-C₁₋₄ alkylcarbamoyl, (17) N-di-C₁₋₄ alkylcarbamoyl, (18) cycloaminocarbonyl, (19) halogen, (20) mono-, di- or tri-halo-C₁₋₄ alkyl, (21) oxo, (22) amidino, (23) imino, (24) amino which may be protected with a group selected from (i) formyl, (ii) C₁₋₆ alkyl-carbonyl which may be substituted with halogen atoms, (iii) C₆₋₁₀ aryl-carbonyl which may be substituted with 1 to 3 halogen atoms, C₁₋₆ alkylcarbonyl or nitro group, (iv) C₁₋₆ alkyloxycarbonyl which may be substituted with 1 to 3 halogen atoms, C₁₋₆ alkylcarbonyl or nitro group, (v) C₆₋₁₀ aryloxycarbonyl which may be substituted with 1 to 3 halogen atoms, C₁₋₆ alkylcarbonyl or nitro group, (vi) C₇₋₁₂ aralkyl-carbonyl which may be substituted with 1 to 3 halogen atoms, C₁₋₆ alkylcarbonyl or nitro group, (vii) trityl which may be substituted with 1 to 3 halogen atoms, C₁₋₆ alkylcarbonyl or nitro group and (viii) phthaloyl which may be substituted with 1 to 3 halogen atoms, C₁₋₆ alkylcarbonyl or nitro group, (25) mono-C₁₋₄ alkylamino, (26) di-C₁₋₄ alkylamino, (27) 3- to 6-membered cycloamino which may contain 1 to 3 hetero atoms selected from oxygen, sulfur, and nitrogen, (28) C₁₋₆ alkanamido, (29) benzamido, (30) carbamoylamino, (31) N-C₁₋₄ alkylcarbamoylamino, (32) N,N-di-C₁₋₄ alkylcarbamoylamino, (33) C₁₋₃ alkylenedioxy, (34) -B(OH)₂, (35) hydroxy, (36) epoxy, (37) nitro, (38) cyano, (39) mercapto, (40) sulfo, (41) sulfino, (42) phosphono, (43) dihydroxypolyol, (44) sulfamoyl, (45) mono-C₁₋₆ alkylsulfamoyl, (46) di-C₁₋₄ alkylsulfamoyl, (47) C₁₋₆ alkylthio, (48) phenylthio, (49) C₁₋₆ alkylsulfinyl, (50) phenylsulfinyl, (51) C₁₋₆ alkylsulfonyl, (52) phenylsulfonyl, and (53) 5- or 6-membered heterocyclic group containing 1 to 4 hetero atoms selected from oxygen, sulfur, and nitrogen which may bound through one or two atomic chain containing oxygen, sulfur nitrogen, and carbon, in which any group having a carbon chain of 2 or more carbon atoms or a cyclic group may be further substituted with one or two substituents selected from the group consisting of (a) halogen (b) hydroxy, (c) oxo, (d) C₁₋₄ alkoxy, (e) di-C₁₋₄ alkylamino, (f) halo-C₁₋₄ alkyl, (g) C₁₋₄ acyl, (h) hydroxy-C₁₋₄ alkyl, (i) C₁₋₄ alkoxy-C₁₋₄ alkyl, (j) cyano, (k) thioxo, and (l) C₁₋₄ alkylthio, and in which when the substituent mentioned above exists on two ring-forming atoms adjacent to each other, they may bind together to form a ring.

4. A compound or a salt thereof as claimed in any of claims 1-3, wherein ring A is a pyridyl group.

5. A compound or a salt thereof as claimed in any of claims 1-4, wherein R¹ is a hydrogen atom.

6. A compound or a salt thereof as claimed in any of claims 1-4, wherein R¹ is a C₁₋₇ acyl or a C₁₋₄ alkyl group.

7. A compound or a salt thereof as claimed in any of claims 1-6, wherein R² and R³ are each a hydrogen atom or a C₁₋₄ alkyl group.

8. A compound or a salt thereof as claimed in any of claims 1-7, wherein R⁴ and R⁵ are each a hydrogen atom, a halogen atom, a phenyl group or a C₁₋₄ alkyl group.

9. A compound or a salt thereof as claimed in any of claims 1-8, wherein R⁶ is a hydrogen atom or a C₁₋₄ alkyl group.

10. A compound or salt thereof as claimed in claim 1, wherein said compound has a structure of the formula: 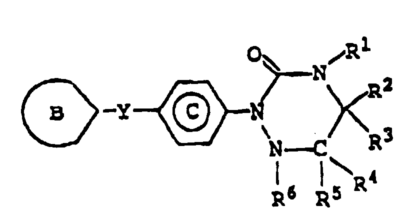
wherein ring B is a 5- or 6-membered cyclic group which may contain one or more hetero-atoms, and/or which may be substituted with 1 to 3 substituents selected from the group consisting of C₁₋₄ alkyl, C₁₋₄ alkoxy, carboxy, carbamoyl, halogen, mono-, di- or tri halo-C₁₋₄ alkyl, amino, -B(OH)₂, hydroxy, nitro, cyano, mercapto, sulfo, sulfino, phospho and C₁₋₄ acyl, in which the substituents as mentioned above exist on two ring-forming atoms adjacent to each other, they may bind together to form a ring which is condensed with the ring B;
ring C is a phenylene group which may be substituted with 1 to 4 substituents selected from the group consisting of (1) C₁₋₄ alkyl, (2) C₂₋₄ alkenyl, (3) C₂₋₄ alkynyl, (4) C₃₋₆ cycloalkyl, (5) C₅₋₇ cycloalkenyl, (6) C₇₋₁₁ aralkyl, (7) phenyl, (8) C₁₋₆ alkoxy, (9) phenoxy, (10) C₁₋₆ alkanoyl, (11) benzoyl, (12) C₁₋₆ alkanoyloxy, (13) carboxyl, (14) C₂₋₇ alkoxycarbonyl, (15) carbamoyl, (16) N-mono-C₁₋₄ alkylcarbamoyl, (17) N-di-C₁₋₄ alkylcarbamoyl, (18) cycloaminocarbonyl, (19) halogen, (20) mono-, di- or tri-halo-C₁₋₄ alkyl, (21) oxo, (22) amidino, (23) imino, (24) amino which may be protected with a group selected from (i) formyl, (ii) C₁₋₆ alkyl-carbonyl which may be substituted with halogen atoms, (iii) C₆₋₁₀ aryl-carbonyl which may be substituted with 1 to 3 halogen atoms, C₁₋₆ alkylcarbonyl or nitro group, (iv) C₁₋₆ alkyloxycarbonyl which may be substituted with 1 to 3 halogen atoms, C₁₋₆ alkylcarbonyl or nitro group, (v) C₆₋₁₀ aryloxycarbonyl which may be substituted with 1 to 3 halogen atoms, C₁₋₆ alkylcarbonyl or nitro group, (vi) C₇₋₁₂ aralkyl-carbonyl which may be substituted with 1 to 3 halogen atoms, C₁₋₆ alkylcarbonyl or nitro group, (vii) trityl which may be substituted with 1 to 3 halogen atoms, C₁₋₆ alkylcarbonyl or nitro group and (viii) phthaloyl which may be substituted with 1 to 3 halogen atoms, C₁₋₆ alkylcarbonyl or nitro group, (26) mono-C₁₋₄ alkylamino, (26) di-C₁₋₄ alkylamino, (27) 3- to 6-membered cycloamino which may contain 1 to 3 hetero atoms selected from oxygen, sulfur, and nitrogen, (28) C₁₋₆ alkanamido, (29) benzamido, (30) carbamoylamino, (31) N-C₁₋₄ alkylcarbamoylamino, (32) N,N-di-C₁₋₄ alkylcarbamoylamino, (33) C₁₋₃ alkylenedioxy, (34) -B(OH)₂, (35) hydroxy, (36) epoxy, (37) nitro, (38) cyano, (39) mercapto, (40) sulfo, (41) sulfino, (42) phosphono, (43) dihydroxypolyol, (44) sulfamoyl, (45) mono-C₁₋₆ alkylsulfamoyl, (46) di-C₁₋₄ alkylsulfamoyl, (47) C₁₋₆ alkylthio, (48) phenylthio, (49) C₁₋₆ alkylsulfinyl, (50) phenylsulfinyl, (51) C₁₋₆ alkylsulfonyl, (52) phenylsulfonyl, and (53) 5- or 6-membered heterocyclic group containing 1 to 4 hereto atoms selected from oxygen, sulfur, and nitrogen which may bound through one or two atomic chain containing oxygen, sulfur nitrogen, and carbon, in which any group having a carbon chain of 2 or more carbon atoms or a cyclic group may be further substituted with one or two substituents selected from the group consisting of (a) halogen, (b) hydroxy, (c) oxo, (d) C₁₋₄ alkoxy, (e) di-C₁₋₄ alkylamino, (f) halo-C₁₋₄ alkyl, (g) C₁₋₄ acyl, (h) hydroxy-C₁₋₄ alkyl, (i) C₁₋₄ alkoxy-C₁₋₄ alkyl, (j) cyano, (k) thioxo, and (l) C₁₋₄ alkylthio, and in which when the substituent mentioned above exists on two ring-forming atoms adjacent to each other, they may bind together to form a ring
R₁ and R₆ are each a hydrogen atom, a C₁₋₇ acyl or a C₁₋₄ alkyl group;
R₂ and R₃ are each a hydrogen atom, a halogen atom, or a C₁₋₄ alkyl group or a phenyl group which may be bound through an oxygen or sulfur atom;
R⁴ and R⁵ are each a hydrogen atom, a halogen atom, a phenyl group or a C₁₋₄ alkyl group;
R¹ and R² or R⁵ and R⁶ together form a chemical bond;
Y is a chemical bond, -0-, -S(O)ₘ-, or a C₁₋₄ alkylene group which may be substituted with 1 to 4 substituents selected from the group consisting of halogen, hydroxy, oxo cyano, C₁₋₄ alkoxy, mono- or di-C₁₋₄ alkylamino, halo-C₁₋₄ alkyl, C₁₋₄ acyl, hydroxy C₁₋₄ alkyl, C₁₋₄ alkoxy C₁₋₄ alkyl, C₁₋₄ alkoxy-carbonyl, thioxo and C₁₋₄ alkylthio and m is 0, 1 or 2.

11. A compound or a salt thereof as claimed in claim 10, wherein the ring B is a phenyl, pyridyl, thiazolyl or imidazolyl group, which may be substituted with 1 to 3 substituents selected from the group consisting of C₁₋₄ alkyl, C₁₋₄ alkoxy, carboxy, carbamoyl, halogen, mono-, di- or tri-halo-C₁₋₄ alkyl, amino, -B(OH)₂, hydroxy, nitro, cyano, mercapto, sulfo, sulfino, phospho and C₁₋₄ acyl, and when the substituents exist on two ring-forming atoms adjacent to each other, they may bind together to form a ring.

12. A compound or a salt thereof as claimed in claim 11, wherein the ring B is a phenyl group which may be substituted with 1 to 3 substituents selected from the group consisting of C₁₋₄ alkyl, C₁₋₄, alkoxy, carboxy, carbamoyl, halogen, mono-, di- or tri-halo-C₁₋₄ alkyl, amino, -B(OH)₂, hydroxy, nitro, cyano, mercapto, sulfo, sulfino, phospho and C₁₋₄ acyl, and when the substituents exist on two ring-forming atoms adjacent to each other, they may bind together to form a ring.

13. A compound or a salt thereof as claimed in any of claims 10-12, wherein R¹ and R⁶ are each, independently, hydrogen or a C₁₋₄ alkyl group.

14. A compound or a salt thereof as claimed in any of claims 10-12 wherein R¹ is hydrogen a C₁₋₇ acyl or a C₁₋₄ alkyl group.

15. A compound or a salt thereof as claimed in claim 14, wherein R¹ is hydrogen.

16. A compound or a salt thereof as claimed in any of claims 10-15, wherein R² and R³ are each, independently, a hydrogen atom, a halogen atom, a phenyl, a C₁₋₄ alkyl, C₁₋₄ alkoxy or C₁₋₄ alkylthio group.

17. A compound or a salt thereof as claimed in claim 16, wherein R² and R³ are each, independently, a hydrogen atom or C₁₋₄ alkyl group.

18. A compound or a salt thereof as claimed in claim 17, wehrein R² and R³ are both hydrogen atoms.

19. A compound or a salt thereof as claimed in any of claims 10-18, wherein R⁴ and R⁵ are each, independently, a hydrogen atom, a phenyl or a C₁₋₄ alkyl group.

20. A compound or a salt thereof as claimed in claim 19, wherein R⁴ is a hydrogen atom.

21. A compound or a salt thereof as claimed in any of claims 10-20 wherein Y is a C₁₋₄ alkylene group which may be substituted with 1 to 4 substitutents selected from the group consisting of halogen, hydroxy, oxo, cyano, C₁₋₄ alkoxy, mono- or di-C₁₋₄ alkylamino, halo-C₁₋₄ alkyl, C₁₋₄ acyl, hydroxy-C₁₋₄ alkyl, C₁₋₄ alkoxy-C₁₋₄ alkyl, C₁₋₄ alkoxy-carbonyl, thioxo and C₁₋₄ alkylthio.

22. A compound or a salt thereof as claimed in claim 13 wherein R⁶ is hydrogen or a C₁₋₄ alkyl group.

23. A compound or a salt thereof as claimed in any of claims 10-12 wherein R⁵ and R⁶ together form a chemical bond.

24. A compound or a salt thereof as claimed in claim 1 wherein R¹, R², R³ and R⁴ are a hydrogen atom and R⁵ and R⁶ form a chemical bond.

25. A compound or a salt thereof as claimed in claim 10,
wherein the ring B is a phenyl which may be substituted with 1 to 3 substituents selected from the group consisting of C₁₋₄ alkyl, C₁₋₄ alkoxy, carboxy, carbamoyl, halogen, mono-, di- or tri-halo-C₁₋₄ alkyl, amino, -B(OH)₂, hydroxy, nitro, cyano, mercapto, sulfo, sulfino, phospho and C₁₋₄ acyl, and when the substituents exist on two ring-forming atoms adjacent to each other, they may bind together to form a ring;
the ring C is a phenylene which may be substituted with 1 to 4 substituents selected from the group consisting of halogen, C₁₋₄ alkyl, C₁₋₆ alkoxy and halo-C₁₋₄ alkyl;
R¹, R², R³ and R⁴ are hydrogen atoms; R⁵ and R⁶ together form a chemical bond; and Y is a C₁₋₄ alkylene group which may be substituted with 1 to 4 substituents selected from the group consisting of halogen, hydroxy, oxo, cyano, C₁₋₄ alkoxy, mono- or di-C₁₋₄ alkylamino, halo-C₁₋₄ alkyl, C₁₋₄ acyl, hydroxy-C₁₋₄ alkyl, C₁₋₄ alkoxy-C₁₋₄ alkyl, C₁₋₄ alkoxy-carbonyl, thioxo and C₁₋₄ alkylthio.

26. A compound as claimed in claim 10 which is 2-[3,5-dichloro-4-(4-chlorobenzyl)phenyl]-2,3,4,5-tetrahydro-1,2,4-triazin-3-one or a salt thereof.

27. A compound as claimed in claim 10 which is 2-[3-chloro-4-(4-chlorobenzyl)-5-methylphenyl]-2,3,4,5-tetrahydro-1,2,4-triazin-3-one or a salt thereof.

28. A compound as claimed in claim 10 which is 2-[3,5-dichloro-4-(4'-chloro-1-methylbenzyl)phenyl]-2,3,4,5-tetrahydro-1,2,4-triazin-3-one or a salt thereof.

29. An antiprotozoan composition comprising an effective amount of a compound as claimed in any of claims 1-28 or a salt thereof; and a pharmaceutically acceptable carrier, excipient or diluent.

30. The composition as claimed in claim 29, wherein said compound is a compound as claimed in claim 10, or a salt thereof.

31. An anticoccidian composition comprising an effective amount of a compound as claimed in claim 10, or a salt thereof, and a pharmaceutically acceptable carrier, excipient or diluent.

32. An animal feed additive premix comprising a compound according to claim 29 or a physiologically acceptable salt thereof.

33. A method for preparing a compound as claimed in any of claims 1-28 or a salt thereof;
comprising cyclizing the compound of the formula: 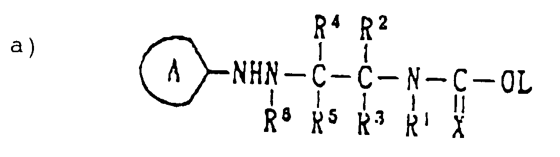 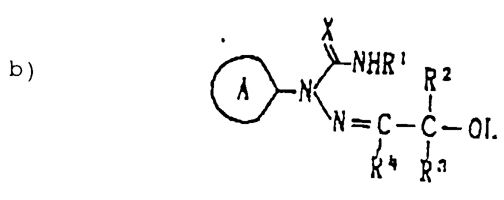 or 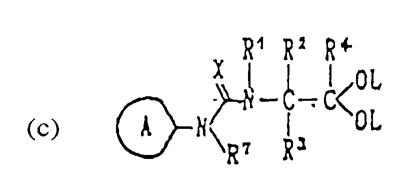 wherein A, R¹, R², R³, R⁴, R⁵ and R⁶ are of the same meaning defined in claim 1, R⁷ is an optionally protected amino group and L is an alkyl or acyl.

34. The method as claimed in claim 33, wherein the cyclizing reaction is conducted in an inert solvent or in the absence of a solvent optionally in the presence of a Lewis acid or Lewis base.

35. The method as claimed in claim 33 or 34, wherein the cyclizing reaction of the compound of the formula a) is conducted at a temperature of about 60°C to about 200°C.

36. The method as claimed in claim 33 or 34, wherein the cyclizing reaction of the compound of the formula b) is conducted at a temperature of about 0°C to about 200°C.

37. The method as claimed in claim 33 or 34, wherein the cyclizing reaction of the compound of the formula c) is conducted at a temperature of about 60°C to about 160°C.

## Patentansprüche

1. Verbindung der Formel 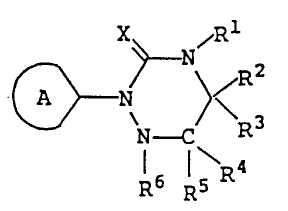 wobei der Ring A eine Phenyl- oder eine 5- oder 6-gliedrige ungesättigte heterocyclische Gruppe ist, welche substituiert sein kann mit 1 bis 3 Substituenten, ausgewählt aus der Gruppe, bestehend aus (1) C₁₋₄-Alkyl, (2) C₂₋₄-Alkenyl, (3) C₂₋₄-Alkinyl, (4) C₃₋₆-Cycloalkyl, (5) C₅₋₇-Cycloalkenyl, (6) C₇₋₁₁-Aralkyl, (7) Phenyl, (8) C₁₋₆-Alkoxy, (9) Phenoxy, (10) C₁₋₆-Alkanoyl, (11) Benzoyl, (12) C₁₋₆-Alkanoyloxy, (13) Carboxyl, (14) C₂₋₇-Alkoxycarbonyl, (15) Carbamoyl, (16) N-Mono-C₁₋₄-alkylcarbamoyl, (17) N-Di-C₁₋₄-alkylcarbamoyl, (18) Cycloaminocarbonyl, (19) Halogen, (20) Mono-, Di- oder Tri-halo-C₁₋₄-alkyl, (21) Oxo, (22) Amidino, (23) Imino, (24) Amino, welches mit einer Gruppe geschützt sein kann, ausgewählt aus (i) Formyl, (ii) C₁₋₆-Alkylcarbonyl, welches mit Halogenatomen substituiert sein kann, (iii) C₆₋₁₀-Arylcarbonyl, welches mit 1 bis 3 Halogenatomen, C₁₋₆-Alkylcarbonyl- oder Nitrogruppe substituiert sein kann, (iv) C₁₋₆-Alkyloxycarbonyl, welches mit 1 bis 3 Halogenatomen, C₁₋₆-Alkylcarbonyl- oder Nitrogruppe substituiert sein kann, (v) C₆₋₁₀-Aryloxycarbonyl, welche mit 1 bis 3 Halogenatomen, C₁₋₆-Alkylcarbonyl- oder Nitrogruppe substituiert sein kann, (vi) C₇₋₁₂-Aralkylcarbonyl, welches mit 1 bis 3 Halogenatomen, C₁₋₆-Alkylcarbonyl- oder Nitrogruppe substituiert sein kann, (vii) Trityl, welches mit 1 bis 3 Halogenatomen, C₁₋₆-Alkylcarbonyl- oder Nitrogruppe substituiert sein kann und (viii) Phthaloyl, welches mit 1 bis 3 Halogenatomen, C₁₋₆-Alkylcarbonyl- oder Nitrogruppe substituiert sein kann, (25) Mono-C₁₋₄-alkylamino, (26) Di-C₁₋₄-alkylamino, (27) 3- bis 6-gliedrigem Cycloamino, welches 1 bis 3 Heteroatome enthalten kann, ausgewählt aus Sauerstoff, Schwefel und Stickstoff, (28) C₁₋₆-Alkanamido, (29) Benzamido, (30) Carbamoylamino, (31) N-C₁₋₄-Alkylcarbamoylamino, (32) N,N-Di-C₁₋₄-alkylcarbamoylamino, (33) C₁₋₃-Alkylendioxy, (34) -B(OH)₂, (35) Hydroxy, (36) Epoxy, (37) Nitro, (38) Cyano, (39) Mercapto, (40) Sulfo, (41) Sulfino, (42) Phosphono, (43) Dihydroxypolyol, (44) Sulfamoyl, (45) Mono-C₁₋₆-alkylsulfamoyl, (46) Di-C₁₋₄-alkylsulfamoyl, (47) C₁₋₆-Alkylthio, (48) Phenylthio, (49) C₁₋₆-Alkylsulfinyl, (50) Phenylsulfinyl, (51) C₁₋₆-Alkylsulfonyl, (52) Phenylsulfonyl und (53) einer 5- oder 6-gliedrigen heterocyclischen Gruppe, welche 1 bis 4 Heteroatome enthält, ausgewählt aus Sauerstoff, Schwefel und Stickstoff, welche über eine oder zwei Atomketten gebunden sein kann, die Sauerstoff, Schwefel, Stickstoff und Kohlenstoff enthalten, wobei jede Gruppe mit einer Kohlenstoffkette aus zwei oder mehr Kohlenstoffatomen oder einer cyclischen Gruppe ferner mit einem oder zwei Substituenten substituiert sein kann, ausgewählt aus der Gruppe, bestehend aus (a) Halogen, (b) Hydroxy, (c) Oxo, (d) C₁₋₄-Alkoxy, (e) Di-C₁₋₄-alkylamino, (f) Halo-C₁₋₄-alkyl, (g) C₁₋₄-Acyl, (h) Hydroxy-C₁₋₄-alkyl, (i) C₁₋₄-Alkoxy-₁₋₄-alkyl, (j) Cyano, (k) Thioxo und (1) C₁₋₄-Alkylthio, und wobei, wenn der oben erwähnte Substituent an zwei zueinander benachbarten ringbildenden Atomen vorhanden ist, sie miteinander verbunden sein können, um einen Ring zu bilden;
X gleich Sauerstoff oder Schwefel ist;
R¹ und R⁶ jeweils ein Wasserstoffatom, eine C₁₋₇-Acyl- oder eine C₁₋₄-Alkylgruppe sind;
R² und R³ jeweils ein Wasserstoffatom, ein Halogenatom oder eine C₁₋₄-Alkyl- oder eine Phenylgruppe, welche über ein Sauerstoff- oder Schwefelatom gebunden sein kann, sind;
R⁴ und R⁵ jeweils ein Wasserstoffatom, ein Halogenatom, eine Phenylgruppe oder eine C₁₋₄-Alkylgruppe sind;
R¹ und R² oder R⁵ und R⁶ zusammen eine chemische Bindung bilden können, vorausgesetzt, daß wenn der Ring A eine Phenylgruppe mit mindestens einem Halogenatom in der Position 2 oder 4 besitzt und X ein Sauerstoffatom ist, R⁵ und R⁶ dann nicht miteinander unter Ausbildung einer chemischen Bindung verbunden sind;
oder ein Salz davon.

2. Verbindung oder Salz davon nach Anspruch 1, wobei der Ring A eine Phenylgruppe, welche substituiert sein kann mit 1 bis 3 Substituenten, ausgewählt aus der Gruppe, bestehend aus Halogen, C₁₋₄-Alkylgruppe, Halo-C₁₋₄-alkylgruppe, Phenyl-C₁₋₄-Alkylgruppe und Phenyl, oder eine 5- oder 6-gliedrige heterocyclische Gruppe ist, welche 1 bis 4 Heteroatome enthält, ausgewählt aus Sauerstoff, Schwefel und Stickstoff, welche über eine Atomkette aus 1 oder 2 Atomen gebunden sein kann, die Sauerstoff, Schwefel, Stickstoff und Kohlenstoff enthält.

3. Verbindung oder Salz davon nach Anspruch 1, wobei die heterocyclische Gruppe eine monocyclische heterocyclische Gruppe ist, welche substituiert sein kann mit 1 bis 3 Substituenten, ausgewählt aus der Gruppe, bestehend aus (1) C₁₋₄-Alkyl, (2) C₂₋₄-Alkenyl, (3) C₂₋₄-Alkinyl, (4) C₃₋₆-Cycloalkyl, (5) C₅₋₇-Cycloalkenyl, (6) C₇₋₁₁-Aralkyl, (7) Phenyl, (8) C₁₋₆-Alkoxy, (9) Phenoxy, (10) C₁₋₆-Alkanoyl, (11) Benzoyl, (12) C₁₋₆-Alkanoyloxy, (13) Carboxyl, (14) C₂₋₇-Alkoxycarbonyl, (15) Carbamoyl, (16) N-Mono-C₁₋₄-alkylcarbamoyl, (17) N-Di-C₁₋₄-alkylcarbamoyl, (18) Cycloaminocarbonyl, (19) Halogen, (20) Mono-, Di- oder Tri-halo-C₁₋₄-alkyl, (21) Oxo, (22) Amidino, (23) Imino, (24) Amino, welches mit einer Gruppe geschützt sein kann, ausgewählt aus (i) Formyl, (ii) C₁₋₆-Alkylcarbonyl, welches mit Halogenatomen substituiert sein kann, (iii) C₆₋₁₀-Arylcarbonyl, welches mit 1 bis 3 Halogenatomen, C₁₋₆-Alkylcarbonyl- oder Nitrogruppe substituiert sein kann, (iv) C₁₋₆-Alkyloxycarbonyl, welches mit 1 bis 3 Halogenatomen, C₁₋₆-Alkylcarbonyl- oder Nitrogruppe substituiert sein kann, (v) C₆₋₁₀-Aryloxycarbonyl, welche mit 1 bis 3 Halogenatomen, C₁₋₆-Alkylcarbonyl- oder Nitrogruppe substituiert sein kann, (vi) C₇₋₁₂-Aralkylcarbonyl, welches mit 1 bis 3 Halogenatomen, C₁₋₆-Alkylcarbonyl- oder Nitrogruppe substituiert sein kann, (vii) Trityl, welches mit 1 bis 3 Halogenatomen, C₁₋₆-Alkylcarbonyl- oder Nitrogruppe substituiert sein kann und (viii) Phthaloyl, welches mit 1 bis 3 Halogenatomen, C₁₋₆-Alkylcarbonyl- oder Nitrogruppe substituiert sein kann, (25) Mono-C₁₋₄-alkylamino, (26) Di-C₁₋₄-alkylamino, (27) 3- bis 6-gliedrigem Cycloamino, welches 1 bis 3 Heteroatome enthalten kann, ausgewählt aus Sauerstoff, Schwefel und Stickstoff, (28) C₁₋₆-Alkanamido, (29) Benzamido, (30) Carbamoylamino, (31) N-C₁₋₄-Alkylcarbamoylamino, (32) N,N-Di-C₁₋₄-alkylcarbamoylamino, (33) C₁₋₃-Alkylendioxy, (34) -B(OH)₂, (35) Hydroxy, (36) Epoxy, (37) Nitro, (38) Cyano, (39) Mercapto, (40) Sulfo, (41) Sulfino, (42) Phosphono, (43) Dihydroxypolyol, (44) Sulfamoyl, (45) Mono-C₁₋₆-alkylsulfamoyl, (46) Di-C₁₋₄-alkylsulfamoyl, (47) C₁₋₆-Alkylthio, (48) Phenylthio, (49) C₁₋₆-Alkylsulfinyl, (50) Phenylsulfinyl, (51) C₁₋₆-Alkylsulfonyl, (52) Phenylsulfonyl und (53) einer 5- oder 6-gliedrigen heterocyclischen Gruppe, welche 1 bis 4 Heteroatome enthält, ausgewählt aus Sauerstoff, Schwefel und Stickstoff, welche über eine oder zwei Atomketten gebunden sein kann, die Sauerstoff, Schwefel, Stickstoff und Kohlenstoff enthalten, wobei jede Gruppe mit einer Kohlenstoffkette aus zwei oder mehr Kohlenstoffatomen oder einer cyclischen Gruppe ferner mit einem oder zwei Substituenten substituiert sein kann, ausgewählt aus der Gruppe, bestehend aus (a) Halogen, (b) Hydroxy, (c) Oxo, (d) C₁₋₄-Alkoxy, (e) Di-C₁₋₄-alkylamino, (f) Halo-C₁₋₄-alkyl, (g) C₁₋₄-Acyl, (h) Hydroxy-C₁₋₄-alkyl, (i) C₁₋₄-Alkoxy-₁₋₄-alkyl, (j) Cyano, (k) Thioxo und (1) C₁₋₄-Alkylthio, und wobei, wenn der oben erwähnte Substituent an zwei zueinander benachbarten ringbildenden Atomen vorhanden ist, sie miteinander verbunden sein können, um einen Ring zu bilden.

4. Verbindung oder Salz davon nach einem der Ansprüche 1 bis 3, wobei der Ring A eine Pyridylgruppe ist.

5. Verbindung oder Salz davon nach einem der Ansprüche 1 bis 4, wobei R¹ ein Wasserstoffatom ist.

6. Verbindung oder Salz davon nach einem der Ansprüche 1 bis 4, wobei R¹ eine C₁₋₇-Acyl- oder eine C₁₋₄-Alkyl-gruppe ist.

7. Verbindung oder Salz davon nach einem der Ansprüche 1 bis 6, wobei R² und R³ jeweils ein Wasserstoffatom oder eine C₁₋₄-Alkylgruppe sind.

8. Verbindung oder Salz davon nach einem der Ansprüche 1 bis 7, wobei R⁴ und R⁵ jeweils ein Wasserstoffatom, ein Halogenatom, eine Phenylgruppe oder eine C₁₋₄-Alkylgruppe sind.

9. Verbindung oder Salz davon nach einem der Ansprüche 1 bis 8, wobei R⁶ ein Wasserstoffatom oder eine C₁₋₄-Alkylgruppe ist.

10. Verbindung oder Salz davon nach Anspruch 1, wobei die Verbindung eine Struktur der folgenden Formel besitzt: 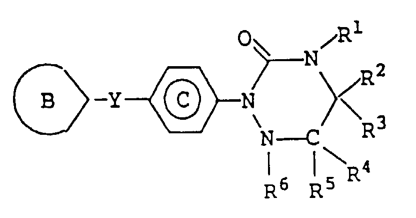
wobei der Ring B eine 5- oder 6-gliedrige cyclische Gruppe ist, welche ein oder mehrere Heteroatome enthalten kann, und/oder welche mit 1 bis 3 Substituenten substituiert sein kann, ausgewählt aus der Gruppe, bestehend aus C₁₋₄-Alkyl, C₁₋₄-Alkoxy, Carboxy, Carbamoyl, Halogen, Mono-, Di- oder Tri-halo-C₁₋₄-alkyl, Amino, -B(OH)₂, Hydroxy, Nitro, Cyano, Mercapto, Sulfo, Sulfino, Phospho und C₁₋₄-Acyl, wobei in dem Fall in dem die oben erwähnten Substituenten an zwei zueinander benachbarten ringbildenden Atomen vorkommen, sie miteinander verbunden sein können, um einen Ring zu bilden, der mit dem Ring B kondensiert ist;
wobei der Ring C eine Phenylengruppe ist, welche mit 1 bis 4 Substituenten substituiert sein kann, ausgewählt aus der Gruppe, bestehend aus (1) C₁₋₄-Alkyl, (2) C₂₋₄-Alkenyl, (3) C₂₋₄-Alkinyl, (4) C₃₋₆-Cycloalkyl, (5) C₅₋₇-Cycloalkenyl, (6) C₇₋₁₁-Aralkyl, (7) Phenyl, (8) C₁₋₆-Alkoxy, (9) Phenoxy, (10) C₁₋₆-Alkanoyl, (11) Benzoyl, (12) C₁₋₆-Alkanoyloxy, (13) Carboxyl, (14) C₂₋₇-Alkoxycarbonyl, (15) Carbamoyl, (16) N-Mono-C₁₋₄-alkylcarbamoyl, (17) N-Di-C₁₋₄-alkylcarbamoyl, (18) Cycloaminocarbonyl, (19) Halogen, (20) Mono-, Di- oder Tri-halo-C₁₋₄-alkyl, (21) Oxo, (22) Amidino, (23) Imino, (24) Amino, welches mit einer Gruppe geschützt sein kann, ausgewählt aus (i) Formyl, (ii) C₁₋₆-Alkylcarbonyl, welches mit Halogenatomen substituiert sein kann, (iii) C₆₋₁₀-Arylcarbonyl, welches mit 1 bis 3 Halogenatomen, C₁₋₆-Alkylcarbonyl- oder Nitrogruppe substituiert sein kann, (iv) C₁₋₆-Alkyloxycarbonyl, welches mit 1 bis 3 Halogenatomen, C₁₋₆-Alkylcarbonyl- oder Nitrogruppe substituiert sein kann, (v) C₆₋₁₀-Aryloxycarbonyl, welche mit 1 bis 3 Halogenatomen, C₁₋₆-Alkylcarbonyl- oder Nitrogruppe substituiert sein kann, (vi) C₇₋₁₂-Aralkylcarbonyl, welches mit 1 bis 3 Halogenatomen, C₁₋₆-Alkylcarbonyl- oder Nitrogruppe substituiert sein kann, (vii) Trityl, welches mit 1 bis 3 Halogenatomen, C₁₋₆-Alkylcarbonyl- oder Nitrogruppe substituiert sein kann und (viii) Phthaloyl, welches mit 1 bis 3 Halogenatomen, C₁₋₆-Alkylcarbonyl- oder Nitrogruppe substituiert sein kann, (25) Mono-C₁₋₄-alkylamino, (26) Di-C₁₋₄-alkylamino, (27) 3- bis 6-gliedrigem Cycloamino, welches 1 bis 3 Heteroatome enthalten kann, ausgewählt aus Sauerstoff, Schwefel und Stickstoff, (28) C₁₋₆-Alkanamido, (29) Benzamido, (30) Carbamoylamino, (31) N-C₁₋₄-Alkylcarbamoylamino, (32) N,N-Di-C₁₋₄-alkylcarbamoylamino, (33) C₁₋₃-Alkylendioxy, (34) -B(OH)₂, (35) Hydroxy, (36) Epoxy, (37) Nitro, (38) Cyano, (39) Mercapto, (40) Sulfo, (41) Sulfino, (42) Phosphono, (43) Dihydroxypolyol, (44) Sulfamoyl, (45) Mono-C₁₋₆-alkylsulfamoyl, (46) Di-C₁₋₄-alkylsulfamoyl, (47) C₁₋₆-Alkylthio, (48) Phenylthio, (49) C₁₋₆-Alkylsulfinyl, (50) Phenylsulfinyl, (51) C₁₋₆-Alkylsulfonyl, (52) Phenylsulfonyl und (53) einer 5- oder 6-gliedrigen heterocyclischen Gruppe, welche 1 bis 4 Heteroatome enthält, ausgewählt aus Sauerstoff, Schwefel und Stickstoff, welche über eine oder zwei Atomketten gebunden sein kann, die Sauerstoff, Schwefel, Stickstoff und Kohlenstoff enthalten, wobei jede Gruppe mit einer Kohlenstoffkette aus zwei oder mehr Kohlenstoffatomen oder einer cyclischen Gruppe ferner mit einem oder zwei Substituenten substituiert sein kann, ausgewählt aus der Gruppe, bestehend aus (a) Halogen, (b) Hydroxy, (c) Oxo, (d) C₁₋₄-Alkoxy, (e) Di-C₁₋₄-alkylamino, (f) Halo-C₁₋₄-alkyl, (g) C₁₋₄-Acyl, (h) Hydroxy-C₁₋₄-alkyl, (i) C₁₋₄-Alkoxy-₁₋₄-alkyl, (j) Cyano, (k) Thioxo und (1) C₁₋₄-Alkylthio, und wobei, wenn der oben erwähnte Substituent an zwei zueinander benachbarten ringbildenden Atomen vorhanden ist, sie miteinander verbunden sein können, um einen Ring zu bilden;
R₁ und R₆ jeweils ein Wasserstoffatom, eine C₁₋₇-Acyl- oder C₁₋₄-Alkylgruppe sind;
R₂ und R₃ jeweils ein Wasserstoffatom, ein Halogenatom oder eine C₁₋₄-Alkylgruppe oder eine Phenylgruppe, welche über ein Sauerstoff- oder Schwefelatom gebunden sein kann, sind;
R⁴ und R⁵ jeweils ein Wasserstoffatom, ein Halogenatom, eine Phenylgruppe oder eine C₁₋₄-Alkylgruppe sind;
R¹ und R² oder R⁵ und R⁶ zusammen eine chemische Bindung bilden;
Y eine chemische Bindung, -O-, -S(O)ₘ- oder eine C₁₋₄-Alkylengruppe ist, welche mit 1 bis 4 Substituenten substituiert sein kann, ausgewählt aus der Gruppe, bestehend aus Halogen, Hydroxy, Oxo, Cyano, C₁₋₄-Alkoxy, Mono- oder Di-C₁₋₄-alkylamino, Halo-C₁₋₄-alkyl, C₁₋₄-Acyl, Hydroxy-C₁₋₄-Alkyl, C₁₋₄-Alkoxy, C₁₋₄-Alkyl, C₁₋₄-Alkoxycarbonyl, Thioxo und C₁₋₄-Alkylthio, und m gleich 0, 1 oder 2 ist.

11. Verbindung oder ein Salz davon nach Anspruch 10, wobei der Ring B eine Phenyl-, Pyridyl-, Thiazolyl- oder Imidazolylgruppe ist, welche mit 1 bis 3 Substituenten substituiert sein kann, ausgewählt aus der Gruppe, bestehend aus C₁₋₄-Alkyl, C₁₋₄-Alkoxy, Carboxy, Carbamoyl, Halogen, Mono-, Di- oder Tri-halo-C₁₋₄-alkyl, Amino, -B(OH)₂, Hydroxy, Nitro, Cyano, Mercapto, Sulfo, Sulfino, Phospho und C₁₋₄-Acyl, und wobei die Substituenten, wenn sie an zwei zueinander benachbarten ringbildenden Atomen vorkommen, miteinander verbunden sein können, um einen Ring zu bilden.

12. Verbindung oder ein Salz davon nach Anspruch 11, wobei der Ring B eine Phenylgruppe ist, welche substituiert sein kann mit 1 bis 3 Substituenten, ausgewählt aus der Gruppe bestehend aus C₁₋₄-Alkyl, C₁₋₄-Alkoxy, Carboxy, Carbamoyl, Halogen, Mono-, Di- oder Tri-halo-C₁₋₄-alkyl, Amino, -B(OH)₂, Hydroxy, Nitro, Cyano, Mercapto, Sulfo, Sulfino, Phospho und C₁₋₄-Acyl, und wobei die Substituenten, wenn sie an zwei zueinander benachbarten ringbildenden Atomen vorkommen, miteinander verbunden sein können, um einen Ring zu bilden.

13. Verbindung oder ein Salz davon nach einem der Ansprüche 10 bis 12, wobei R¹ und R⁶ jeweils unabhängig Wasserstoff oder eine C₁₋₄-Alkylgruppe sind.

14. Verbindung oder ein Salz davon nach einem der Ansprüche 10 bis 12, wobei R¹ gleich Wasserstoff, eine C₁₋₇-Acyl- oder eine C₁₋₄-Alkylgruppe ist.

15. Verbindung oder ein Salz davon nach Anspruch 14, wobei R¹ gleich Wasserstoff ist.

16. Verbindung oder ein Salz davon nach einem der Ansprüche 10 bis 15, wobei R² und R³ jeweils unabhängig ein Wasserstoffatom, ein Halogenatom, eine Phenyl-, eine C₁₋₄-Alkyl-, C₁₋₄-Alkoxy- oder C₁₋₄-Alkylthiogruppe sind.

17. Verbindung oder ein Salz davon nach Anspruch 16, wobei R² und R³ jeweils unabhängig ein Wasserstoffatom oder eine C₁₋₄-Alkylgruppe sind.

18. Verbindung oder ein Salz davon nach Anspruch 17, wobei R² und R³ beide Wasserstoffatome sind.

19. Verbindung oder ein Salz davon nach einem der Ansprüche 10 bis 18, wobei R⁴ und R⁵ jeweils unabhängig ein Wasserstoffatom, ein Phenyl- oder eine C₁₋₄-Alkylgruppe sind.

20. Verbindung oder ein Salz davon nach Anspruch 19, wobei R⁴ ein Wasserstoffatom ist.

21. Verbindung oder ein Salz davon nach einem der Ansprüche 10 bis 20, wobei Y eine C₁₋₄-Alkylengruppe ist, welche mit 1 bis 4 Substituenten substituiert sein kann, ausgewählt aus der Gruppe, bestehend aus Halogen, Hydroxy, Oxo, Cyano, C₁₋₄-Alkoxy, Mono- oder Di-C₁₋₄-alkylamino, Halo-C₁₋₄-alkyl, C₁₋₄-Acyl, Hydroxy-C₁₋₄-alkyl, C₁₋₄-Alkoxy-C₁₋₄-alkyl, C₁₋₄-Alkoxycarbonyl, Thioxo und C₁₋₄-Alkylthio.

22. Verbindung oder ein Salz davon nach Anspruch 13, wobei R⁶ gleich Wasserstoff oder eine C₁₋₄-Alkylgruppe ist.

23. Verbindung oder ein Salz davon nach einem der Ansprüche 10 bis 12, wobei R⁵ und R⁶ zusammen eine chemische Bindung bilden.

24. Verbindung oder ein Salz davon nach Anspruch 1, wobei R¹, R², R³ und R⁴ ein Wasserstoffatom sind und R⁵ und R⁶ eine chemische Bindung bilden.

25. Verbindung oder ein Salz davon nach Anspruch 10, wobei der Ring B ein Phenyl ist, welches mit 1 bis 3 Substituenten substituiert sein kann, ausgewählt aus C₁₋₄-Alkyl, C₁₋₄-Alkoxy, Carboxy, Carbamoyl, Halogen, Mono-, Di- oder Tri-halo-C₁₋₄-alkyl, Amino, -B(OH)₂, Hydroxy, Nitro, Cyano, Mercapto, Sulfo, Sulfino, Phospho und C₁₋₄-Acyl, und wobei die Substituenten, wenn sie an zwei zueinander benachbarten ringbildenden Atomen vorkommen, miteinander verbunden sein können, um einen Ring zu bilden; wobei der Ring C ein Phenylen ist, welches mit 1 bis 4 Substituenten substituiert sein kann, ausgewählt aus der Gruppe, bestehend aus Halogen, C₁₋₄-Alkyl, C₁₋₆-Alkoxy und Halo-C₁₋₄-alkyl; R¹, R², R³ und R⁴ Wasserstoffatome sind; R⁵ und R⁶ zusammen eine chemische Bindung bilden; und Y eine C₁₋₄-Alkylengruppe ist, welche substituiert sein kann mit 1 bis 4 Substituenten, ausgewählt aus der Gruppe, bestehend aus Halogen, Hydroxy, Oxo, Cyano, C₁₋₄-Alkoxy, Mono- oder Di-C₁₋₄-alkylamino, Halo-C₁₋₄-alkyl, C₁₋₄-Acyl, Hydroxy-C₁₋₄-alkyl, C₁₋₄-Alkoxy-C₁₋₄-alkyl, C₁₋₄-Alkoxycarbonyl, Thioxo und C₁₋₄-Alkylthio.

26. Verbindung nach Anspruch 10, welche 2-[3,5-Dichlor-4-(4-chlorbenyzl)phenyl]-2,3,4,5-tetrahydro-1,2,4-triazin-3-on oder ein Salz davon ist.

27. Verbindung nach Anspruch 10, welche 2-[3-Chlor-4-(4-chlorbenzyl)-5-methylphenyl]-2,3,4,5-tetrahydro-1,2,4-triazin-3-on oder ein Salz davon ist.

28. Verbindung nach Anspruch 10, welche 2-[3,5-Dichlor-4-(4'chlor-1-methylbenzyl)phenyl]-2,3,4,5-tetrahydro-1,2,4-triazin-3-on oder ein Salz davon ist.

29. Antiprotozoenzusammensetzung, umfassend eine wirksame Menge einer Verbindung gemäß einem der Ansprüche 1 bis 28 oder ein Salz davon und einen pharmazeutisch akzeptablen Träger, Arzneimittelträger oder Verdünnungsmittel.

30. Zusammensetzung nach Anspruch 29, wobei die Verbindung eine Verbindung gemäß Anspruch 10 oder ein Salz davon ist.

31. Antikokzidienzusammensetzung, umfassend eine wirksame Menge einer Verbindung gemäß Anspruch 10 oder ein Salz davon und einen pharmazeutisch akzeptablen Träger, Arzneimittelträger oder Verdünnungsmittel.

32. Tierfuttermittel-Zusatzstoffvormischung, umfassend eine Verbindung gemäß Anspruch 29 oder ein physiologisch akzeptables Salz davon.

33. Verfahren zur Herstellung einer Verbindung gemäß einem der Ansprüche 1 bis 28 oder eines Salzes davon, umfassend das Cyclisieren der Verbindung der Formel: 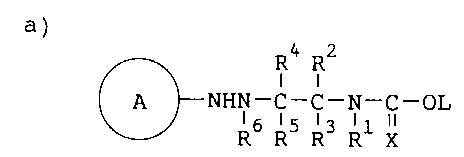 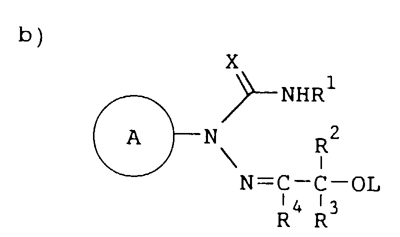 oder 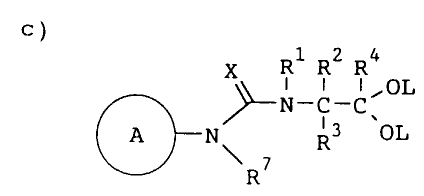 wobei A, R¹, R², R³, R⁴, R⁵ und R⁶ dieselbe Bedeutung haben, wie sie in Anspruch 1 definiert ist, R⁷ eine gegebenenfalls geschützte Aminogruppe ist und L ein Alkyl oder Acyl ist.

34. Verfahren nach Anspruch 33, wobei die Cyclisierungsreaktion in einem inerten Lösungsmittel oder in Abwesenheit eines Lösungsmittels gegebenenfalls in Gegenwart einer Lewissäure oder Lewisbase durchgeführt wird.

35. Verfahren nach Anspruch 33 oder 34, wobei die Cyclisierungsreaktion der Verbindung der Formel a) bei einer Temperatur von ungefähr 60°C bis ungefähr 200°C durchgeführt wird.

36. Verfahren nach Anspruch 33 oder 34, wobei die Cyclisierungsreaktion der Verbindung der Formel b) bei einer Temperatur von ungefähr 0°C bis ungefähr 200°C durchgeführt wird.

37. Verfahren nach Anspruch 33 oder 34, wobei die Cyclisierungsreaktion der Verbindung der Formel c) bei einer Temperatur von ungefähr 60°C bis ungefähr 160°C durchgeführt wird.

## Revendications

1. Composé de formule 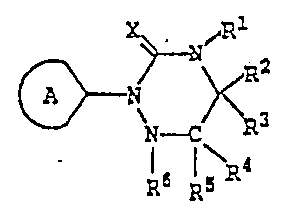 où le cycle A représente un groupe phényle ou un groupe hétérocyclique insaturé à 5 ou 6 chaînons qui peut porter de 1 à 3 substituants choisis parmi les résidus (1) alkyle en C₁₋₄, (2) alcényle en C₂₋₄, (3) alcynyle en C₂₋₄, (4) cycloalkyle en C₃₋₆, (5) cycloalcényle en C₅₋₇, (6) aralkyle en C₇₋₁₁, (7) phényle, (8) alcoxy en C₁₋₆, (9) phénoxy, (10) alcanoyle en C₁₋₆, (11) benzoyle, (12) alcanoyloxy en C₁₋₆, (13) carboxyle, (14) (alcoxy en C₃₋₇)-carbonyle, (15) carbamoyle, (16) N-mono-(alkyle en C₁₋₄)-carbamoyle, (17) N-di-(alkyle en C₁₋₄)-carbamoyle, (18) cycloaminocarbonyle, (19) halogéno, (20) mono-, di- ou tri-halogéno-(alkyle en C₁₋₄), (21) oxo, (22) amidino, (23) imino, (24) amino qui peut être protégé par un groupe choisi parmi les résidus
(i) formyle,
(ii) (alkyle en C₁₋₆)-carbonyle pouvant être substitué par des atomes d'halogène,
(iii) (aryle en C₆₋₁₀)-carbonyle pouvant porter de 1 à 3 substituants halogéno, (alkyle en C₁₋₆)-carbonyle ou nitro,
(iv) (alkyle en C₁₋₆)-oxycarbonyle pouvant porter de 1 à 3 substituants halogéno, (alkyle en C₁₋₆)-carbonyle ou nitro,
(v) (aryle en C₆₋₁₀)-oxycarbonyle pouvant porter de 1 à 3 substituants halogéno, (alkyle en C₁₋₆)-carbonyle ou nitro,
(vi) (aralkyle en C₇₋₁₂)-carbonyle pouvant porter de 1 à 3 substituants halogéno, (alkyle en C₁₋₆)-carbonyle ou nitro,
(vii) trityle pouvant porter de 1 à 3 substituants halogéno, (alkyle en C₁₋₆)-carbonyle ou nitro, et
(viii) phtaloyle pouvant porter de 1 à 3 substituants halogéno, (alkyle en C₁₋₆)-carbonyle ou nitro,
(25) mono-(alkyle en C₁₋₄)-amino, (26) di-(alkyle en C₁₋₄), (27) cycloamino comportant de 3 à 6 chaînons et pouvant contenir de 1 à 3 hétéroatomes choisis parmi l'oxygène, le soufre et l'azote, (28) N-(alcane en C₁₋₆)-amino, (29) benzamido, (30) carbamoylamino, (31) N-(alkyle en C₁₋₄)-carbamoylamino, (32) N,N-di(alkyle en C₁₋₄)-carbamoylamino, (33) (alkylène en C₁₋₃)-dioxy, (34) -B(OH₂), (35) hydroxy, (36) époxy, (37) nitro, (38) cyano, (39) mercapto, (40) sulfo, (41) sulfino, (42) phosphono, (43) dihydroxypolyol, (44) sulfamoyle, (45) mono-(alkyle en C₁₋₆)-sulfamoyle, (46) di(alkyle en C₁₋₄)-sulfamoyle, (47) (alkyle en C₁₋₆)-thio, (48) phénylthio, (49) (alkyle en C₁₋₆)-sulfinyle, (50) phénylsulfinyle, (51) (alkyle en C₁₋₆)-sulfonyle, (52) phénylsulfonyle et (53) un groupe hétérocyclique à 5 ou 6 chaînons comportant de 1 à 4 hétéroatomes choisis parmi l'oxygène, le soufre et l'azote, et qui peut être lié par l'intermédiaire d'une ou de deux chaînes d'atomes contenant de l'oxygène, du soufre, de l'azote et du carbone, chaque groupe comportant une chaîne carboné contenant 2 ou plus d'atomes de carbone ou un groupe cyclique, pouvant porter en outre un ou deux susbtituants choisis dans le groupe formé par les résidus
(a) halogéno,
(b) hydroxy,
(c) oxo,
(d) alcoxy en C₁₋₄,
(e) di(alkyle en C₁₋₄)-amino,
(f) halogéno-(alkyle en C₁₋₄),
(g) acyle en C₁₋₄,
(h) hydroxy-(alkyle en C₁₋₄),
(i) (alcoxy en C₁₋₄)-(alkyle en C₁₋₄),
(j) cyano,
(k) thioxo, et
(l) alkylthio en C₁₋₄,
et dans lequel, lorsque deux atomes adjacents du cycle portent des substiuants mentionnés ci-dessus, ceux-ci peuvent être liés l'un à l'autre pour former un cycle ;
X est un atome d'oxygène ou de soufre,
R¹ et R⁶ représentent chacun un atome d'hydrogène, un groupe acyle en C₁₋₇ ou alkyle en C₁₋₄ ;
R² et R³ représentent chacun un atome d'hydrogène ou d'halogène, ou un groupe alkyle en C₁₋₄ ou phényle pouvant être lié par l'intermédiaire d'un atome d'oxygène ou de soufre ;
R⁴ et R⁵ représentent chacun un atome d'hydrogène ou d'halogène, ou un groupe phényle ou alkyle en C₁₋₄,
R¹ et R² ou R⁵ et R⁶ peuvent former une liaison chimique, à condition que, lorsque-le cycle A est un groupe phényle portant au moins un atome d'halogène en position 2 ou 4 et X est un atome d'oxygène, alors R⁵ et R⁶ ne sont pas liés l'un à l'autre pour former une liaison chimique, ou un sel d'un tel composé.

2. Composé ou sel d'un composé selon la revendication 1 dans lequel le cycle A représente un groupe phényle pouvant porter de 1 à 3 substituants choisis parmi les résidus halogéno, alkyle en C₁₋₄, halogéno-(alkyle en C₁₋₄), phényl-(alkyle en C₁₋₄) et phényle ou un groupe hétérocyclique à 5 ou 6 chaînons contenant de 1 à 4 hétéroatomes choisis parmi l'oxygène, le soufre et l'azote, qui peut être lié par l'intermédiaire d'une chaîne d'atomes comportant 1 ou 2 atomes englobant l'oxygène, le soufre, l'azote et le carbone.

3. Composé ou sel d'un composé selon la revendication 1, dans lequel ledit groupe hétérocyclique est un groupe hétérocycique monocylique pouvant porter de 1 à 3 substituants choisis parmi les résidus (1) alkyle en C₁₋₄, (2) alcényle en C₂₋₄, (3) alcynyle en C₂₋₄, (4) cycloalkyle en C₃₋₆, (5) cycloalcényle en C₅₋₇, (6) aralkyle en C₇₋₁₁, (7) phényle, (8) alcoxy en C₁₋₆, (9) phénoxy, (10) alcanoyle en C₁₋₆, (11) benzoyle, (12) alcanoyloxy en C₁₋₆, (13) carboxyle, (14) (alcoxy en C₃₋₇)-carbonyle, (15) carbamoyle, (16) N-mono-(alkyle en C₁₋₄)-carbamoyle, (17) N-di-(alkyle en C₁₋₄)-carbamoyle, (18) cycloaminocarbonyle, (19) halogéno, (20) mono-, di- ou tri-halogéno-(alkyle en C₁₋₄), (21) oxo, (22) amidino, (23) imino, (24) amino qui peut être protégé par un groupe choisi parmi les résidus
(i) formyle,
(ii) (alkyle en C₁₋₆)-carbonyle pouvant être substitué par des atomes d'halogène,
(iii) (aryle en C₆₋₁₀)-carbonyle pouvant porter de 1 à 3 substituants halogéno, (alkyle en C₁₋₆)-carbonyle ou nitro,
(iv) (alkyle en C₁₋₆)-oxycarbonyle pouvant porter de 1 à 3 substituants halogéno, (alkyle en C₁₋₆)-carbonyle ou nitro,
(v) (aryle en C₆₋₁₀)-oxycarbonyle pouvant porter de 1 à 3 substituants halogéno, (alkyle en C₁₋₆)-carbonyle ou nitro,
(vi) (aralkyle en C₇₋₁₂)-carbonyle pouvant porter de 1 à 3 substituants halogéno, (alkyle en C₁₋₆)-carbonyle ou nitro,
(vii) trityle pouvant porter de 1 à 3 substituants halogéno, (alkyle en C₁₋₆)-carbonyle ou nitro, et
(viii) phtaloyle pouvant porter de 1 à 3 substituants halogéno, (alkyle en C₁₋₆)-carbonyle ou nitro,
(25) mono-(alkyle en C₁₋₄)-amino, (26) di-(alkyle en C₁₋₄), (27) cycloamino comportant de 3 à 6 chaînons et pouvant contenir de 1 à 3 hétéroatomes choisi parmi l'oxygène, le soufre et l'azote, (28) N-(alcane en C₁₋₆)-amino, (29) benzamido, (30) carbamoylamino, (31) N-(alkyle en C₁₋₄)-carbamoylamino, (32) N,N-di(alkyle en C₁₋₄)-carbamoylamino, (33) (alkylène en C₁₋₃)-dioxy, (34) -B(OH₂), (35) hydroxy, (36) époxy, (37) nitro, (38) cyano, (39) mercapto, (40) sulfo, (41) sulfino, (42) phosphono, (43) dihydroxypolyol, (44) sulfamoyle, (45) mono-(alkyle en C₁₋₆)-sulfamoyle, (46) di(alkyle en C₁₋₄)-sulfamoyle, (47) (alkyle en C₁₋₆)-thio, (48) phénylthio, (49) (alkyle en C₁₋₆)-sulfinyle, (50) phénylsulfinyle, (51) (alkyle en C₁₋₆)-sulfonyle, (52) phénylsulfonyle et (53) un groupe hétérocyclique à 5 ou 6 chaînons comportant de 1 à 4 hétéroatomes choisis parmi l'oxygène, le soufre et l'azote, et qui peut être lié par l'intermédiaire d'une ou de deux chaînes d'atomes contenant de l'oxygène, du soufre, de l'azote et du carbone, chaque groupe comportant une chaîne carboné contenant 2 ou plus d'atomes de carbone ou un groupe cyclique, pouvant porter en outre un ou deux susbtituants choisis dans le groupe formé par les résidus
(a) halogéno,
(b) hydroxy,
(c) oxo,
(d) alcoxy en C₁₋₄,
(e) di(alkyle en C₁₋₄)-amino,
(f) halogéno-(alkyle en C₁₋₄),
(g) acyle en C₁₋₄,
(h) hydroxy-(alkyle en C₁₋₄),
(i) (alcoxy en C₁₋₄)-(alkyle en C₁₋₄),
(j) cyano,
(k) thioxo, et
(l) alkylthio en C₁₋₄,
et dans lequel, lorsque deux atomes adjacents du cycle portent des substiuants mentionnés ci-dessus, ceux-ci peuvent être liés l'un à l'autre pour former un cycle.

4. Composé ou sel d'un composé selon l'une quelconque des revendications 1 à 3, dans lequel le cycle A est un groupe pyridyle.

5. Composé ou sel d'un composé selon l'une quelconque des revendications 1 à 4 dans lequel R¹ représente un atome d'hydrogène.

6. Composé ou sel d'un composé selon l'une quelconque des revendications 1 à 4 dans lequel R¹ représente un groupe acyle en C₁₋₇ ou alkyle en C₁₋₄.

7. Composé ou sel d'un composé selon l'une quelconque des revendications 1 à 6 dans lequel R² et R³ représentent chacun un atome d'hydrogène ou un groupe alkyle en C₁₋₄.

8. Composé ou sel d'un composé selon l'une quelconque des revendications 1 à 7 dans lequel R⁴ et R⁵ représentent chacun un atome d'hydrogène, un atome d'halogène, un groupe phényle ou un groupe alkyle en C₁₋₄.

9. Composé ou sel d'un composé selon l'une quelconque des revendications 1 à 8 dans lequel R⁶ représente un atome d'hydrogène ou un groupe alkyle en C₁₋₄·

10. Composé ou sel d'un composé selon la revendication 1 où ledit composé a une structure de formule 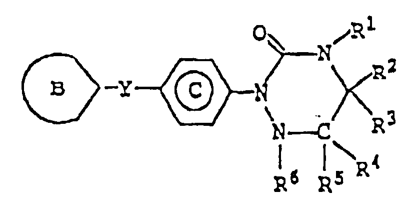 dans laquelle B représente un groupe cyclique à 5 ou 6 chaînons qui peut contenir un ou plusieurs hétéroatomes et/ou qui peut porter de 1 à 3 substituants choisi parmi les groupes alkyle en C₁₋₄, alcoxy en C₁₋₄, carboxy, carbamoyle, halogéno, mono-, di- ou tri-halogéno-(alkyle en C₁₋₄), amino, -B(OH)₂, hydroxy, nitro, cyano, mercapto, sulfo, sulfino, phospho et acyle en C₁₋₄, et dans lequel, lorsque deux atomes adjacents du cycle portent des substiuants mentionnés ci-dessus, ceux-ci peuvent être liés l'un à l'autre pour former un cycle qui est condensé au cycle B ;
le cycle C représente un groupe phénylène pouvant porter de 1 à 4 substituants choisis parmi les résidus (1) alkyle en C₁₋₄, (2) alcényle en C₂₋₄, (3) alcynyle en C₂₋₄, (4) cycloalkyle en C₃₋₆, (5) cycloalcényle en C₅₋₇, (6) aralkyle en C₇₋₁₁, (7) phényle, (8) alcoxy en C₁₋₆, (9) phénoxy, (10) alcanoyle en C₁₋₆, (11) benzoyle, (12) alcanoyloxy en C₁₋₆, (13) carboxyle, (14) (alcoxy en C₃₋₇)-carbonyle, (15) carbamoyle, (16) N-mono-(alkyle en C₁₋₄)-carbamoyle, (17) N-di-(alkyle en C₁₋₄)-carbamoyle, (18) cycloaminocarbonyle, (19) halogéno, (20) mono-, di- ou tri-halogéno-(alkyle en C₁₋₄), (21) oxo, (22) amidino, (23) imino, (24) amino qui peut être protégé par un groupe choisi parmi les résidus
(i) formyle,
(ii) (alkyle en C₁₋₆)-carbonyle pouvant être substitué par des atomes d'halogène,
(iii) (aryle en C₆₋₁₀)-carbonyle pouvant porter de 1 à 3 substituants halogéno, (alkyle en C₁₋₆)-carbonyle ou nitro,
(iv) (alkyle en C₁₋₆)-oxycarbonyle pouvant porter de 1 à 3 substituants halogéno, (alkyle en C₁₋₆)-carbonyle ou nitro,
(v) (aryle en C₆₋₁₀)-oxycarbonyle pouvant porter de 1 à 3 substituants halogéno, (alkyle en C₁₋₆)-carbonyle ou nitro,
(vi) (aralkyle en C₇₋₁₂)-carbonyle pouvant porter de 1 à 3 substituants halogéno, (alkyle en C₁₋₆)-carbonyle ou nitro,
(vii) trityle pouvant porter de 1 à 3 substituants halogéno, (alkyle en C₁₋₆)-carbonyle ou nitro, et
(viii) phtaloyle pouvant porter de 1 à 3 substituants halogéno, (alkyle en C₁₋₆)-carbonyle ou nitro,
(25) mono-(alkyle en C₁₋₄)-amino, (26) di-(alkyle en C₁₋₄), (27) cycloamino comportant de 3 à 6 chaînons et pouvant contenir de 1 à 3 hétéroatomes choisis parmi l'oxygène, le soufre et l'azote, (28) N-(alcane en C₁₋₆)-amino, (29) benzamido, (30) carbamoylamino, (31) N-(alkyle en C₁₋₄)-carbamoylamino, (32) N,N-di(alkyle en C₁₋₄)-carbamoylamino, (33) (alkylène en C₁₋₃)-dioxy, (34) -B(OH₂), (35) hydroxy, (36) époxy, (37) nitro, (38) cyano, (39) mercapto, (40) sulfo, (41) sulfino, (42) phosphono, (43) dihydroxypolyol, (44) sulfamoyle, (45) mono-(alkyle en C₁₋₆₎-sulfamoyle, (46) di(alkyle en C₁₋₄)-sulfamoyle, (47) (alkyle en C₁₋₆)-thio, (48) phénylthio, (49) (alkyle en C₁₋₆)-sulfinyle, (50) phénylsulfinyle, (51) (alkyle en C₁₋₆)-sulfonyle, (52) phénylsulfonyle et (53) un groupe hétérocyclique à 5 ou 6 chaînons comportant de 1 à 4 hétéroatomes choisis parmi l'oxygène, le soufre et l'azote, et qui peut être lié par l'intermédiaire d'une ou de deux chaînes d'atomes contenant de l'oxygène, du soufre, de l'azote et du carbone, chaque groupe comportant une chaîne carboné contenant 2 ou plus d'atomes de carbone ou un groupe cyclique, pouvant porter en outre un ou deux susbtituants choisis dans le groupe formé par les résidus
(a) halogéno,
(b) hydroxy,
(c) oxo,
(d) alcoxy en C₁₋₄,
(e) di(alkyle en C₁₋₄)-amino,
(f) halogéno-(alkyle en C₁₋₄),
(g) acyle en C₁₋₄,
(h) hydroxy-(alkyle en C₁₋₄),
(i) (alcoxy en C₁₋₄)-(alkyle en C₁₋₄),
(j) cyano,
(k) thioxo, et
(l) alkylthio en C₁₋₄,
et dans lequel, lorsque deux atomes adjacents du cycle portent des substiuants mentionnés ci-dessus, ceux-ci peuvent être liés l'un à l'autre pour former un cycle,
R¹ et R⁶ représentent chacun un atome d'hydrogène, un groupe acyle en C₁₋₇ ou alkyle en C₁₋₄;
R² et R³ représentent chacun un atome d'hydrogène ou d'halogène, ou un groupe alkyle en C₁₋₄ ou phényle pouvant être lié par l'intermédiaire d'un atome d'oxygène ou de soufre ;
R⁴ et R⁵ représentent chacun un atome d'hydrogène ou d'halogène, ou un groupe phényle ou alkyle en C₁₋₄,
R¹ et R² ou R⁵ et R⁶ peuvent former une liaison chimique,
Y représente une liaison chimique, un groupe -O-, -S(O)ₘ- ou alkylène en C₁₋₄ pouvant porter de 1 à 4 substituants choisis parmi les résidus halogéno, hydroxy, oxo, cyano, alcoxy en C₁₋₄, mono- ou di-(alkyle en C₁₋₄)-amino, halogéno-(alkyle en C₁₋₄), acyle en C₁₋₄, hydroxy-(alkyle en C₁₋₄), (alcoxy en C₁₋₄)-(alkyle en C₁₋₄), (alcoxy en C₁₋₄)-carbonyle, thioxo et (alkyle en C₁₋₄)-thio et m vaut 0, 1 ou 2.

11. Composé ou sel d'un composé selon la revendication 10 dans lequel le cycle B est un groupe phényle, pyridyle, thiazolyle ou imidazolyle pouvant porter de 1 à 3 substituants choisis parmi les groupes alkyle en C₁₋₄, alcoxy en C₁₋₄, carboxy, carbamoyle, halogéno, mono-, di- ou tri-halogéno-(alkyle en C₁₋₄), amino, -B(OH)₂, hydroxy, nitro, cyano, mercapto, sulfo, sulfino, phospho et acyle en C₁₋₄, et dans lequel, lorsque deux atomes adjacents du cycle portent des substiuants, ceux-ci peuvent être liés l'un à l'autre pour former un cycle.

12. Composé ou sel d'un composé selon la revendication 11, dans lequel le cycle B est un groupe phényle pouvant porter de 1 à 3 substituants choisis parmi les groupes alkyle en C₁₋₄, alcoxy en C₁₋₄, carboxy, carbamoyle, halogéno, mono-, di- ou tri-halogéno-(alkyle en C₁₋₄), amino, -B(OH)₂, hydroxy, nitro, cyano, mercapto, sulfo, sulfino, phospho et acyle en C₁₋₄, et dans lequel, lorsque deux atomes adjacents du cycle portent des substiuants, ceux-ci peuvent être liés l'un à l'autre pour former un cycle.

13. Composé ou sel d'un composé selon l'une quelconque des revendications 10 - 12, dans lequel R¹ et R⁶ représentent chacun indépendamment un atome d'hydrogène ou un groupe alkyle en C₁₋₄.

14. Composé ou sel d'un composé selon l'une quelconque des revendications 1 à 12 dans lequel R¹ représente un atome d'hydrogène, un gorupe acyle en C₁₋₇ ou alkyle en C₁₋₄.

15. Composé ou sel d'un composé selon la revendication 14 dans lequel R¹ est un atome d'hydrogène.

16. Composé ou sel d'un composé selon l'une quelconque des revendications 10 - 15, dans lequel R² et R³ représentent chacun indépendamment l'un de l'autre un atome d'hydrogène, un atome d'halogène ou un groupe phényle, alkyle en C₁₋₄, alcoxy en C₁₋₄ ou alkylthio en C₁₋₄.

17. Composé ou sel d'un composé selon la revendication 16, dans lequel R² et R³ représentent indépendamment l'un de l'autre chacun un atome d'hydrogène ou un groupe alkyle en C₁₋₄.

18. Composé ou sel d'un composé selon la revendication 17, dans lequel R² et R³ représentent chacun un atome d'hydrogène.

19. Composé ou sel d'un composé selon l'une quelconque des revendications 10 - 18 dans lequel R⁴ et R⁵ représentent indépendamment l'un de l'autre chacun un atome d'hydrogène, un groupe phényle ou alkyle en C₁₋₄.

20. Composé ou sel d'un composé selon la revendication 19 dans leque R⁴ est un atome d'hydrogène.

21. Composé ou sel d'un composé selon l'une quelconque des revendications 10 - 20 dans lequel Y est un groupe alkylène en C₁₋₄ pouvant porter de 1 à 4 substituants choisis parmi les résidus halogéno, hydroxy, oxo, cyano, alcoxy en C₁₋₄, mono- ou di-(alkyle en C₁₋₄)-amino, halogéno-(alkyle en C₁₋₄), acyle en C₁₋₄, hydroxy-(alkyle en C₁₋₄), (alcoxy en C₁₋₄)-(alkyle en C₁₋₄), (alcoxy en C₁₋₄)-carbonyle, thioxo et (alkyle en C₁₋₄)-thio.

22. Composé ou sel d'un composé selon la revendication 13 dans lequel R⁶ représente un atome d'hydrogène ou un groupe alkyle en C₁₋₄.

23. Composé ou sel d'un composé selon l'une quelconque des revendications 10 - 12 dans lequel R⁵ et R⁶ sont liés par une liaison chimique.

24. Composé ou sel d'un composé selon la revendication 1 dans lequel R¹, R², R³ et R⁴ représentent un atome d'hydrogène et R⁵ et R⁶ sont liés par une liaison chimique.

25. Composé ou sel d'un composé selon la revendication 10 dans lequel le cycle B est un groupe phényle pouvant porter de 1 à 3 substituants choisis parmi les résidus alkyle en C₁₋₄, alcoxy en C₁₋₄, carboxy, carbamoyle, halogéno, mono-, di- ou tri-halogéno-(alkyle en C₁₋₄), amino, -B(OH)₂, hydroxy, nitro, cyano, mercapto, sulfo, sulfino, phospho et acyle en C₁₋₄, et dans lequel, lorsque deux atomes adjacents du cycle portent des substiuants, ceux-ci peuvent être liés l'un à l'autre pour former un cycle;
le cycle C est un groupe phénylène pouvant porter de 1 à 4 substituants choisis parmi les résidus halogéno, alkyle en C₁₋₄, alcoxy en C₁₋₆ et halogéno-(alkyle en C₁₋₄);
R¹, R², R³ et R⁴ représentent des atomes d'hydrogène ;
R⁵ et R⁶ sont reliés par une liaison chimique, et
Y est un groupe alkylène en C₁₋₄ pouvant porter de 1 à 4 substituants choisis parmi les résidus halogéno, hydroxy, oxo, cyano, alcoxy en C₁₋₄, mono- ou di-(alkyle en C₁₋₄)-amino, halogéno-(alkyle en C₁₋₄), acyle en C₁₋₄, hydroxy-(alkyle en C₁₋₄), (alcoxy en C₁₋₄)-(alkyle en C₁₋₄), (alcoxy en C₁₋₄)-carbonyle, thioxo et (alkyle en C₁₋₄)-thio.

26. Composé selon la revendication 10 qui est la 2-[3,5-dichloro-4-(4-chlorobenzyl)phényl]-2,3,4,5-tétrahydro-1,2,4,-triazin-3-one ou un sel de ce composé.

27. Composé selon la revendication 10 qui est la 2-[3-chloro-4-(4-chlorobenzyl)-5-méthylphényl]-2,3,4,5-tétrahydro-1,2,4,-triazin-3-one ou un sel de ce composé.

28. Composé selon la revendication 10 qui est la 2-[3,5-dichloro-4-(4'-chloro-1-méthylbenzyl)phényl]-2,3,4,5-tétrahydro-1,2,4,-triazin-3-one ou un sel de ce composé.

29. Composition anti-protozoaires comprenant une quantité efficace d'un composé selon l'une quelconque des revendications 1 à 28 ou un sel d'un tel composé, ainsi qu'un véhicule, excipient ou diluant pharmaceutiquement acceptable.

30. Composition selon la revendication 29 dans lequele ledit composé est un composé selon la revendication 10 ou un sel d'un tel composé.

31. Composition pour le traitement de la coccidiose comprenant une quantité efficace d'un composé selon la revendication 10 ou un sel d'un tel composé, ainsi qu'un véhicule, excipient ou diluant pharmaceutiquement acceptable.

32. Prémélange de complément alimentaire pour animaux comprenant un composé selon la revendication 29 ou un sel physiologiquement acceptable d'un tel composé.

33. Procédé de préparation d'un composé selon l'une quelconque des revendications 1 à 28 ou d'un sel d'un tel composé comprenant la cyclisation d'un composé de formule 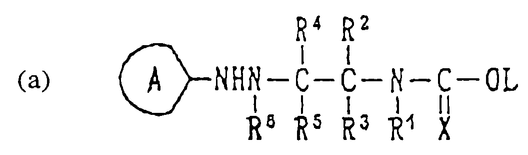 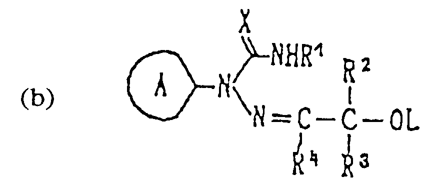 ou 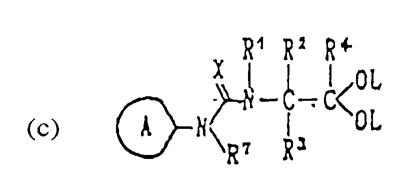 dans lesquels A, R¹, R², R³, R⁴, R⁵ et R⁶ ont la même signification que dans la revendication 1, R⁷ est un groupe amino protégé et L représente un groupe alkyle ou acyle.

34. Procédé selon la revendication 33, dans lequel la réaction de cyclisation est effectuée dans un solvant inerte ou en l'absence d'un solvant et éventuellement en présence d'un acide de Lewis ou d'une base de Lewis.

35. Procédé selon la revendication 33 ou 34, dans lequel la réaction de cyclisation du composé de formule (a) est effectuée à une température comprise entre environ 60 °C et environ 200 °C.

36. Procédé selon la revendication 33 ou 34, dans lequel la réaction de cyclisation du composé de formule (b) est effectuée à une température comprise entre environ 0 °C et environ 200 °C.

37. Procédé selon la revendication 33 ou 34, où la réaction de cyclisation du composé de formule (c) est effectué à une température comprise entre environ 60 °C et environ 160 °C.
